# EUROPEAN PATENT SPECIFICATION

(11) **EP 2 069 504 B1**
(45) Date of publication and mention of the grant of the patent: **03.06.2015**
(21) Application number: 07857537.0
(22) Date of filing: 13.12.2007
(51) Int. Cl.: C12N 15/82, C07K 14/415, A01H 5/00

(54) **PLANTS HAVING ENHANCED SEED YIELD-RELATED TRAITS AND A METHOD FOR MAKING THE SAME**
PFLANZEN MIT VERBESSERTEN EIGENSCHAFTEN BEZÜGLICH DES SAMENERTRAGS UND VERFAHREN ZU IHRER HERSTELLUNG
PLANTES AYANT DES CARACTÉRISTIQUES LIÉES À UN RENDEMENT AMÉLIORÉ ET LEUR PROCÉDÉ DE FABRICATION

(30) Priority: 15.12.2006 EP 06126279; 03.01.2007 US 883168 P
(43) Date of publication of application: 17.06.2009
(60) Divisional application: 12151882.3
(73) Proprietor: CropDesign N.V., 9052 Zwijnaarde (BE)
(72) Inventor: SANZ MOLINERO, Ana Isabel, 9050 Gentbrugge (BE); VANDESTEENE, Lies, 9500 Geraardsbergen (BE); RAMON, Matthew, 8500 Kortrijk (BE); ROLLAND, Filip, 3020 Winksele (BE); VAN DIJCK, Patrick, 3217 Zichem (BE); THEVELEIN, Johan, 6052 Oud-Heverlee (BE)
(74) Representative: Saelens, Claire
(86) International application number: PCT/EP2007/063890
(87) International publication number: WO 2008/071767

(56) References cited:
- WO-A-97/42326
- WO-A-2006/060376
- US-A1- 2004 229 364
- US-A1- 2006 191 040
- SATOH-NAGASAWA NAMIKO ET AL: "A trehalose metabolic enzyme controls inflorescence architecture in maize." NATURE 11 MAY 2006, vol. 441, no. 7090, 11 May 2006 (2006-05-11), pages 227-230, XP002428616 ISSN: 1476-4687
- SCHLUEPMANN HENRIETTE ET AL: "Trehalose 6-phosphate is indispensable for carbohydrate utilization and growth in Arabidopsis thaliana." PROCEEDINGS OF THE NATIONAL ACADEMY OF SCIENCES OF THE UNITED STATES OF AMERICA, vol. 100, no. 11, 27 May 2003 (2003-05-27), pages 6849-6854, XP002428617 ISSN: 0027-8424
- VOGEL G ET AL: "TREHALOSE METABOLISM IN ARABIDOPSIS: OCCURRENCE OF TREHALOSE AND MOLECULAR CLONING AND CHARACTERIZATION OF TREHALOSE-6-PHOSPHATE SYNTHASE HOMOLOGUES" JOURNAL OF EXPERIMENTAL BOTANY, OXFORD UNIVERSITY PRESS, GB, vol. 362, no. 52, September 2001 (2001-09), pages 1817-1826, XP001063315 ISSN: 0022-0957
- DATABASE EMBL Similar to trehalose 6 phosphate phosphatase 12 December 2002 (2002-12-12), SHOEMAKER ET AL.: XP002443765 retrieved from EBI Database accession no. CA783255
- DATABASE EMBL Serpentine sunflower cDNA 29 August 2006 (2006-08-29), MICHELMORE ET AL.: XP002443766 retrieved from EBI Database accession no. EE642400
- DATABASE UniProt Trehalose-6-phosphate phosphatase (AtTPPB) 1 June 2001 (2001-06-01), XP002475177 retrieved from EBI Database accession no. Q9C9S4
- DATABASE EPO PROTEINS Soybean trehalose-6-phosphate phosphatase 18 November 2004 (2004-11-18), CAIMI ET AL.: XP002475178 retrieved from EBI Database accession no. ADU47505
- DATABASE EPO PROTEINS Plant full length insert polypeptide 21 April 2005 (2005-04-21), LIU ET AL.: XP002475179 retrieved from EBI Database accession no. ADX92172

## Description

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as, corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Another yield-related trait of importance is plant biomass, particularly in the case of forage crops, such as alfalfa, silage corn and hay. A larger plant with a greater leaf area can typically absorb more light and carbon dioxide than a smaller plant and therefore will likely gain a greater weight during the same period (Fasoula & Tollenaar 2005 Maydica 50:39).

The ability to increase plant yield would have many applications in areas such as agriculture, including in the production of ornamental plants, arboriculture, horticulture and forestry. Increasing yield may also find use in the production of algae for use in bioreactors (for the biotechnological production of substances such as pharmaceuticals, antibodies or vaccines, or for the bioconversion of organic waste) and other such areas.

Plant breeders are often interested in improving specific aspects of yield depending on the crop or plant in question, and the part of that plant or crop which is of economic value. For example, for certain crops, or for certain end uses, a plant breeder may look specifically for improvements in plant biomass (weight) of one or more parts of a plant, which may include aboveground (harvestable) parts and/or (harvestable) parts below ground. This is particularly relevant where the aboveground parts or below ground parts of a plant are for consumption. For many crops, particularly cereals, it is an improvement in seed yield that is highly desirable. Increased seed yield may manifest itself in many ways, with each individual aspect of seed yield being of varying importance to a plant breeder depending on the crop or plant in question and its end use.

It would be of great advantage to a plant breeder to be able to be able to pick and choose the aspects of yield or seed yield to be altered. It would be highly desirable to be able to pick off the shelf, so to speak, a gene suitable for altering a particular aspect, or component, of seed yield. For example an increase in the fill rate, combined with increased thousand kernel weight would be highly desirable for a crop such as corn. For rice and wheat a combination of increased fill rate, harvest index and increased thousand kernel weight would be highly desirable.

It has now been found that modulating expression in a plant of a nucleic acid encoding a Class III Trehalose Phosphate Phosphatase (TPP) polypeptide gives plants having various enhanced yield-related traits.

Trehalose, a non-reducing disaccharide consisting of two glucose molecules linked via alpha-1,1 bonds, is found in several life kingdoms. In bacteria, fungi and insects, trehalose has been shown to have a carbohydrate storage function and to play a role in stress tolerance. In plants, trehalose was initially thought to be confined to extremophites, such as the resurrection plant Selaginella lepidophylla, however it is now widely accepted that trehalose metabolism is ubiquitous in the plant kingdom.

Trehalose is synthesized from UDP-glucose and Glucose-6-phosphate in two enzymatic reactions. In a first step, UDP-glucose and Glucose-6-phosphate are converted to UDP (uridine diphosphate) and alpha, alpha-trehalose 6-phosphate (T-6-P) by the enzyme TPS, trehalose phosphate synthase. In a second step, which is catalyzed by the enzyme TPP (trehalose phosphate phosphatase), T-6-P is de-phosphorylated to produce trehalose and orthophosphate.

In yeast, the two enzymatic activities (TPS and TPP activity) reside in a large protein complex, containing the active subunits, TPS1 and TPS2, and the regulatory subunits, with TPS1 having TPS activity and TPS2 having TPP activity. In *E. coli,* the two enzymatic activities are found in separate protein complexes. In plants, the protein complex has not been characterized to date.

In *Arabidopsis thaliana,* trehalose biosynthetic enzymes have been classified into three classes:
**Class I:** containing four genes, AtTPS1 to AtTPS4 having high similarity to ScTPS1;
**Class II:** having seven members, AtTPS5 to AtTPS11, with high sequence similarity to ScTPS2; and
**Class III:,** containing 10 members, AtTPPA to AtTPPJ, encoding proteins with similarity to *E*. *coli* TPS2 and the C-terminus of ScTPS2 proteins.

Genes encoding proteins within these classes are also present in other plant species.

Within Class I and Class II, enzymatic activity has only been unambiguously determined for AtTPS1, which displays TPS activity (Blazquez et al. Plant J. 1998 Mar;13(5):685-9.). Surprisingly, no TPP activity has been reported to date for any of the other Class II TPS proteins. In contrast, TPP activity was previously described for A*t*TPPA and A*t*TPPB, two of the members of Class III (Vogel et al. Plant J. 1998 Mar;13(5):673-83). Plant Class III TPPs contain two phosphatase consensus sequence motifs found in all TPP enzymes described to date (Thaller et al. Protein Sci. 1998 Jul;7(7):1647-52).

The genetic manipulation of trehalose biosynthesis genes has been reported to lead to improved stress tolerance in plants, as well as causing striking developmental alterations. Overexpression of *E.coli* OtsA and OtsB genes in transgenic tobacco and potato plants was reported to cause developmental aberrations in roots and leaves, and plant stunting. Fewer seeds were produced in the OtsA transgenic tobacco plants, and the OtsB transgenic potato plants did not produced tubers (Goddijn et al. Plant Physiol. 1997 Jan;113(1):181-90). Similar results have been described by others (Holmstrom et al. Nature, 379, 683-684; Romero et al. Planta, 201, 293-297; Pilont-Smits et al. 1998; J Plant Physiol. 152:525-532; Schluepmann et al. Proc Natl Acad Sci USA. 2003;100(11):6849-54). Mutants defective in TPS and TPP genes have also reportedly shown developmental defects. TPS1 knock out mutants in *Arabidopsis* showed impaired embryo development *(*Eastmond et al. Plant J. 2002 Jan; 29(2):225-35*).*

US20040229364 discloses nucleic acids encoding inter alia trehalose 6-phosphate phosphatases. The document is silent about seed yield.

WO97/42326 discloses that the carbon flow through the glycolysis can be stimulated by a decrease in the intracellular level of trehalose 6-phosphate-phosphatases.

WO2006/060376 discloses the down regulation of an endogenous trehalose 6-phosphate•phosphatase gene in maize.

Published US patent application, US 20060191040, in the name of Jackson et al. mentions the isolation and characterization of a maize gene, RAMOSA3 (RA3), reported to be responsible for meristem development and inflorescence development including branching. It is suggested that the gene, gene product, and regulatory regions may be used to manipulate branching, meristem growth, inflorescence development and arrangement, and ultimately to improve yield of plants. Although altered architecture (due to a change in branching or a change in the inflorescence structure) can in some cases contribute to increased yield or increased seed yield, this is by no means certain. There are many other components which would need to be in place before an increase in yield or seed yield could be realised. For example, without seed set, seed filling, fertility of a plant etc. there would be no increase in seed yield. Furthermore, Jackson *et al.* does not mention which aspects of yield may be improved, whether that be biomass of specific parts of a plant, improved seed yield (which could be seed size, seed number, thousand kernel weight, harvest index, or other seed yield-related parameters).

It was therefore surprising to find that modulating expression in a plant of a Class III TPP polynucleotide gives plants having various enhanced yield-related traits, particularly increased seed yield relative to control plants.

Accordingly, the present invention relates to a method for increasing seed yield in plants relative to control plants,
wherein said increased seed yield is any one or more of the following:
(i) Increased number of seeds per plant;
(ii) Increased number of filled seeds per plant;
(iii) Increased seed weight per plant;
comprising increasing expression in a plant of a nucleic acid encoding a Class III Trehalose Phosphate Phosphatase (TPP) polypeptide, wherein said increased expression is effected by introducing and expressing in a plant a nucleic acid encoding a Class III TPP polypeptide, wherein said a nucleic acid encodes a Class III TPP polypeptide having at least 85% sequence identity to the amino acid sequence given in SEQ ID NO: 4, or
wherein said Class III TPP polypeptide comprises:
(i) a Trehalose-PPase domain having at least 50% amino acid sequence identity to SEQ ID NO: 93 or SEQ ID NO: 94, wherein said Trehalose-PPase domain comprises:
   a. a Phosphatase Box A having at least 50% sequence identity to SEQ ID NO: 96; and/or
   b. a Phosphatase Box B having at least 50% sequence identity to SEQ ID NO: 97; and/or
(ii) a Serine-rich domain having at least 50% sequence identity to SEQ ID NO: 95,
wherein said control plants are corresponding wild-type plants or corresponding plants without said nucleic acid encoding said class III trehalose phosphate polypeptide.

Moreover, the present invention relates to an isolated nucleic acid molecule comprising:
(i) a nucleic acid of SEQ ID NO: 3;
(ii) the complement of the SEQ ID NO given in (i);
(iii) a nucleic acid encoding a Class III TPP protein having at least 90% sequence identity to the amino acid sequence given in SEQ ID NO: 4,
(iv)

Further, the present invention relates to an isolated polypeptide comprising:
(i) an amino acid sequence of SEQ ID NO: 4;
(ii) an amino acid sequence having at least 90% sequence identity to the amino acid sequence given in SEQ ID NO: 4;

In addition, the present relates to a construct comprising:
(a) a nucleic acid encoding a Class III TPP polypeptide according to claim 6; and
(b) one or more control sequences capable of driving expression of the nucleic acid sequence of (a), wherein said one control sequence is a GOS2 promoter; and
(c) optionally a transcription termination sequence.

Further, the present invention relates to the use of said construct in a method for making plants having increased seed yield relative to control plants, wherein said seed yield is one or more of the following:
(i) Increased number of seeds per plant;
(ii) Increased number of filled seeds per plant;
(iii) Increased (total) seed weight per plant,
wherein said control plants are corresponding wild-type plants or corresponding plants without said nucleic acid encoding said class III trehalose phosphate polypeptide.

Also envisaged is a plant, plant part or plant cell transformed with said construct, wherein said construct is integrated into the genome of the plant, plant part or plant cell.

Further, the present invention relates to a method for the production of a transgenic plant having increased seed yield,
wherein said seed yield is one or more of the following:
(i) Increased number of seeds per plant;
(ii) Increased number of filled seeds per plant;
(iii) Increased (total) seed weight per plant.
comprising:
(i) Introducing and expressing in a plant a nucleic acid encoding a Class III TPP protein according to claim 1 or 6 and
(ii) cultivating the plant cell under conditions promoting plant growth and development.

Also envisaged is a transgenic plant having increased seed yield relative to control plants, said increased seed yield resulting from increased expression of an introduced nucleic acid encoding a Class III TPP protein as defined in claim 6, or a transgenic plant cell obtainable from said transgenic plant, wherein said transgenic plant is a crop plant or a monocot or a cereal, such as rice, maize, wheat, barley, millet, rye, sorghum and oats, or a transgenic plant cell derived from said transgenic plant, wherein said control plants are corresponding wild-type plants or corresponding plants without said nucleic acid encoding said class III trehalose phosphate polypeptide.

Furthermore, the present invention relates to the use of a nucleic acid encoding a Class III TPP protein according to claim 1 or 6 in increasing seed yield in plants relative to control plants when introduced and expressed in said plants, wherein said seed yield is one or more of the following:
(i) Increased number of seeds per plant;
(ii) Increased number of filled seeds per plant;
(iii) Increased (total) seed weight per plant,
wherein said control plants are corresponding wild-type plants or corresponding plants without said nucleic acid encoding said class III trehalose phosphate polypeptide.

Any reference hereinafter to a "protein useful in the methods of the invention" is taken to mean a Class III TPP polypeptide as in the claims. Any reference hereinafter to a "nucleic acid useful in the methods of the invention" is taken to mean a nucleic acid capable of encoding such a Class III TPP polypeptide. The terms "polypeptide" and "protein" are used interchangeably herein and refer to amino acids in a polymeric form of any length. The terms "polynucleotide(s)", "nucleic acid sequence(s)", "nucleotide sequence(s)", "polynucleotide molecule(s)" are used interchangeably herein and refer to nucleotides, either ribonucleotides or deoxyribonucleotides or a combination of both, in a polymeric form of any length.

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. A "control plant" as used herein refers not only to whole plants, but also to plant parts, including seeds and seed parts.

A preferred method for increasing expression of a nucleic acid encoding a protein useful in the methods of the invention is by introducing and expressing in a plant a nucleic acid encoding a protein useful in the methods of the invention as defined below.

The nucleic acid to be introduced into a plant (and therefore useful in performing the methods of the invention) is any nucleic acid encoding the type of protein, which will now be described, herein also named "Class III TPP nucleic acid" or "Class III TPP gene" or a "Class III TPP polynucleotide".

A "Class III TPP polypeptide" as disclosed herein refers to any polypeptide with trehalose-6-phosphate phosphatase activity comprising at least one Trehalose-phosphatase (Trehalose-PPase) domain.

Trehalose-PPase domains are typically between 200 and 250 amino acids in length and typically comprise a phosphatase consensus sequence motif that is found in all TPP enzymes described to date (Thaller et al. 1998). A representative consensus sequence for the Trehalose-PPase domain is given in SEQ ID NO: 93. The amino acid sequence for the Trehalose-PPase domain comprised in SEQ ID NO: 2 is given in SEQ ID NO: 94. A person skilled in the art will readily be able to identify the presence of a Trehalose-PPase domain using tools and techniques known in the art. Examples 2, 3 and 4 herein provide further details concerning the identification of a Trehalose-PPase domain. In Class III TPP polypeptides, this phosphatase consensus sequence motif typically comprises two phosphatase boxes, named A and B-Phosphatase Box. SEQ ID NO: 96 represents a consensus sequence for Phosphatase box A and SEQ ID NO: 97 represents a consensus sequence for Phosphatase box B.

Nucleic acids useful in the methods of the invention encode Class III TPP polypeptides comprising at least one Trehalose-PPase domain, which domain preferably has, in increasing order of preference, at least 50%, 55%, 60%, 65%, 70%, 75%, 80%, 85%, 90%, 95% or 98% or more amino acid sequence identity to either SEQ ID NO: 93 or SEQ ID NO: 94. Further preferably, the Trehalose-PPase domain comprises at least one (preferably two) phosphatase box(es) having, in increasing order of preference, at least 50%, 55%, 60%, 65%, 70%, 75%, 80%, 85%, 90%, 95% or 98% or more sequence identity to the phosphatase boxes or either SEQ ID NO: 96 or SEQ ID NO: 97.

Class III TPP polypeptides may also comprise a serine-rich region, typically located in the N-terminus of the Trehalose-PPase domain. SEQ ID NO: 95 represents a consensus sequence for the serine-rich domain. Preferably, nucleic acids useful in the methods of the invention encode Class III TPP polypeptides comprising a serine-rich domain having, in increasing order of preference, at least 50%, 55%, 60%, 65%, 70%, 75%, 80%, 85%, 90%, 95% or 98% or more sequence identity to SEQ ID NO: 95.

Preferably, the nucleic acid to be introduced into a plant encodes a Class III TPP protein having, in increasing order of preference, at least 90%, 95%, 96%, 97%, 98%, 99% or 100% sequence identity to SEQ ID NO 4. Most preferably, the Class III TPP nucleic acid is as represented by SEQ ID NO 3.

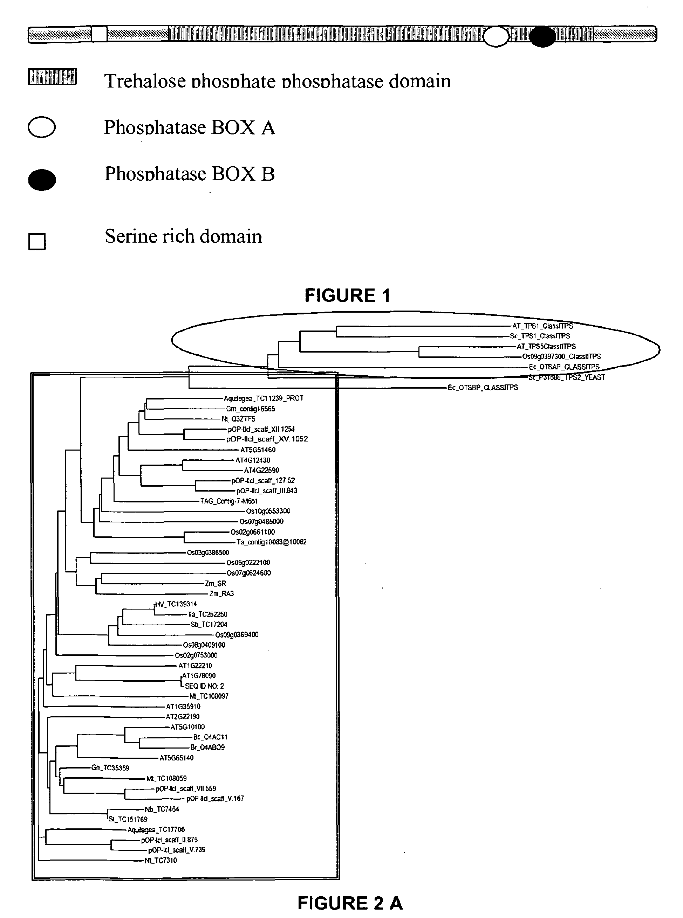

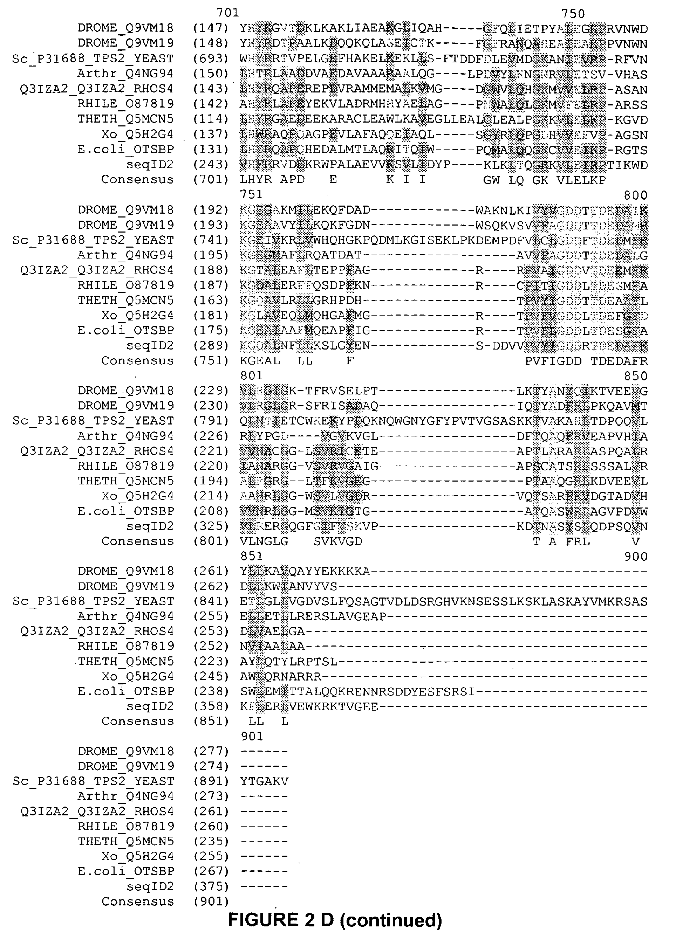

Typically, a Class III TPP polypeptide, when used in the construction of a TPP/TPS phylogenetic tree, such as the one depicted in Fig. 2A, tends to cluster with the group of Class III TPP proteins comprising the amino acid sequence represented by SEQ ID NO: 2 rather than with any other group.

Examples of proteins useful in the methods of the invention and nucleic acids encoding the same are provided herein in Table A of Example 1.

Also useful in the disclosed methods are homologues of any of the amino acid sequences given in Table A of Example 1. "Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG^{®}-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α-helical structures or β-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company and Table 1 below).

**Table 1: Examples of conserved amino acid substitutions**

| **Residue** | **Conservative Substitutions** | **Residue** | **Conservative Substitutions** |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen *in vitro* mutagenesis (USB, Cleveland, OH), QuickChange Site Directed mutagenesis (Stratagene, San Diego, CA), PCR-mediated site-directed mutagenesis or other site-directed mutagenesis protocols.

Also useful in the methods of the invention are derivatives of any one of the polypeptides given in Table A of Example 1 or orthologues or paralogues of any of the polypeptides given in Table A of Example 1 or derivatives of any orthologues or paralogues of any of the polypeptides given in Table A. "Derivatives" include peptides, oligopeptides, polypeptides which may, compared to the amino acid sequence of the naturally-occurring form of the protein, such as the one presented in SEQ ID NO: 2, comprise substitutions of amino acids with non-naturally occurring amino acid residues, or additions of non-naturally occurring amino acid residues. Derivatives of the polypeptides given in Table A of Example 1 are further examples which may be suitable for use in the methods of the invention. Derivatives useful in the methods of the present invention preferably have similar biological and functional activity as the unmodified protein from which they are derived.

"Derivatives" of a polypeptide include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

The invention is illustrated by transforming plants with the *Arabidopsis thaliana* nucleic acid sequence represented by SEQ ID NO: 1, encoding the polypeptide sequence of SEQ ID NO: 2, however performance of the invention is not restricted to these sequences. The methods of the invention may advantageously be performed using any nucleic acid encoding a protein useful in the methods of the invention as defined herein, including nucleic acids encoding orthologues, paralogues and homologues of SEQ ID NO: 2, such as (but not limited to) any of the nucleic acid sequences given in Table A of Example 1.

The amino acid sequences given in Table A of Example 1 are examples of orthologues and paralogues of the Class III TPP polypeptide represented by SEQ ID NO: 2, and nucleic acids encoding the same are useful in the methods of the invention. Orthologues and paralogues encompass evolutionary concepts used to describe the ancestral relationships of genes. Paralogues are genes within the same species that have originated through duplication of an ancestral gene and orthologues are genes from different organisms that have originated through speciation, and are also derived from a common ancestral gene.

Orthologues and paralogues may easily be found by performing a so-called reciprocal blast search. Typically, this involves a first BLAST involving BLASTing a query sequence (for example using any of the sequences listed in Table A of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 1 or SEQ ID NO: 2, the second BLAST would therefore be against *Arabidopsis thaliana* sequences). The results of the first and second BLASTs are then compared. A paralogue is identified if a high-ranking hit from the first blast is from the same species as from which the query sequence is derived, a BLAST back then ideally results in the query sequence amongst the highest hit; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Table A of Example 1 gives examples of orthologues and paralogues of the Class III TPP protein represented by SEQ ID NO 2. Further orthologues and paralogues may readily be identified using the BLAST procedure described above.

The proteins of the disclosure are identifiable by the presence of the conserved Trehalose-PPase domain(s) (shown in Figure 1 and Example 4). The term "domain" refers to a set of amino acids conserved at specific positions along an alignment of sequences of evolutionarily related proteins. While amino acids at other positions can vary between homologues, amino acids that are highly conserved at specific positions indicate amino acids that are essential in the structure, stability or activity of a protein. Identified by their high degree of conservation in aligned sequences of a family of protein homologues, they can be used as identifiers to determine if any polypeptide in question belongs to a previously identified polypeptide family (in this case, the proteins useful in the methods of the invention and nucleic acids encoding the same as defined herein).

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Specialist databases also exist for the identification of domains, for example, SMART (Schultz et al. (1998) Proc. Natl. Acad. Sci. USA 95, 5857-5864; Letunic et al. (2002) Nucleic Acids Res 30, 242-244, InterPro (Mulder et al., (2003) Nucl. Acids. Res. 31, 315-318, Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nucl. Acids. Res. 32:D134-D137. (2004), or Pfam (Bateman et al., Nucleic Acids Research 30(1): 276-280 (2002). A set of tools for *in silico* analysis of protein sequences is available on the ExPASY proteomics server (hosted by the Swiss Institute of Bioinformatics (Gasteiger et al., ExPASy: the proteomics server for in-depth protein knowledge and analysis, Nucleic Acids Res. 31:3784-3788(2003)).

Domains may also be identified using routine techniques, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains (such as the Trehalose-PPase domain, or one of the motifs defined above) may be used as well. The sequence identity values, which are indicated in Example 3 as a percentage, were determined over the entire nucleic acid or amino acid sequence and over selected domains or conserved motif(s), using the programs mentioned above using the default parameters.

Furthermore, Class III TPP proteins (at least in their native form) typically have trehalose phosphatase activity. Polypeptides with trehalose phosphatase activity belong to the enzymatic class of EC:3.1.3.12, according to the classification of the Enzyme Commission of the Nomenclature Committee of the International Union of Biochemistry and Molecular Biology (NC-IUBMB). Enzymes in class EC:3.1.3.12 catalyze the reaction: trehalose-6-phosphate + H₂O = trehalose + phosphate.

The activity of a trehalose phosphatase protein may be measured by determining the levels of the substrate processed and the levels of product accumulated in an *in vitro* reaction, that is, by determining the level of trehalose-6-phosphate and trehalose accumulation from the reaction. Enzymatic methods to measure trehalose can be based on hydrolyzing trehalose to glucose, such as those described by Van Dijck et al. Biochem J. 2002 Aug 15;366(Pt 1):63-71 and Zentella et al. Plant Physiol. 1999 Apr; 119(4): 1473-82.

Trehalose-6-phosphate levels may also be measured by HPLC (High Performance Liquid Chromatography) methods as described by Avonce et al. Plant Physiol. 2004 Nov;136(3):3649-59; Schluepmann et al. 2003. Alternative methods based on determining the release of inorganic phosphate from trehalose-6-phosphate have also been described Klutts et al. J Biol Chem. 2003 Jan 24;278(4):2093-100. An alternative method to determine trehalose-6-phosphate levels using liquid chromatography coupled to MS-Q3 (triple quadrupole MS) has been described by Lunn et al. Biochem J. 2006 Jul 1;397(1):139-48. Further details are provided in Example 5 and Example 6.

Examples of nucleic acids suitable for use in performing the methods of the invention include the nucleic acid sequences given in Table A of Example 1, but are not limited to those sequences. Nucleic acid variants may also be useful in practising the methods of the invention. Examples of such nucleic acid variants include portions of nucleic acids encoding a protein useful in the methods of the invention, nucleic acids hybridising to nucleic acids encoding a protein useful in the methods of the invention, splice variants of nucleic acids encoding a protein useful in the methods of the invention, allelic variants of nucleic acids encoding a protein useful in the methods of the invention and variants of nucleic acids encoding a protein useful in the methods of the invention that are obtained by gene shuffling. The terms portion, hybridising sequence, splice variant, allelic variant and gene shuffling will now be described.

Nucleic acids encoding proteins useful in the methods of the disclosure need not be full-length nucleic acids, since performance of the methods of the disclosure does not rely on the use of full-length nucleic acid sequences. Portions useful in the methods of the disclosure, encode a polypeptide falling within the definition of a nucleic acid encoding a protein useful in the methods of the disclosure as defined herein and having substantially the same biological activity as the amino acid sequences given in Table A of Example 1. Preferably, the portion is a portion of any one of the nucleic acids given in Table A of Example 1. The portion is typically at least 625 consecutive nucleotides in length, preferably at least 825 consecutive nucleotides in length, more preferably at least 1025 consecutive nucleotides in length and most preferably at least 1125 consecutive nucleotides in length, the consecutive nucleotides being of any one of the nucleic acid sequences given in Table A of Example 1. Most preferably the portion is a portion of the nucleic acid of SEQ ID NO: 1. Preferably, the portion encodes an amino acid sequence which when used in the construction of a TPP/TPS phylogenetic tree, such as the one depicted in Fig. 2A, tends to cluster with the group of Class III TPP proteins comprising the amino acid sequence represented by SEQ ID NO: 2 rather than with any other group.

A portion of a nucleic acid encoding a Class III TPP protein as defined herein may be prepared, for example, by making one or more deletions to the nucleic acid. The portions may be used in isolated form or they may be fused to other coding (or non coding) sequences in order to, for example, produce a protein that combines several activities. When fused to other coding sequences, the resultant polypeptide produced upon translation may be bigger than that predicted for the Class III TPP protein portion.

According to the present disclosure, there is provided a method for enhancing yield-related traits in plants, particularly increasing seed yield, comprising introducing and expressing in a plant a portion of any one of the nucleic acid sequences given in Table A of Example 1, or a portion of a nucleic acid encoding an orthologue, paralogue or homologue of any of the amino acid sequences given in Table A of Example 1.

Another nucleic acid variant useful in the methods of the invention is a nucleic acid capable of hybridising under reduced stringency conditions, preferably under stringent conditions, with a nucleic acid encoding a Class III TPP protein as defined herein, or with a portion as defined herein.

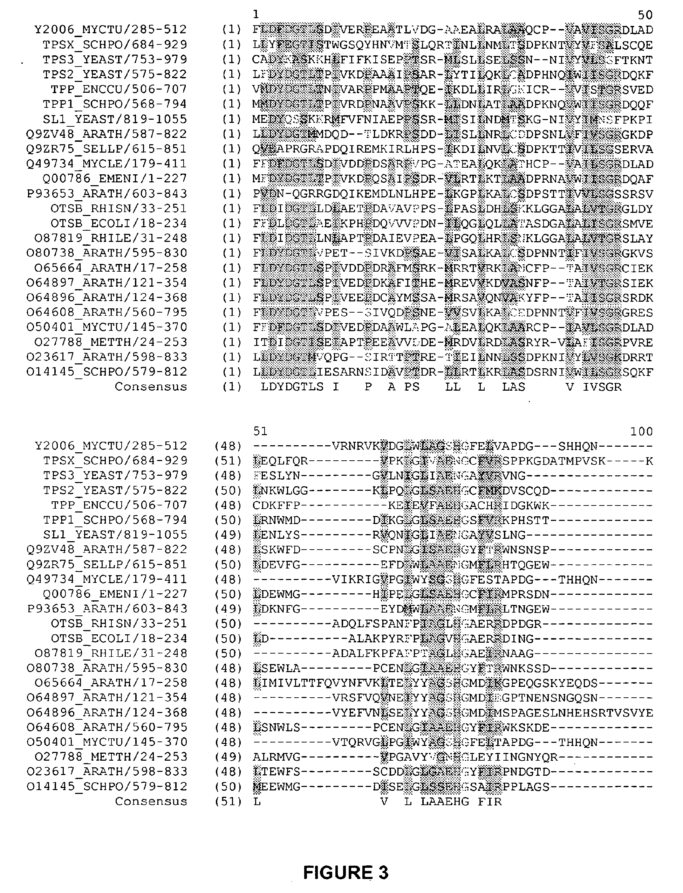

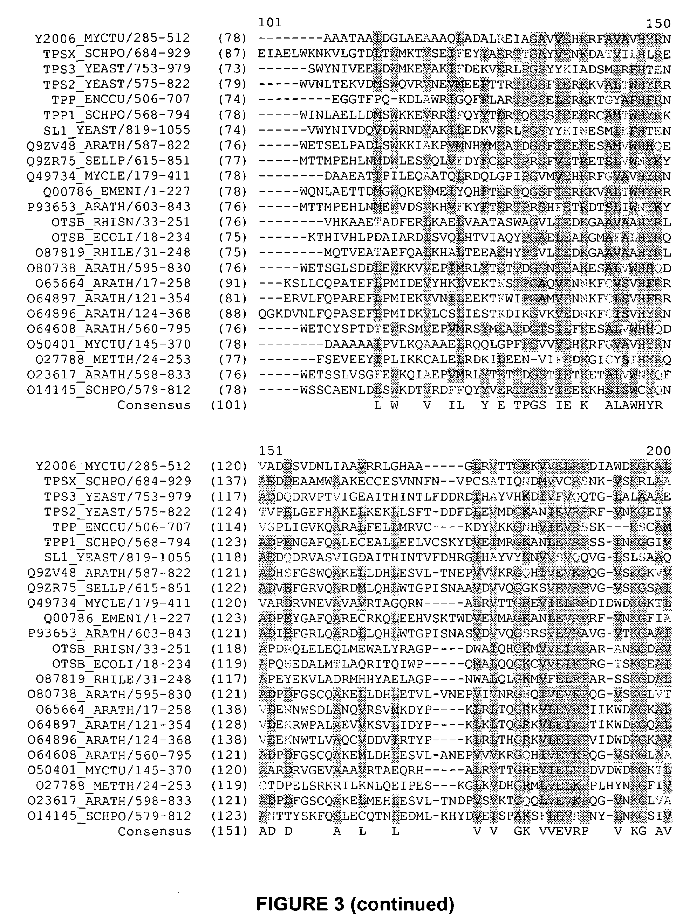

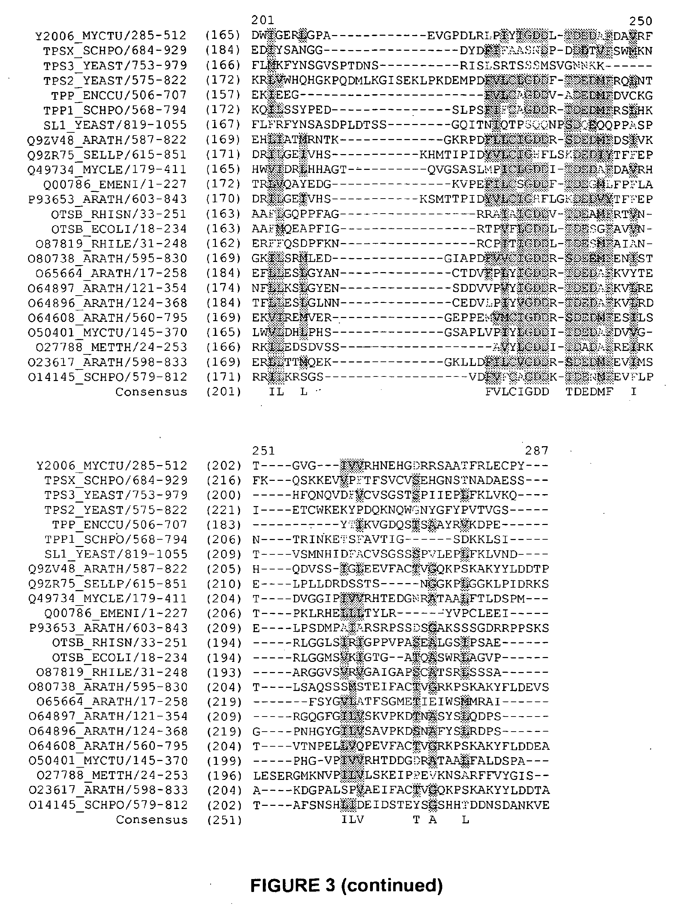

Hybridising sequences useful in the methods of the invention, encode a polypeptide having a Trehalose-PPAse domain (see the alignment of Fig. 2A and Fig. 3) and having substantially the same biological activity as Class III TPP proteins represented by any of the amino acid sequences given in Table A of Example 1. The hybridising sequence is typically at least 625 consecutive nucleotides in length, preferably at least 825 consecutive nucleotides in length, more preferably at least 1025 consecutive nucleotides in length and most preferably at least 1125 consecutive nucleotides in length, the consecutive nucleotides being of any one of the nucleic acid sequences given in Table A of Example 1. Preferably, the hybridising sequence is one that is capable of hybridising to any of the nucleic acids given in Table A of Example 1, or to a portion of any of these sequences, a portion being as defined above. Most preferably, the hybridising sequence is capable of hybridising to a nucleic acid as represented by SEQ ID NO: 1 or to a portion thereof.

Preferably, the hybridising sequence encodes an amino acid sequence which when used in the construction of a TPP/TPS phylogenetic tree, such as the one depicted in Fig. 2A, tends to cluster with the group of Class III TPP proteins comprising the amino acid sequence represented by SEQ ID NO: 2 rather than with any other group.

Most preferably, the isolated polynucleotide molecule is capable of hybridising under stringent conditions to a sequence represented by one of SEQ ID NOs 4, 6 and 8.

According to the present invention, there is provided a method for enhancing yield-related traits in plants, particularly increasing seed yield, comprising introducing and expressing in a plant a nucleic acid capable of hybridizing to any one of the nucleic acids given in Table A of Example 1, or comprising introducing and expressing in a plant a nucleic acid capable of hybridising to a nucleic acid encoding an orthologue, paralogue or homologue of any of the nucleic acid sequences given in Table A of Example 1.

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point (Tₘ) for the specific sequence at a defined ionic strength and pH. Medium stringency conditions are when the temperature is 20°C below Tₘ, and high stringency conditions are when the temperature is 10°C below Tₘ. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tₘ is the temperature under defined ionic strength and pH, at which 50% of the target sequence hybridises to a perfectly matched probe. The Tₘ is dependent upon the solution conditions and the base composition and length of the probe. For example, longer sequences hybridise specifically at higher temperatures. The maximum rate of hybridisation is obtained from about 16°C up to 32°C below Tₘ. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tₘ decreases about 1°C per % base mismatch. The Tₘ may be calculated using the following equations, depending on the types of hybrids:
1) DNA-DNA hybrids (Meinkoth and Wahl, Anal. Biochem., 138: 267-284, 1984):
   81.5°C + 16.6xlog₁₀[Na+]^{a} + 0.41x%[G/C^{b}] - 500x[L^{c}]⁻¹ - 0.61x% formamide
2) DNA-RNA or RNA-RNA hybrids:
   Tm= 79.8 + 18.5 (log₁₀[Na+]^{a}) + 0.58 (%G/C^{b}) + 11.8 (%G/C^{b})² - 820/L^{c}
3) oligo-DNA or oligo-RNA^{d} hybrids:
   For <20 nucleotides: Tm= 2 (lₙ)
   For 20-35 nucleotides: Tm= 22 + 1.46 (lₙ)

^{a} or for other monovalent cation, but only accurate in the 0.01-0.4 M range.
^{b} only accurate for %GC in the 30% to 75% range.
^{c} L = length of duplex in base pairs.
^{d} Oligo, oligonucleotide; lₙ, effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected.

The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1 x SSC or at 42°C in 1 x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5 × Denhardt's reagent, 0.5-1.0% SDS, 100 µg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition Cold Spring Harbor Laboratory Press, CSH, New York or to Current Protocols in Molecular Biology, John Wiley & Sons, N.Y. (1989 and yearly updates).

Another nucleic acid variant useful in the methods of the disclosure is a splice variant encoding a Class III TPP protein as defined hereinabove. The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex, BMC Bioinformatics. 2005; 6: 25).

According to the present disclosure, there is provided a method for enhancing yield-related traits in plants, particularly increasing seed yield, comprising introducing and expressing in a plant a splice variant of any one of the nucleic acid sequences given in Table A of Example 1, or a splice variant of a nucleic acid encoding an orthologue, paralogue or homologue of any of the amino acid sequences given in Table A of Example 1.

Preferred splice variants are splice variants of a nucleic acid represented by SEQ ID NO: 1 or a splice variant of a nucleic acid encoding an orthologue or paralogue of SEQ ID NO: 2. Preferably, the amino acid sequence encoded by the splice variant comprises any one or more of the motifs or domains as defined herein. Preferably, the amino acid sequence encoded by the splice variant, when used in the construction of a TPP/TPS phylogenetic tree, such as the one depicted in Fig. 2A, tends to cluster with the group of Class III TPP proteins comprising the amino acid sequence represented by SEQ ID NO: 2 rather than with any other group.

Another nucleic acid variant useful in performing the methods of the disclosure is an allelic variant of a nucleic acid encoding a Class III TPP protein as defined hereinabove. Alleles or allelic variants are alternative forms of a given gene, located at the same chromosomal position. Allelic variants exist in nature, and encompassed within the methods of the present disclosure is the use of these natural alleles. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms. The allelic variants useful in the methods of the present disclosure have substantially the same biological activity as the Class III TPP protein of SEQ ID NO: 2.

According to the present disclosure, there is provided a method for enhancing yield-related traits in plants, particularly increasing seed yield, comprising introducing and expressing in a plant an allelic variant of any one of the nucleic acids given in Table A of Example 1, or comprising introducing and expressing in a plant an allelic variant of a nucleic acid encoding an orthologue, paralogue or homologue of any of the amino acid sequences given in Table A of Example 1.

Preferably, the allelic variant is an allelic variant of SEQ ID NO: 1 or an allelic variant of a nucleic acid encoding an orthologue or paralogue of SEQ ID NO: 2. Preferably, the amino acid sequence encoded by the allelic variant comprises any one or more of the motifs or domains as defined herein. Preferably, the amino acid sequence encoded by the allelic variant, when used in the construction of a TPS/TPP phylogenetic tree, such as the one depicted in Fig. 2A, tends to cluster with the group of Class III TPP proteins comprising the amino acid sequence represented by SEQ ID NO: 2, rather than with any other group.

A further nucleic acid variant useful in the methods of the disclosure is a nucleic acid variant obtained by gene shuffling. Gene shuffling or directed evolution may also be used to generate variants of nucleic acids encoding Class III TPP proteins as defined above. This consists of iterations of DNA shuffling followed by appropriate screening and/or selection to generate variants of nucleic acids or portions thereof encoding Class III TPP proteins as defined above having a modified biological activity (Castle et al., (2004) Science 304(5674): 1151-4; US patents 5,811,238 and 6,395,547).

According to the present disclosure, there is provided a method for enhancing yield-related traits in plants, comprising introducing and expressing in a plant a variant of any one of the nucleic acid sequences given in Table A of Example 1, or comprising introducing and expressing in a plant a variant of a nucleic acid encoding an orthologue, paralogue or homologue of any of the amino acid sequences given in Table A of Example 1, which variant nucleic acid is obtained by gene shuffling.

Preferably, the variant nucleic acid obtained by gene shuffling encodes an amino acid sequence comprising any one or more of the motifs or domains as defined herein. Preferably, the amino acid encoded sequence by the variant nucleic acid obtained by gene shuffling, when used in the construction of a TPS/TPP phylogenetic tree, such as the one depicted in Fig. 2A, tends to cluster with the group of Class III TPP proteins comprising the amino acid sequence represented by SEQ ID NO: 2 rather than with any other group.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding Class III TPP proteins may be derived from any natural or artificial source. The nucleic acid may be modified from its native form in composition and/or genomic environment through deliberate human manipulation. Preferably the Class III TPP-encoding nucleic acid is from a plant, further preferably from a dicotyledonous plant, more preferably from the Brassicaceae family, most preferably the nucleic acid is from *Arabidopsis thaliana.*

Any reference herein to a Class III TPP protein is therefore taken to mean a Class III TPP protein as defined above. Any nucleic acid encoding such a Class III TPP protein is suitable for use in performing the methods of the disclosure.

The present disclosure also encompasses plants or parts thereof (including seeds) obtainable by the methods according to the present disclosure. The plants or parts thereof comprise a nucleic acid transgene encoding a Class III TPP protein as defined above.

The invention also provides hitherto unknown Class III TPP nucleic acid sequences and Class III TPP protein sequences. These sequences also being useful in performing the methods of the invention.

According to a further embodiment of the present invention, there is therefore provided an isolated nucleic acid molecule comprising:
(i) a nucleic acid represented by SEQ ID NO: 3;
(ii) the complement of any one of the SEQ ID NOs given in (i);
(iii) a nucleic acid encoding a Class III TPP protein having, in increasing order of preference, at least 90%, 95%, 96%, 97%, 98%, 99% or 100% sequence identity to the amino acid sequence given in SEQ ID NO: 4.

According to a further embodiment of the present invention, there is also provided an isolated polypeptide comprising:
(i) an amino acid sequence represented by SEQ ID NO: 4;
(ii) an amino acid sequence having, in increasing order of preference, at least 90%, 95%, 96%, 97%, 98%, 99% or 100% sequence identity to the amino acid sequence given in SEQ ID NO: 4.

The invention also provides genetic constructs and vectors to facilitate introduction and/or expression of the nucleic acid sequences useful in the methods according to the invention, in a plant. The gene constructs may be inserted into vectors, which may be commercially available, suitable for transforming into plants and suitable for expression of the gene of interest in transformed cells. The invention also provides use of a gene construct as defined herein in the methods of the invention.

More specifically, the present invention provides a construct comprising:
(a) nucleic acid encoding Class III TPP protein as defined above;
(b) one or more control sequences capable of driving expression of the nucleic acid sequence of (a), wherein said one control sequence is a GOS2 promoter; and optionally
(c) a transcription termination sequence.

The nucleic acid in the construct according to the invention is a polynucleotide molecule encoding a Class III TPP protein with an amino acid sequence in increasing order of preference of at least 90%, 95%, 96%, 97%, 98%, 99% or 100% sequence identity to a sequence represented by SEQ ID NO: 4. Most preferably, the Class III TPP polynucleotide molecule is the nucleotide sequence of SEQ ID NO: 3.

Plants are transformed with a vector comprising the sequence of interest (i.e., a nucleic acid encoding a Class III TPP polypeptide as defined herein. The skilled artisan is well aware of the genetic elements that must be present on the vector in order to successfully transform, select and propagate host cells containing the sequence of interest. The sequence of interest is operably linked to one or more control sequences (at least to a promoter). The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ. The term "operably linked" as used herein refers to a functional linkage between the promoter sequence and the gene of interest, such that the promoter sequence is able to initiate transcription of the gene of interest.

Advantageously, any type of promoter may be used to drive expression of the nucleic acid sequence. The term "promoter" refers to a nucleic acid control sequence located upstream from the transcriptional start of a gene and which is involved in recognising and binding of RNA polymerase and other proteins, thereby directing transcription of an operably linked nucleic acid. A "plant" promoter comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule is operably to a suitable promoter.

The promoter may be a constitutive promoter, which refers to a promoter that is transcriptionally active during most, but not necessarily all, phases of its growth and development and under most environmental conditions, in at least one cell, tissue or organ. Alternatively, the promoter may be an inducible promoter, i.e. having induced or increased transcription initiation in response to a chemical (for a review see Gatz 1997, Annu. Rev. Plant Physiol. Plant Mol. Biol., 48:89-108), environmental or physical stimulus. Another example of an inducible promoter is a stress-inducible promoter, i.e. a promoter activated when a plant is exposed to various stress conditions, or a pathogen-induced promoter.

Additionally or alternatively, the promoter may be an organ-specific or tissue-specific promoter, i.e. one that is capable of preferentially initiating transcription in certain organs or tissues, such as the leaves, roots, seed tissue etc.; or the promoter may be a ubiquitous promoter, which is active in substantially all tissues or cells of an organism, or the promoter may be developmentally regulated, thereby being active during certain developmental stages or in parts of the plant that undergo developmental changes. Promoters able to initiate transcription in certain organs or tissues only are referred to herein as "organ-specific" or "tissue-specific" respectively, similarly, promoters able to initiate transcription in certain cells only are referred to herein as "cell-specific".

Preferably, the Class III TPP nucleic acid or variant thereof is operably linked to a constitutive promoter. A preferred constitutive promoter is one that is also substantially ubiquitously expressed. Further preferably the promoter is derived from a plant, more preferably from a monocotyledonous plant. Most preferred is the use of a GOS2 promoter, preferably a GOS2 promoter from rice, most preferably a promoter substantially as represented by SEQ ID NO: 98. It should be clear that the applicability of the present invention is not restricted to the Class III TPP nucleic acid represented by SEQ ID NO: 1, nor is the applicability of the invention restricted to expression of a Class III TPP nucleic acid when driven by a GOS2 promoter. According to another preferred feature of the invention, the constitutive promoter is a High Mobility Group Protein (HMGP) promoter, preferably a HMGP promoter from rice, more preferably substantially similar to SEQ ID NO: 131, most preferably identical to SEQ ID NO: 131. Examples of other constitutive promoters which may also be used to drive expression of a Class III TPP nucleic acid are shown in Table 2 below.

**Table 2: Examples of constitutive promoters**

| **Gene Source** | **Reference** |
|---|---|
| Actin | McElroy et al, Plant Cell, 2: 163-171, 1990 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

For the identification of functionally equivalent promoters, the promoter strength and/or expression pattern of a candidate promoter may be analysed for example by operably linking the promoter to a reporter gene and assay the expression level and pattern of the reporter gene in various tissues of the plant. Suitable well-known reporter genes include for example beta-glucuronidase or beta galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally, the term "weak promoter" refers to a promoter that drives expression of a coding sequence at a low level, levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1,000 transcripts per cell.

Optionally, one or more terminator sequences may be used in the construct introduced into a plant. The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene. Additional regulatory elements may include transcriptional as well as translational enhancers. Those skilled in the art will be aware of terminator and enhancer sequences that may be suitable for use in performing the invention. Such sequences would be known or may readily be obtained by a person skilled in the art.

An intron sequence may also be added to the 5' untranslated region (UTR) or in the coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg, Mol. Cell Biol. 8:4395-4405 (1988); Callis et al., Genes Dev. 1:1183-1200 (1987)). Such intron enhancement of gene expression is typically greatest when placed near the 5' end of the transcription unit. Use of the maize introns Adh1-S intron 1, 2, and 6, the Bronze-1 intron are known in the art. For general information, see The Maize Handbook, Chapter 116, Freeling and Walbot, Eds., Springer, N.Y. (1994).

Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. As used herein, the term "selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as *nptII* that phosphorylates neomycin and kanamycin, or *hpt,* phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example *bar* which provides resistance to Basta^{®}; *aroA* or *gox* providing resistance against glyphosate, or the genes conferring resistance to, for example, imidazolinone, phosphinothricin or sulfonylurea), or genes that provide a metabolic trait (such as *manA* that allows plants to use mannose as sole carbon source or xlose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with *Agrobacteria,* the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems, which have the advantage that elimination by crossing can be dispensed with. The best-known system of this type is what is known as the Cre/Iox system. Cre1 is a recombinase that removes the sequences located between the IoxP sequences. If the marker gene is integrated between the IoxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

The invention also provides a method for the production of transgenic plants having enhanced seed yield-related traits relative to control plants, comprising introduction and expression in a plant of any nucleic acid encoding a Class III TPP protein as defined in the claims. Said increased seed yield is any one or more of the following:
(i) Increased number of seeds per plant;
(ii) Increased number of filled seeds per plant;
(iii) Increased seed weight per plant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either
(a) the nucleic acid sequences encoding proteins useful in the methods of the invention, or
(b) genetic control sequence(s) which is operably linked with the nucleic acid sequence according to the invention, for example a promoter, or
(c) a) and b)
are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

More specifically, the present invention provides a method for the production of transgenic plants having increased yield, which method comprises:
(i) introducing and expressing in a plant, plant part or plant cell a *Class III TPP* nucleic acid as defined in the claims; and
(ii) cultivating the plant cell under conditions promoting plant growth and development.

Said seed yield is one or more of the following:
(i) Increased number of seeds per plant;
(ii) Increased number of filled seeds per plant;
(iii) Increased (total) seed weight per plant.

The nucleic acid may be introduced directly into a plant cell or into the plant itself (including introduction into a tissue, organ or any other part of a plant). According to a preferred feature of the present invention, the nucleic acid is preferably introduced into a plant by transformation.

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium-mediated* transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994). In the case of corn transformation, the preferred method is as described in either Ishida et al. (Nat. Biotechnol 14(6): 745-50, 1996) or Frame et al. (Plant Physiol 129(1): 13-22, 2002). Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis (Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known *inter alia* from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the centre of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ und Bent, AF (1998). The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant.

To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The disclosure also includes host cells containing an isolated nucleic acid encoding a Class III TPP protein as defined hereinabove. Preferred host cells according to the invention are plant cells.

Host plants for the nucleic acids or the vector used in the method according to the invention, the expression cassette or construct or vector are, in principle, advantageously all plants, which are capable of synthesizing the polypeptides used in the inventive method.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

The invention also extends to harvestable parts of a plant such as, but not limited to seeds, leaves, fruits, flowers, stems, rhizomes, tubers and bulbs. The harvestable parts comprise a construct as defined in claim 7. The disclosure furthermore relates to products derived, preferably directly derived, from a harvestable part of such a plant, such as dry pellets or powders, oil, fat and fatty acids, starch or proteins.

The modulated expression is increased expression. Methods for increasing expression of nucleic acids or genes, or gene products, are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression. For example, endogenous promoters may be altered *in vivo* by mutation, deletion, and/or substitution (see, Kmiec, U.S. Pat. No. 5,565,350; Zarling et al., PCT/US93/03868), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added as described above.

Other control sequences (besides promoter, enhancer, silencer, intron sequences, 3'UTR and/or 5'UTR regions, micro-RNA target sites, may be protein and/or RNA stabilizing elements.

As mentioned above, a preferred method for increasing expression of a nucleic acid encoding a Class III TPP protein is by introducing and expressing in a plant a nucleic acid encoding a Class III TPP protein; however the effects of performing the method, i.e. enhancing yield-related traits may also be achieved using other well known techniques. A description of some of these techniques will now follow.

One such technique is T-DNA activation tagging (Hayashi et al. Science (1992) 1350-1353), which involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

The effects of the invention may also be reproduced using the technique of TILLING (Targeted Induced Local Lesions In Genomes). This is a mutagenesis technology useful to generate and/or identify a nucleic acid encoding a Class III TPP protein with modified expression and/or activity. TILLING also allows selection of plants carrying such mutant variants. These mutant variants may exhibit modified expression, either in strength or in location or in timing (if the mutations affect the promoter for example). These mutant variants may exhibit higher Class III TPP protein activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

The effects of the invention may also be reproduced using homologous recombination, which allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; lida and Terada (2004) Curr Opin Biotech 15(2): 132-8).

Reference herein to enhanced yield-related traits is taken to mean an increase in biomass (weight) of one or more parts of a plant, which may include aboveground (harvestable) parts and/or (harvestable) parts below ground.

In particular, such harvestable parts are seeds, and performance of the methods of the invention results in plants having increased seed yield relative to the seed yield of suitable control plants.

The term "yield" in general means a measurable produce of economic value, necessarily related to a specified crop, to an area, and to a period of time. Individual plant parts directly contribute to yield based on their number, size and/or weight, whereas the actual yield is the yield per acre for a crop and year, which is determined by dividing total production (includes both harvested and appraised production) by planted acres.

The terms "increase", "improving" or "improve" are interchangeable and shall mean in the sense of the application at least a 5%, 6%, 7%, 8%, 9% or 10%, preferably at least 15% or 20%, more preferably 25%, 30%, 35% or 40% more yield and/or growth in comparison to the wild type plant as defined herein.

Increased seed yield may manifest itself as one or more of the following: a) an increase in seed biomass (total seed weight) which may be on an individual seed basis and/or per plant and/or per hectare or acre; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

Since the transgenic plants according to the present invention have increased yield, it is likely that these plants exhibit an increased growth rate (during at least part of their life cycle), relative to the growth rate of control plants at a corresponding stage in their life cycle. The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soy bean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Performance of the methods of the invention gives plants having an increased growth rate relative to control plants. Therefore, according to the present invention, there is provided a method for increasing the growth rate of plants, which method comprises modulating expression, preferably increasing expression, in a plant of a nucleic acid encoding a Class III TPP protein as defined herein.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects. Another abiotic stress may result from a nutrient deficiency, such as a shortage of nitrogen , phosphorus and potassium.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased yield relative to suitable control plants grown under comparable conditions. Therefore, according to the present invention, there is provided a method for enhancing seed yield-related traits as defined in claim 1 in plants grown under non-stress conditions or under mild drought conditions, which method comprises increasing expression in a plant of a nucleic acid encoding a Class III TPP polypeptide as defined in claim 1.

The enhanced yield-related trait is manifested as an increase in one or more of the following: total number of seeds per plant, number of filled seeds per plant and seed weight per plant. Preferably, these increases are found in plants grown under non-stress conditions.

The methods of the invention are advantageously applicable to any plant.

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agropyron* spp., *Allium* spp., *Amaranthus* spp., *Ananas comosus, Annona* spp., *Apium graveolens, Arachis* spp, *Artocarpus* spp., *Asparagus officinalis, Avena* spp. (e.g. *Avena sativa, Avena fatua, Avena byzantina, Avena fatua* var. sativa, *Avena hybrida), Averrhoa carambola, Benincasa hispida, Bertholletia excelsea, Beta vulgaris, Brassica* spp. (e.g. *Brassica napus, Brassica rapa* ssp. [canola, oilseed rape, turnip rape]), *Cadaba farinosa, Camellia sinensis, Canna indica, Capsicum* spp., *Carex elata, Carica papaya, Carissa macrocarpa, Carya* spp., *Carthamus tinctorius, Castanea* spp., *Cichorium endivia, Cinnamomum* spp., *Citrullus lanatus, Citrus* spp., Cocos spp., *Coffea* spp., *Colocasia esculenta, Cola* spp., *Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., Elaeis (e.g. *Elaeis guineensis, Elaeis oleifera), Eleusine coracana, Eriobotrya japonica, Eugenia unitlora, Fagopyrum* spp., *Fagus* spp., *Ficus carica, Fortunella* spp., *Fragaria* spp., *Ginkgo biloba, Glycine* spp. (e.g. *Glycine max, Soja hispida* or *Soja max), Gossypium hirsutum, Helianthus* spp. (e.g. Helianthus annuus), *Hemerocallis fulva, Hibiscus* spp., *Hordeum* spp. (e.g. *Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. (e.g. *Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus spp., Momordica* spp., *Morus nigra, Musa* spp., *Nicotiana* spp., *Olea* spp., *Opuntia* spp., *Ornithopus* spp., *Oryza* spp. (e.g. *Oryza sativa, Oryza latifolia), Panicum miliaceum, Passiflora edulis, Pastinaca sativa, Persea* spp., *Petroselinum crispum, Phaseolus* spp., *Phoenix* spp., *Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Sambucus* spp., *Secale cereale, Sesamum* spp., *Sinapis* sp., *Solanum* spp. (e.g. *Solanum tuberosum, Solanum integrifolium* or *Solanum lycopersicum), Sorghum bicolor, Spinacia* spp., *Syzygium* spp., *Tagetes* spp., *Tamarindus indica, Theobroma cacao, Trifolium* spp., *Triticosecale rimpaui, Triticum* spp. (e.g. *Triticum aestivum, Triticum durum, Triticum turgidum, Triticum hybernum, Triticum macha, Triticum sativum* or *Triticum vulgare), Tropaeolum minus, Tropaeolum majus, Vaccinium* spp., *Vicia* spp., *Vigna* spp., *Viola odorata, Vitis* spp., Zea *mays, Zizania palustris, Ziziphus* spp., amongst others.

According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, sorghum and oats.

The present invention also encompasses use of nucleic acids encoding the Class III TPP protein described herein and use of these Class III TPP proteins in enhancing yield-related traits in plants.

Nucleic acids encoding the Class III TPP protein described herein, or the Class III TPP proteins themselves, may find use in breeding programmes in which a DNA marker is identified which may be genetically linked to a Class III TPP encoding gene. The nucleic acids/genes, or the Class III TPP proteins themselves may be used to define a molecular marker. This DNA or protein marker may then be used in breeding programmes to select plants having enhanced yield-related traits as defined hereinabove in the methods of the invention.

Allelic variants of a Class III TPP protein-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding Class III TPP proteins may also be used as probes for genetically and physically mapping the genes that they are a part of, and as markers for traits linked to those genes. Such information may be useful in plant breeding in order to develop lines with desired phenotypes. Such use of Class III TPP protein-encoding nucleic acids requires only a nucleic acid sequence of at least 15 nucleotides in length. The Class III TPP protein-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the Class III TPP protein-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the Class III TPP protein-encoding nucleic acid in the genetic map previously obtained using this population (Botstein et al. (1980) Am. J. Hum. Genet. 32:314-331).

The production and use of plant gene-derived probes for use in genetic mapping is described in Bematzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence *in situ* hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

### Description of figures

**Fig. 1** is a schematic representation of the structural elements present in Class III TPP polypeptides. The position of the characteristic elements: trehalose phosphate phosphatase domain, phosphatase BOX A and BOX B and serine-rich domain are indicated.

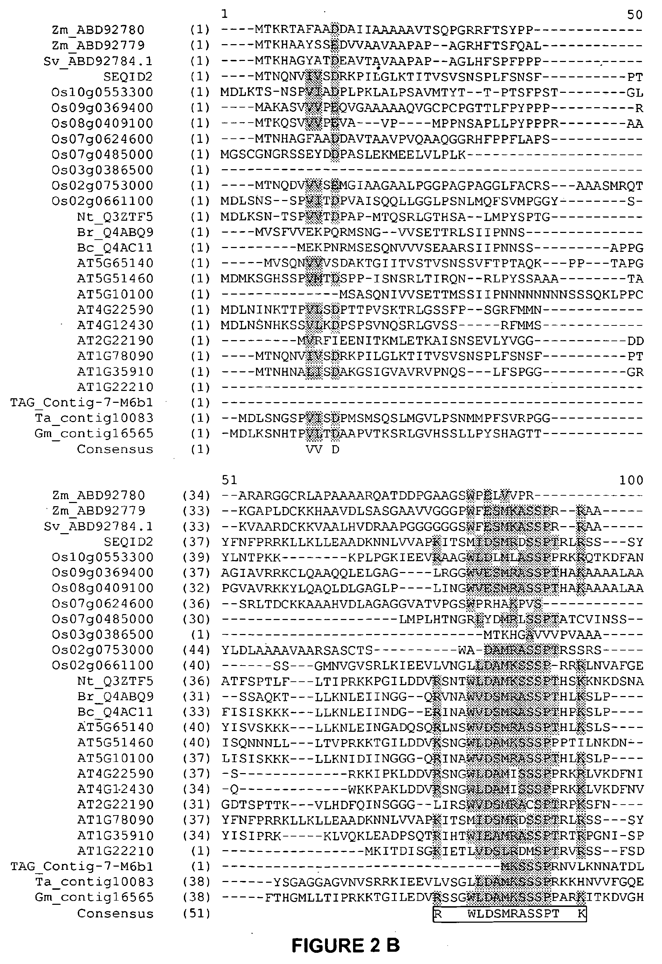

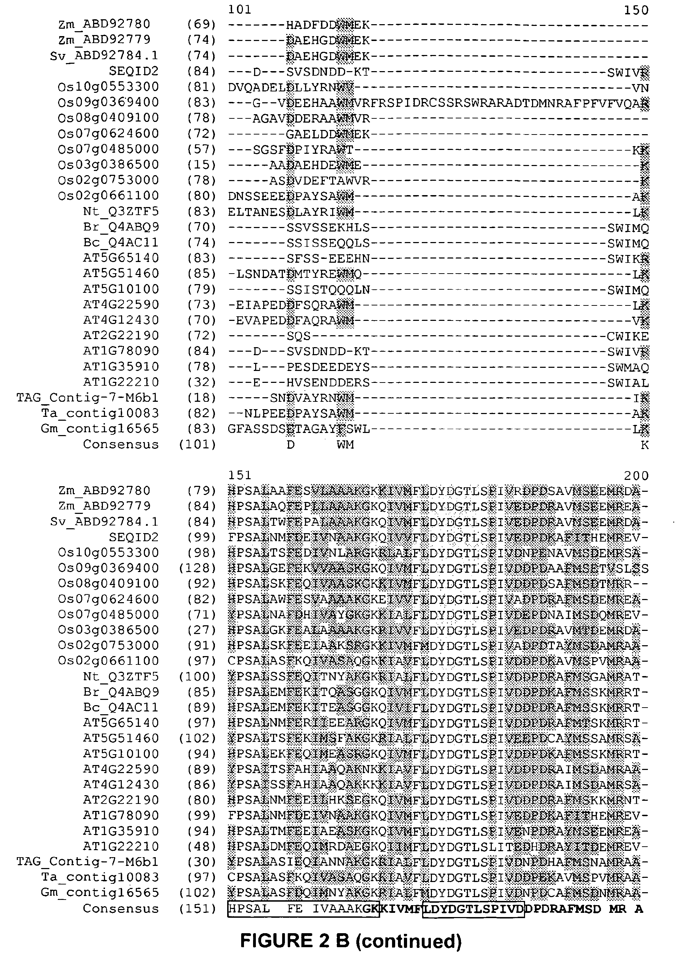

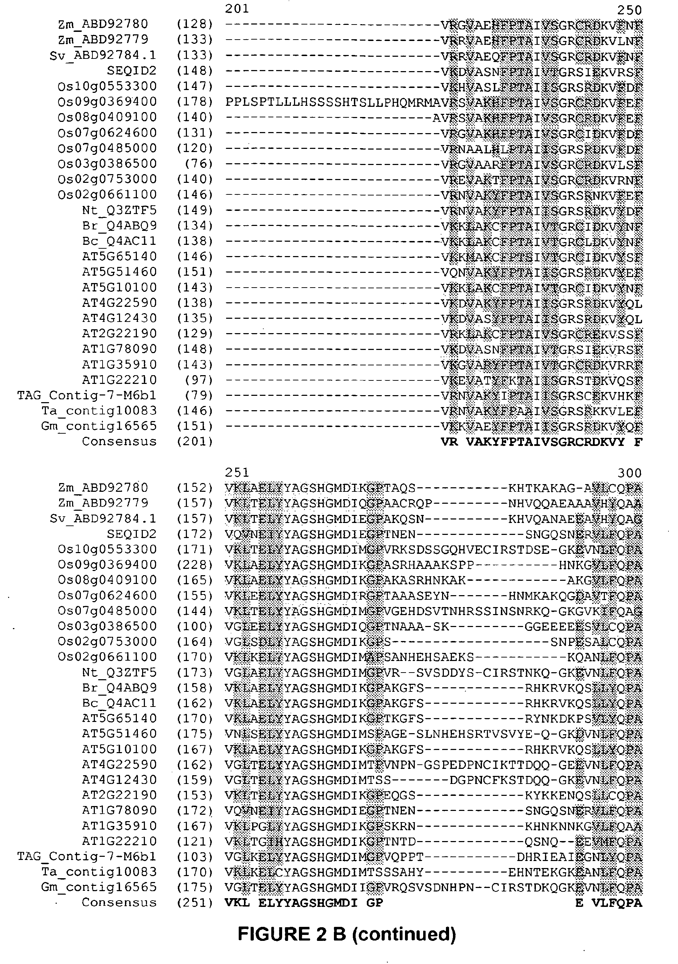

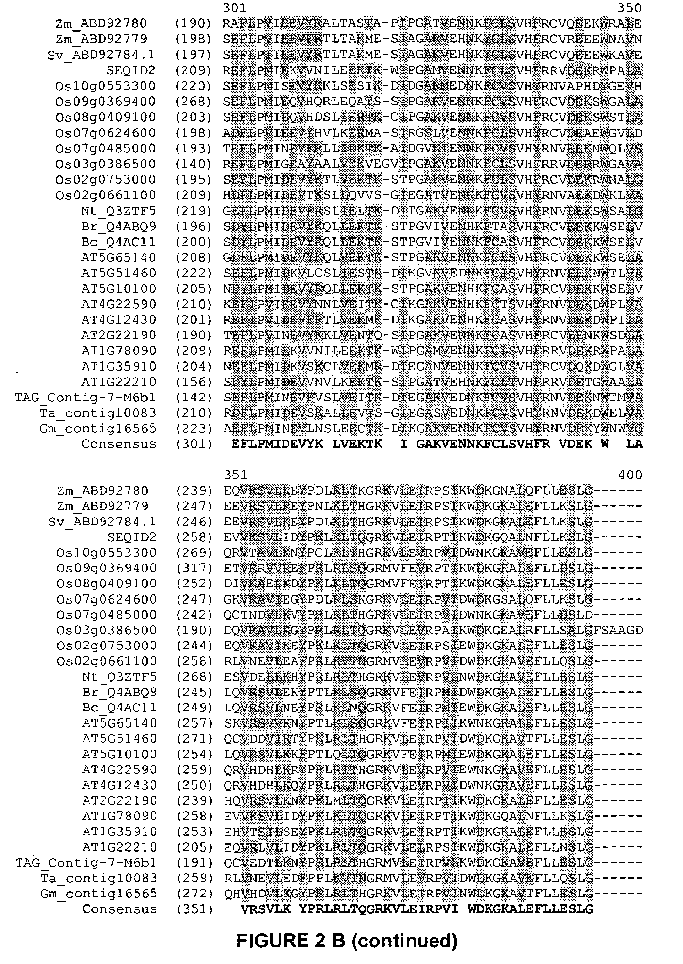

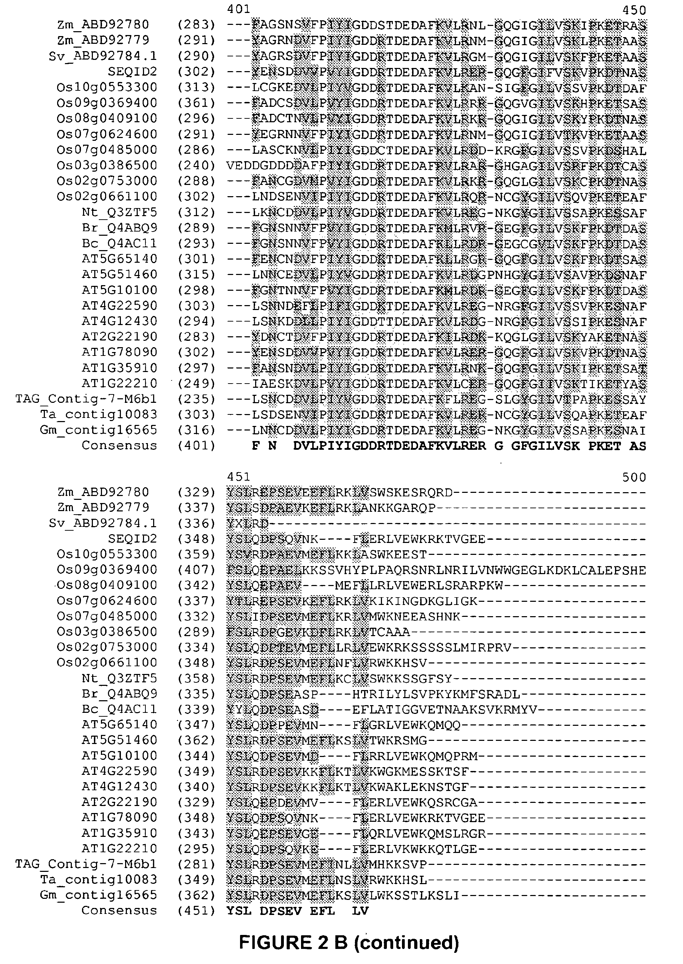

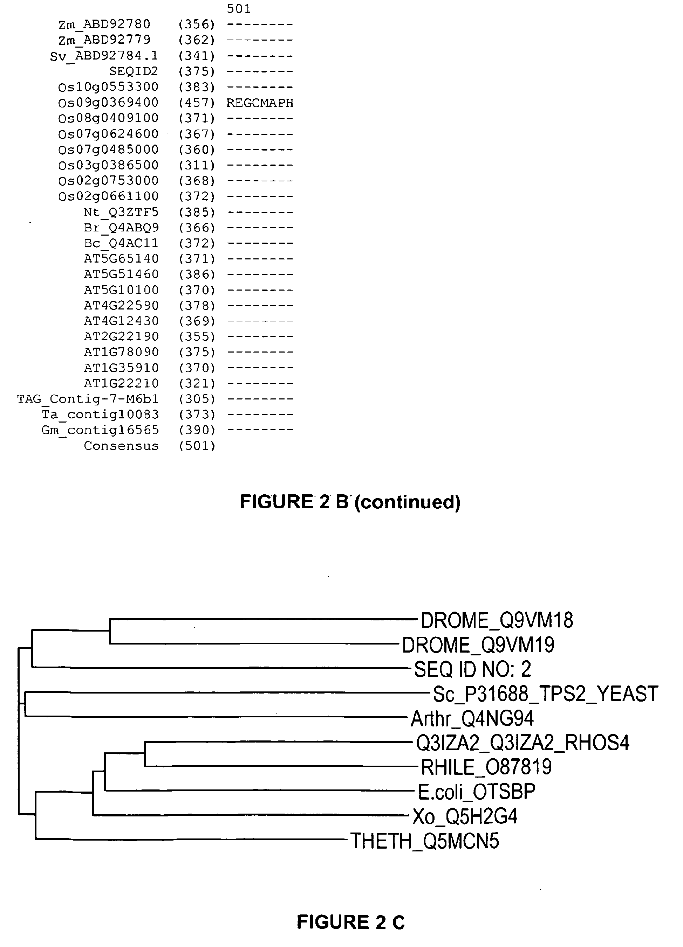

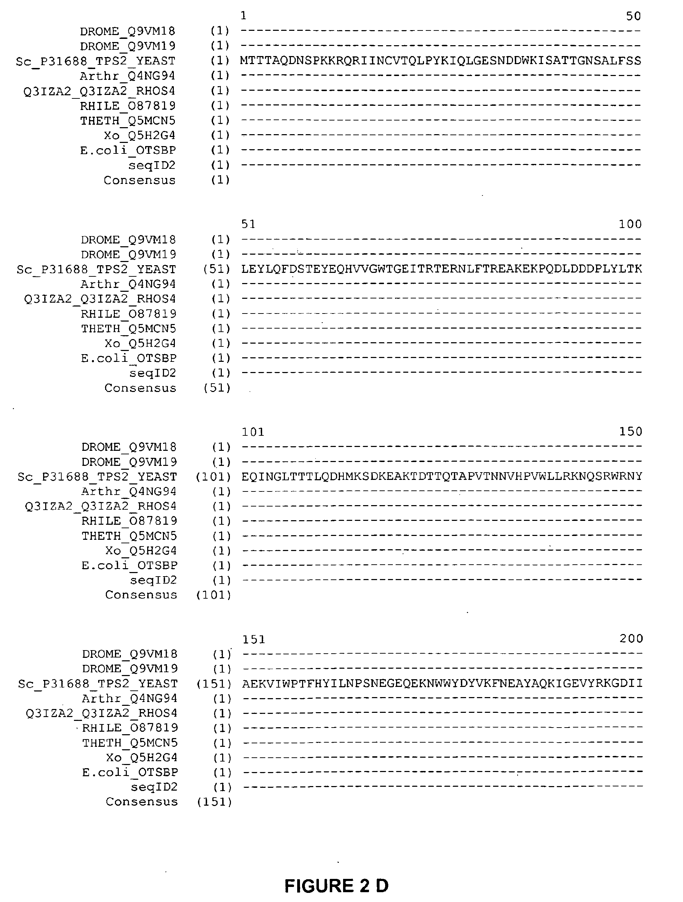

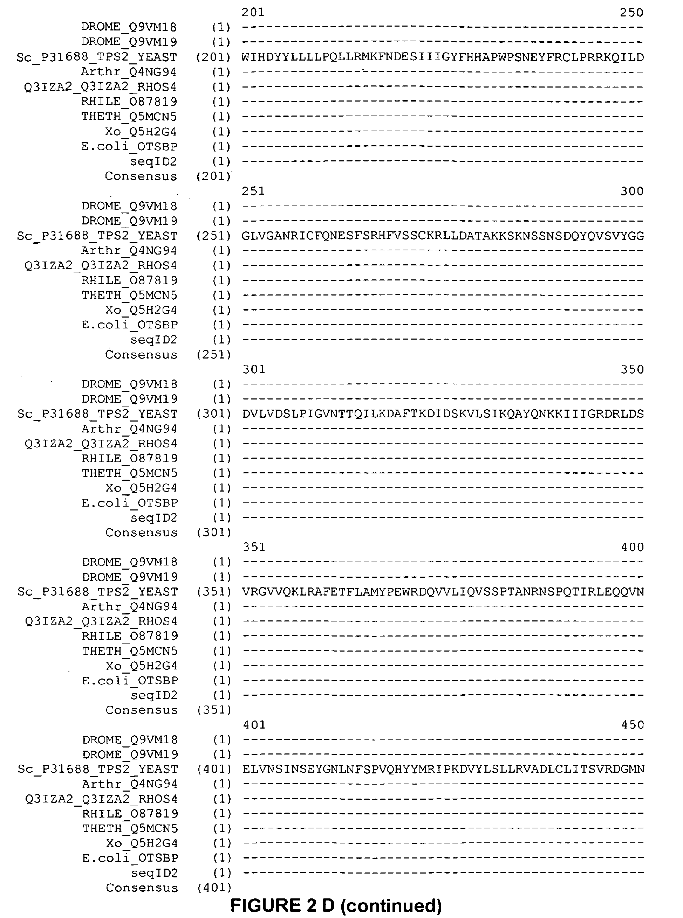

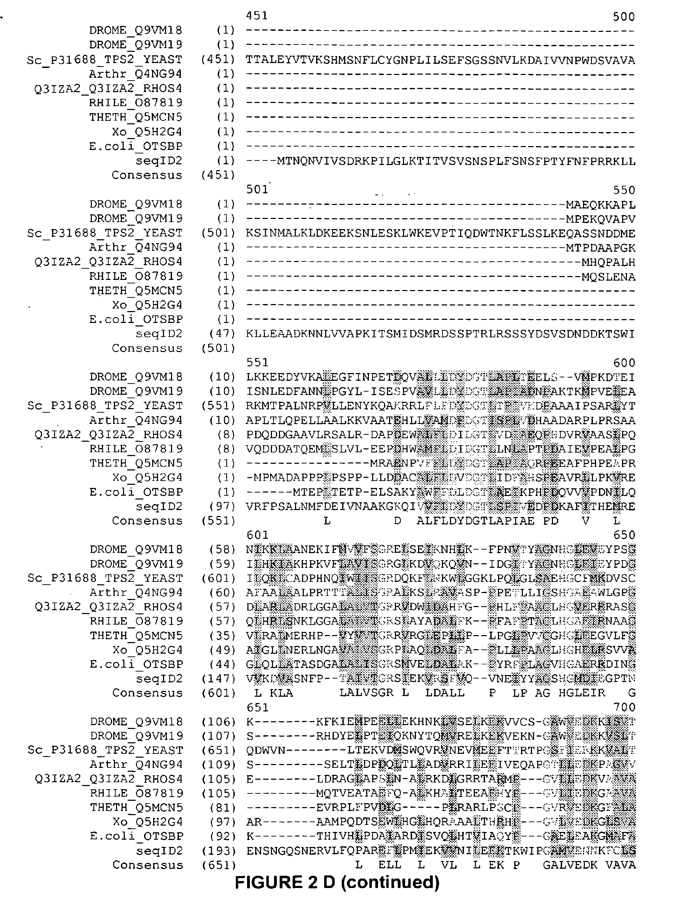

**Fig. 2** is a sequence alignment and phylogenetic tree of TPS/TPP polypeptides as described in Example 2. Figure 2A shows a phylogenetic tree of TPS/TPP polypeptides. Clades encircled in the oval contain proteins of Class I and Class II TPS proteins. Class III TPP proteins (within the rectangle) cluster apart from the Class I and Class II TPS group. Figure 2B shows an alignment of Class III TPP polypeptides of plant origin. Region of the Trehalose-PPase domain is highlighted in bold. Position of the serine-rich and Phosphatase A and B boxes are highlighted by a rectangle. Figure 2C shows a phylogenetic tree of Class III TPP polypeptides of non-plant origin. Figure 2D shows an alignment of Class III TPP polypeptides of non-plant origin.
**Fig. 3** is an alignment of Trehalose-PPase domains found in Class III TPP polypeptides.
**Fig. 4** shows TPP activity as measured by HPLC. The first graph of Fig. 4A corresponds to the control sample pGEX; the second graph of Fig. 4B corresponds to the TPPI sample.
**Fig. 5** is a bar chart showing quantification of TPP activity as detected by HPLC for the control sample, pGEX, and for the TPP sample.
**Fig. 6** shows the complementation of the growth defect on the yeast strain YSH448 (tps2A) by *Arabidopsis thaliana* Class III TPP polypeptides. Picture in Fig. 6A corresponds to a plate of a drop assay at 30 C, showing that all strains are viable at the non-restrictive temperature. Fig. 6B shows a plate at 37 C, showing that only strains transformed with a Class III TPP polynucleotide grow well at the restrictive temperature of 37 C. Fig. 6C shows a plate of a drop assay at 39 C.
**Fig. 7** Binary vector for increased expression in *Oryza sativa* of an *Arabidopsis thaliana* Class III TPP protein-encoding nucleic acid under the control of a GOS2 promoter.

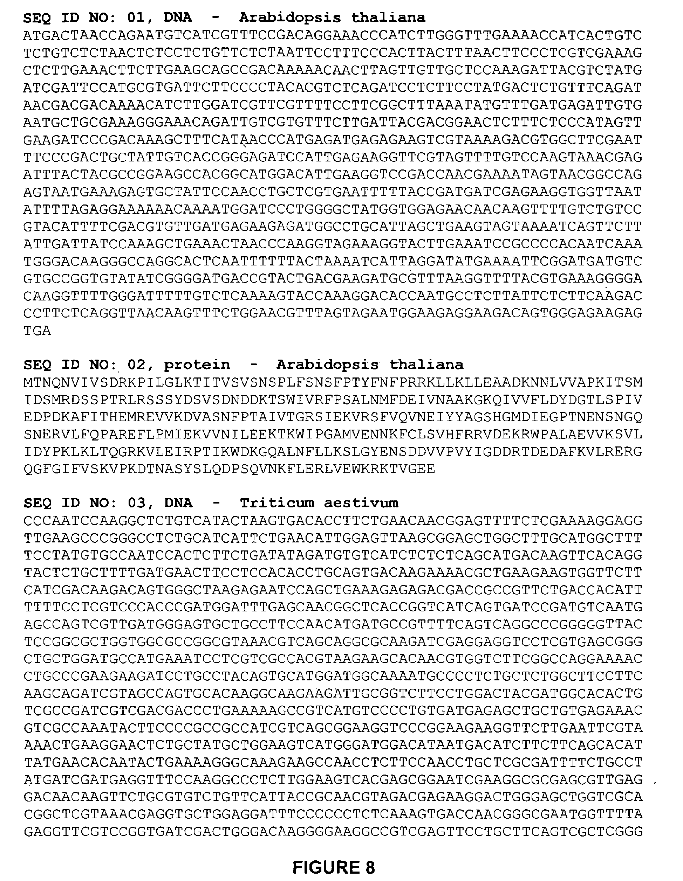

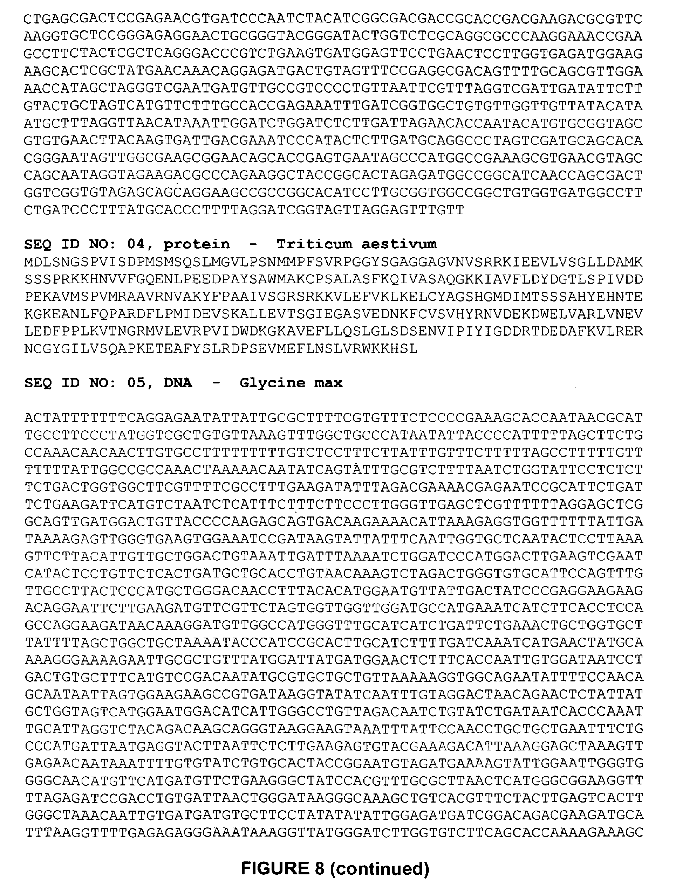

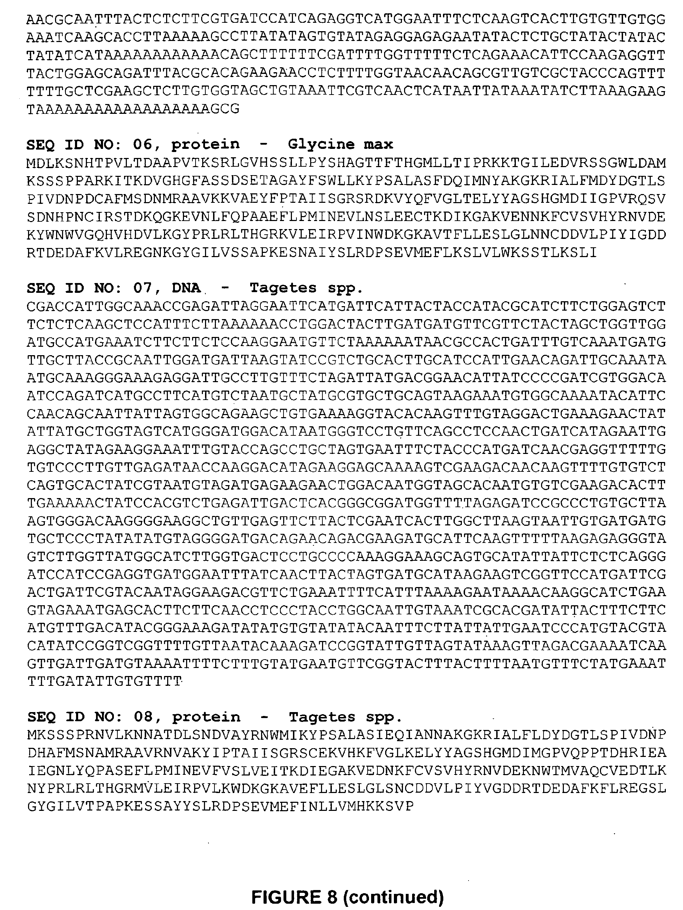

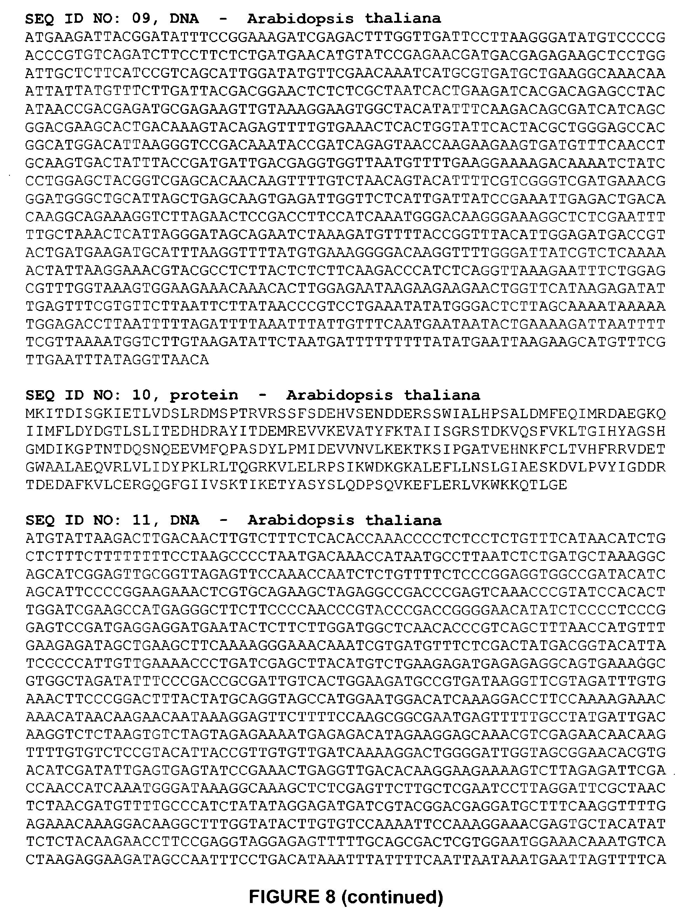

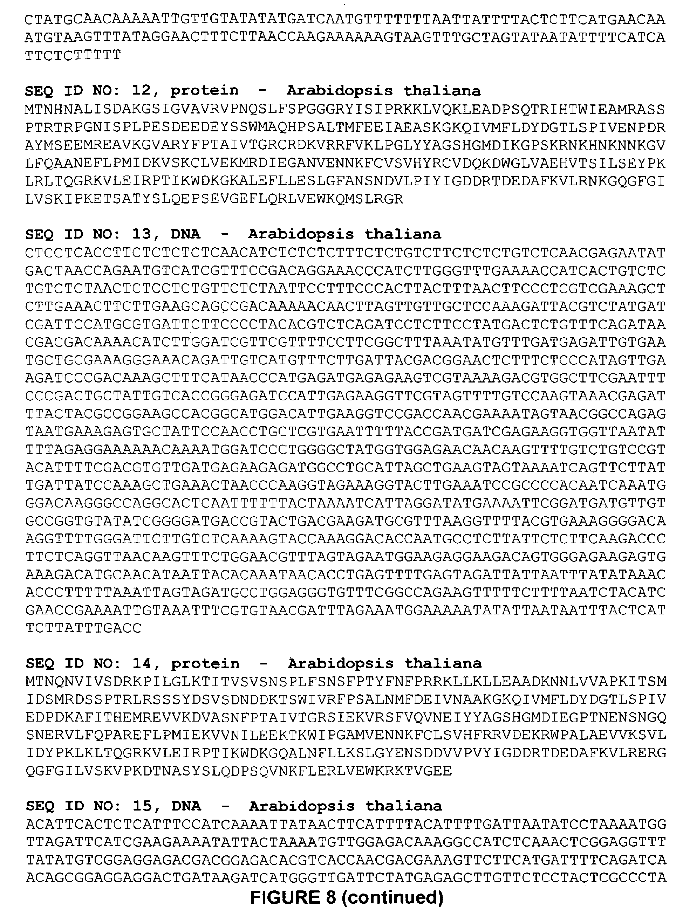

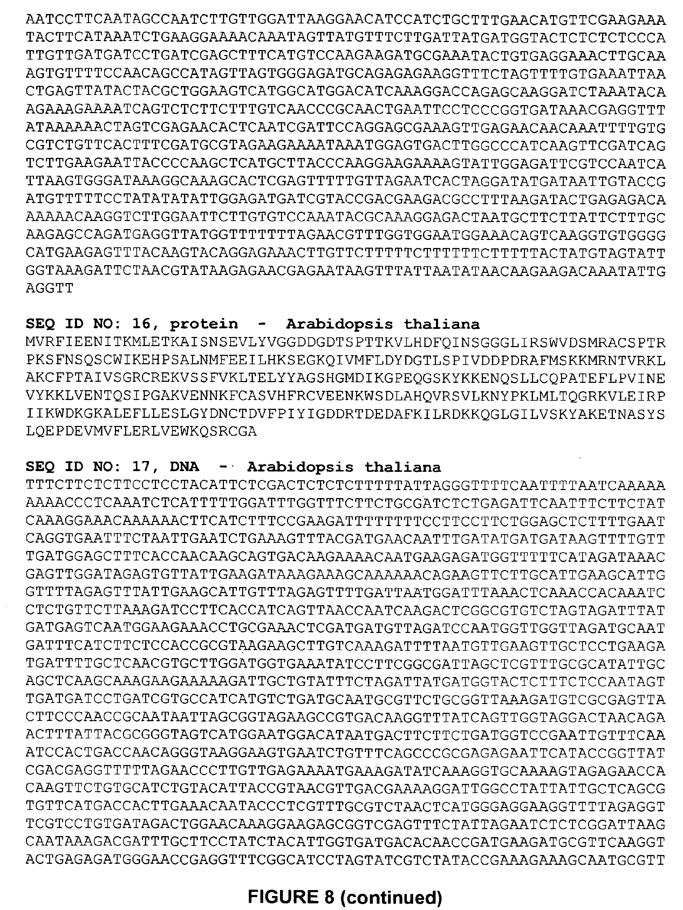

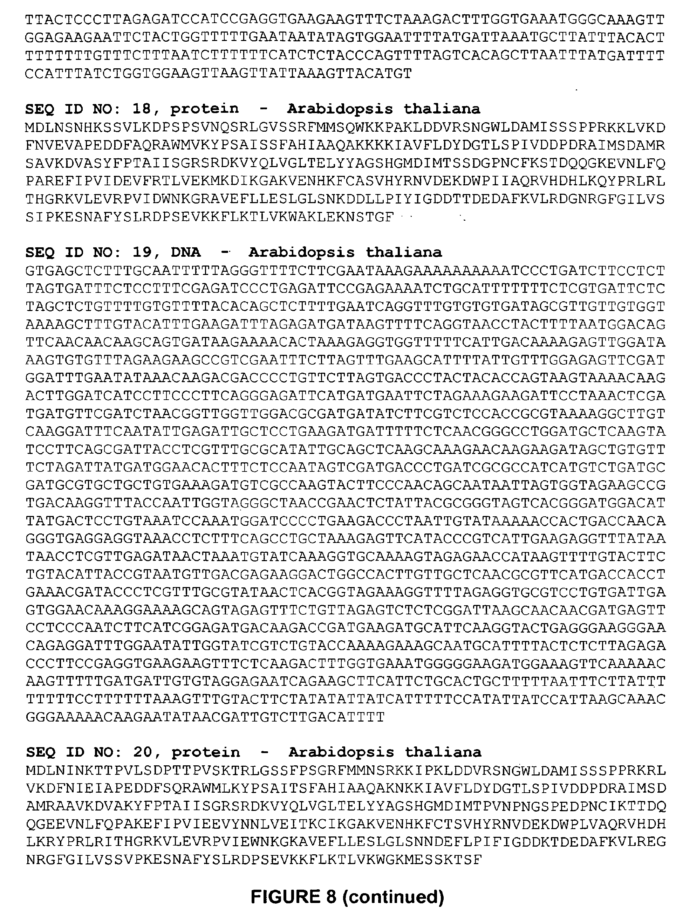

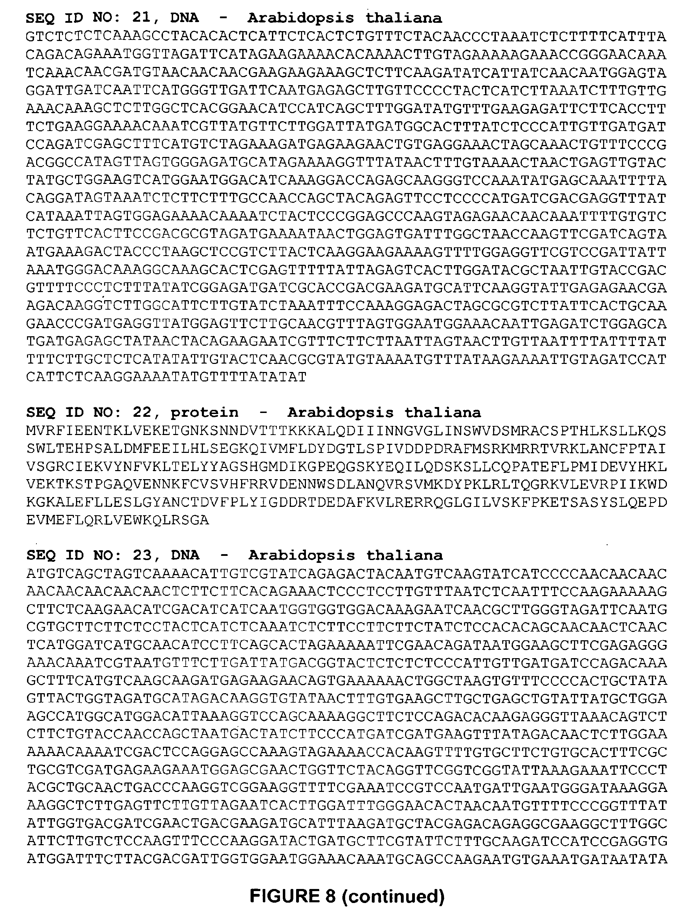

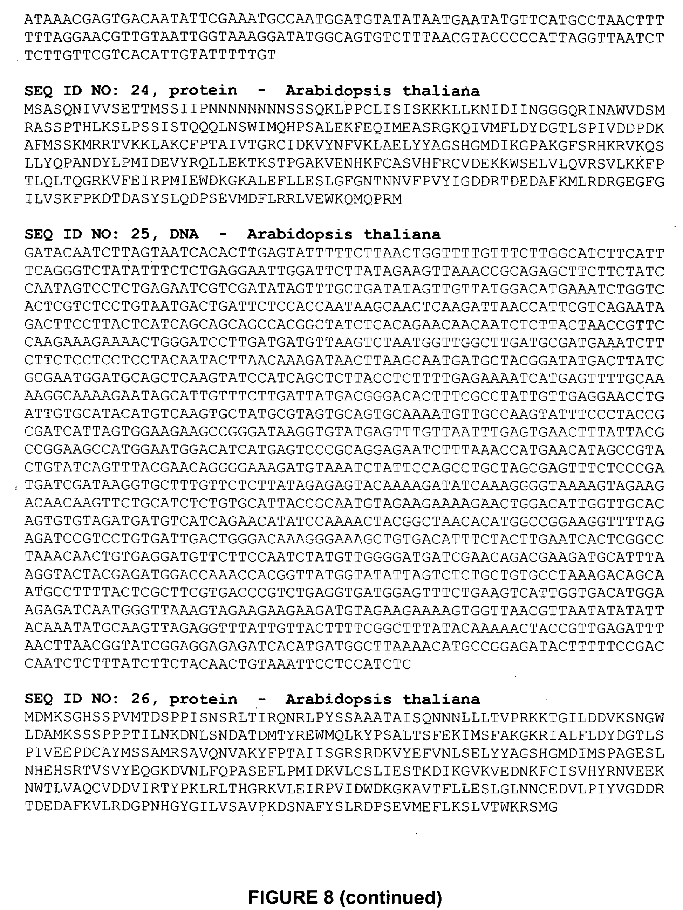

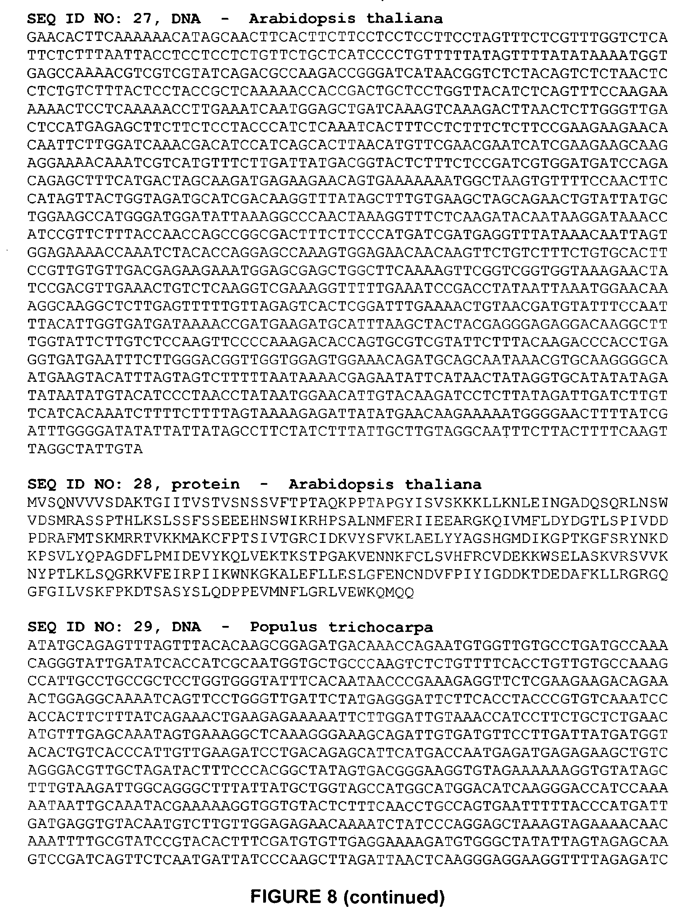

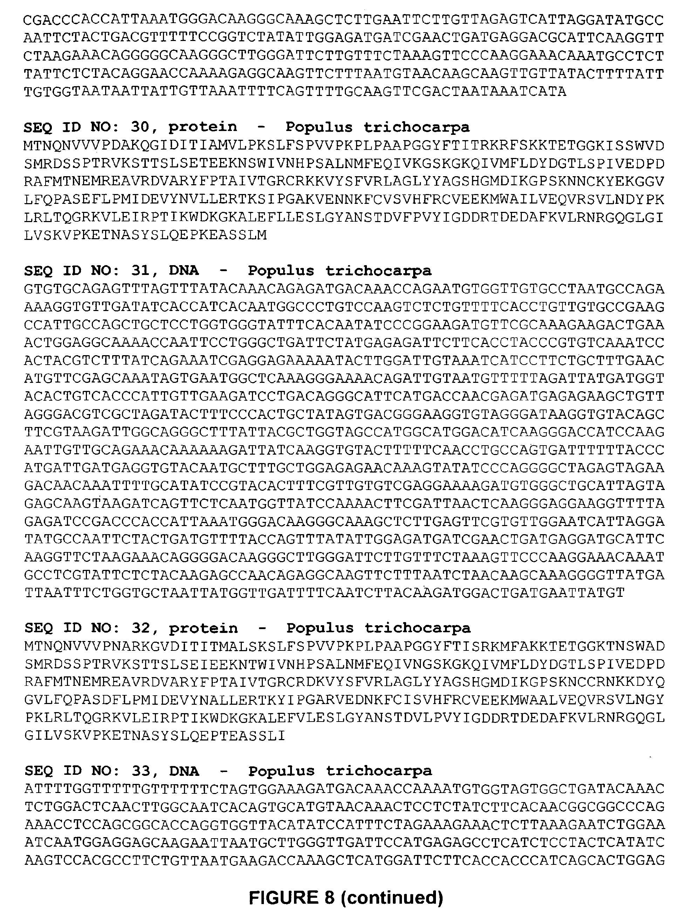

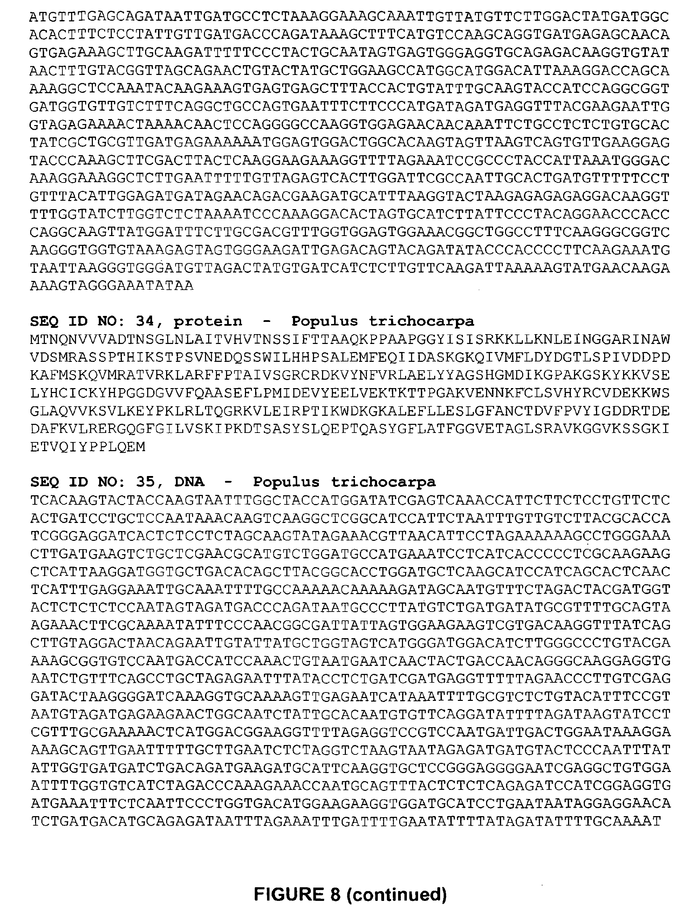

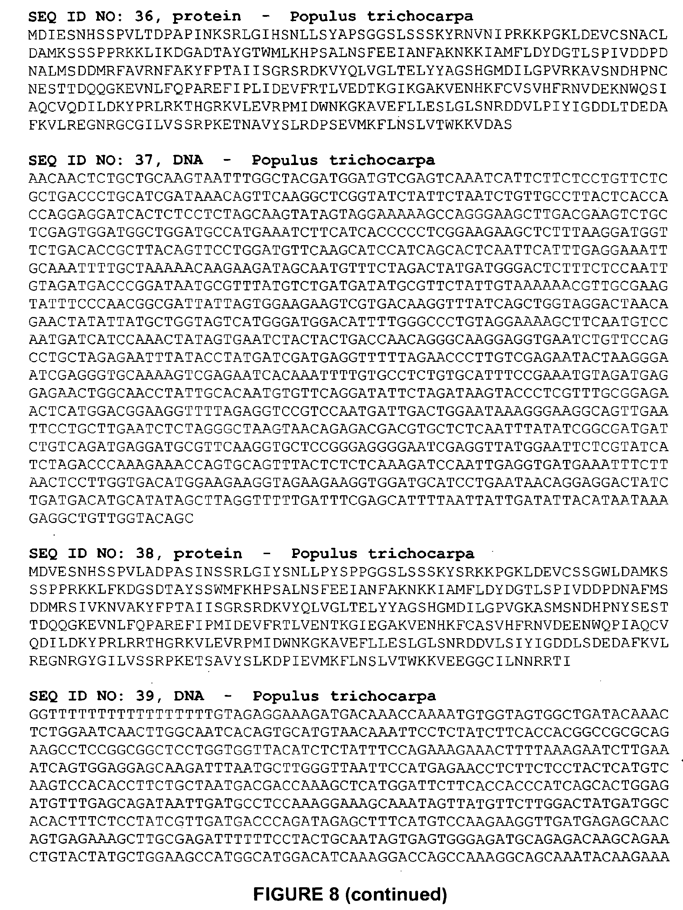

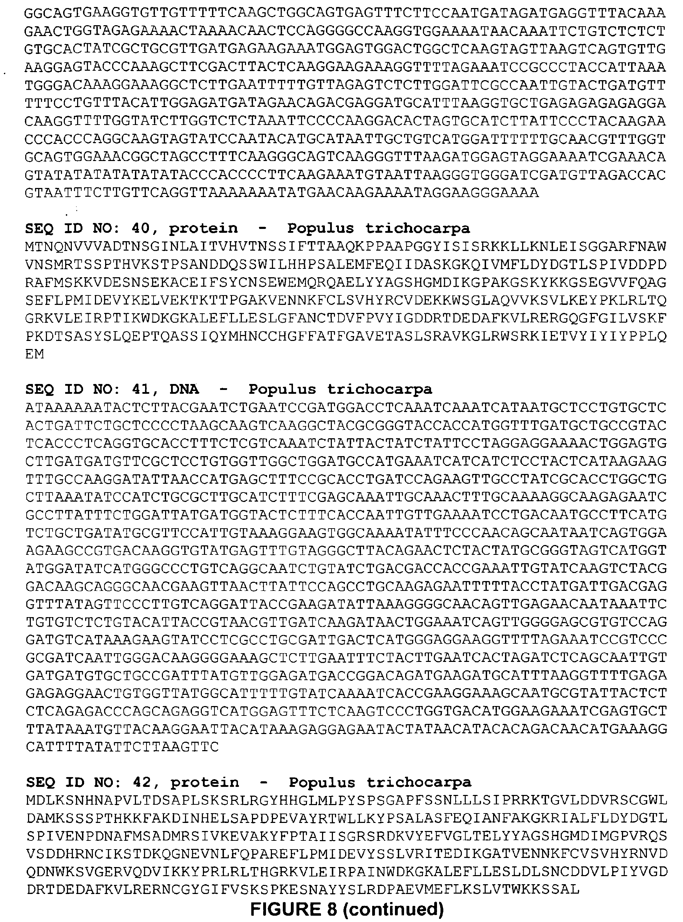

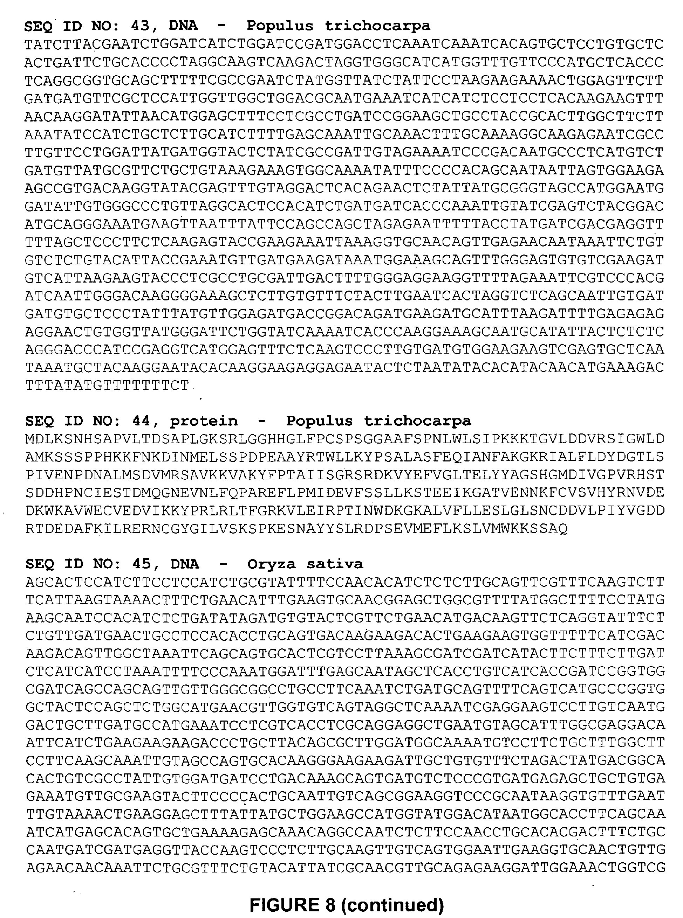

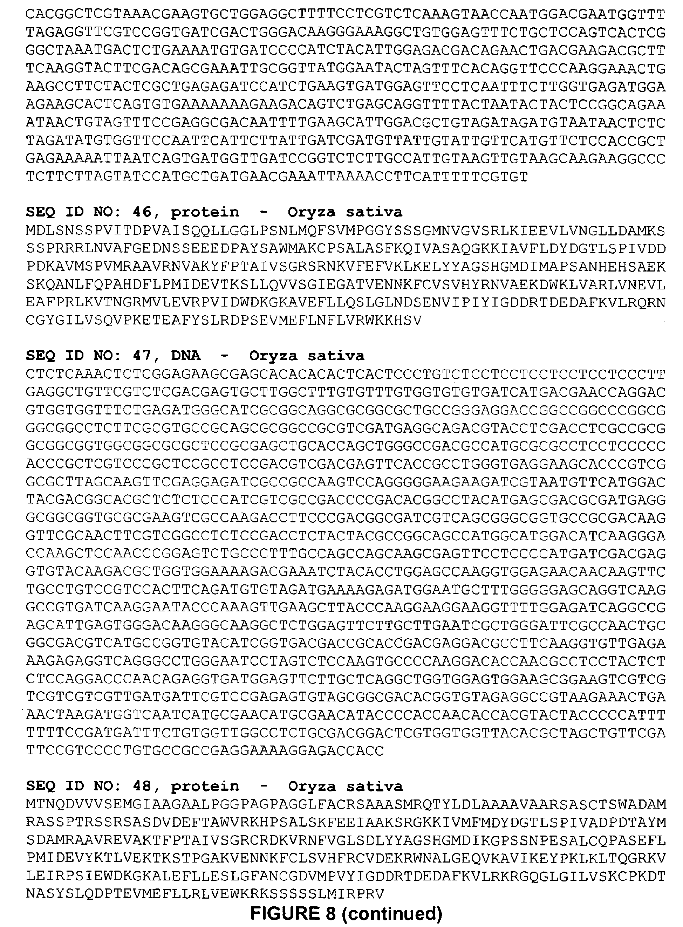

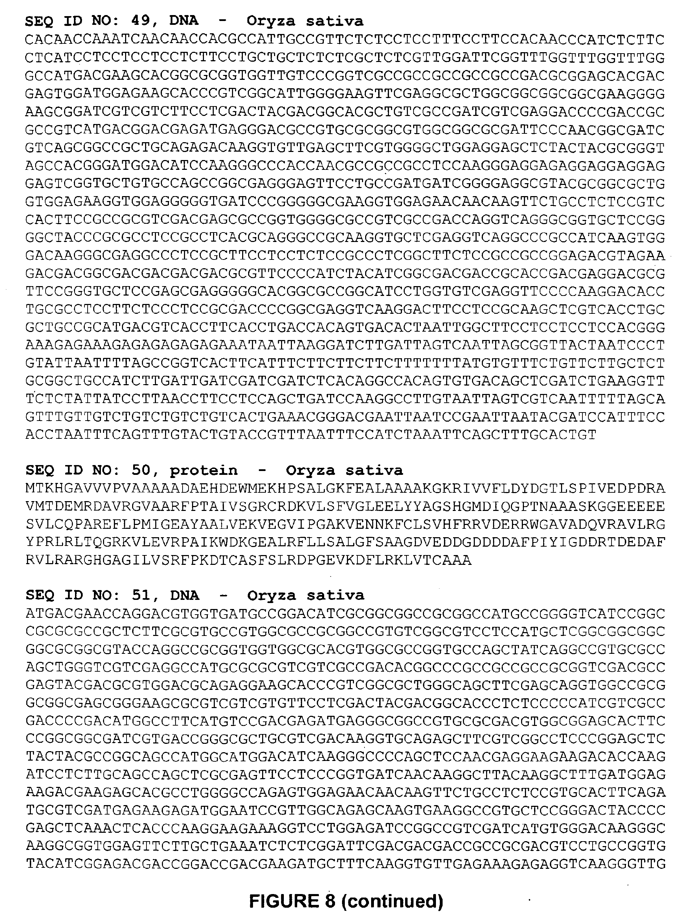

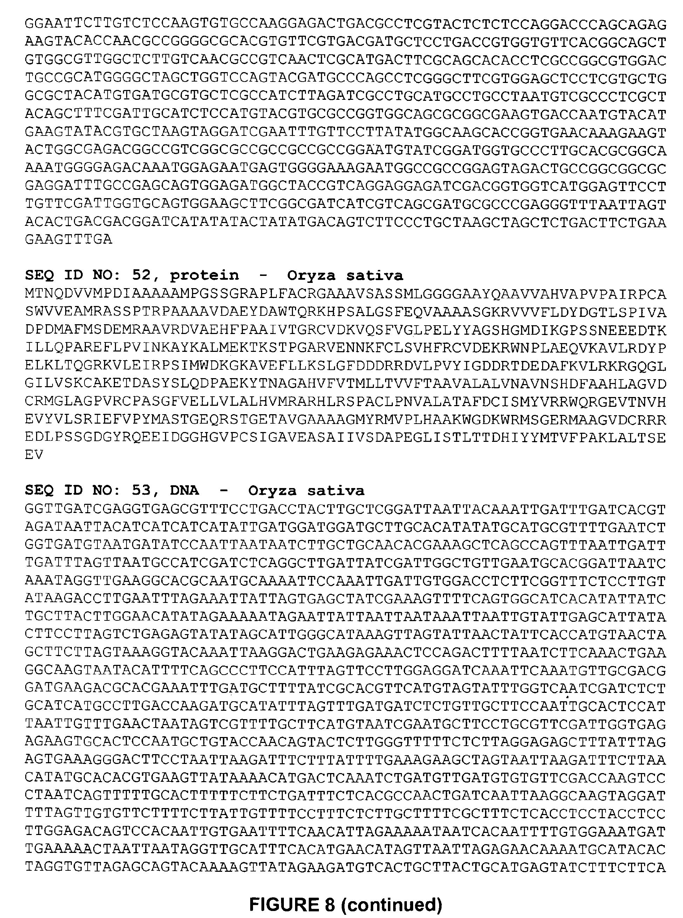

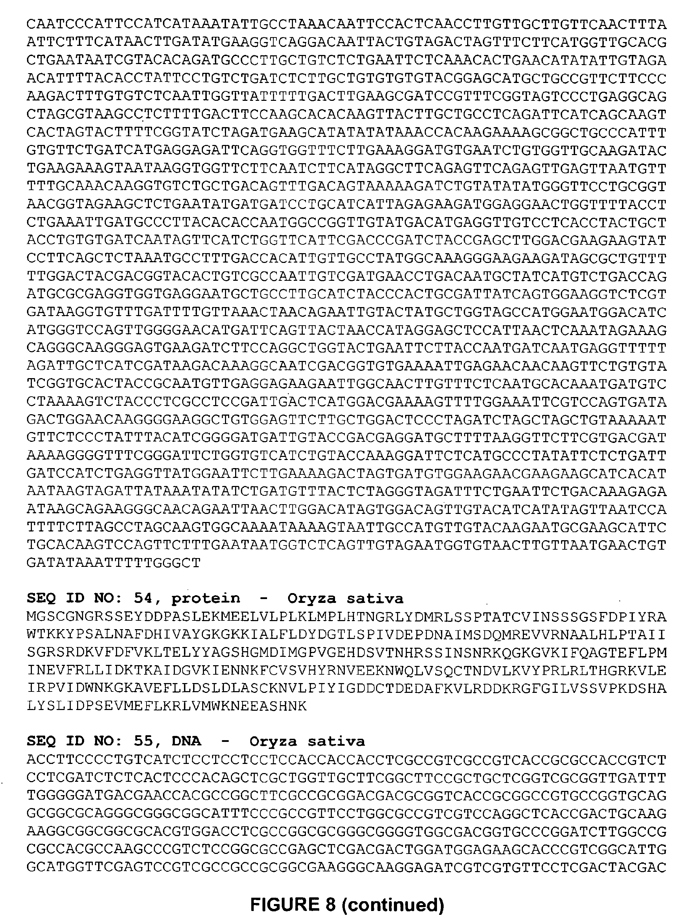

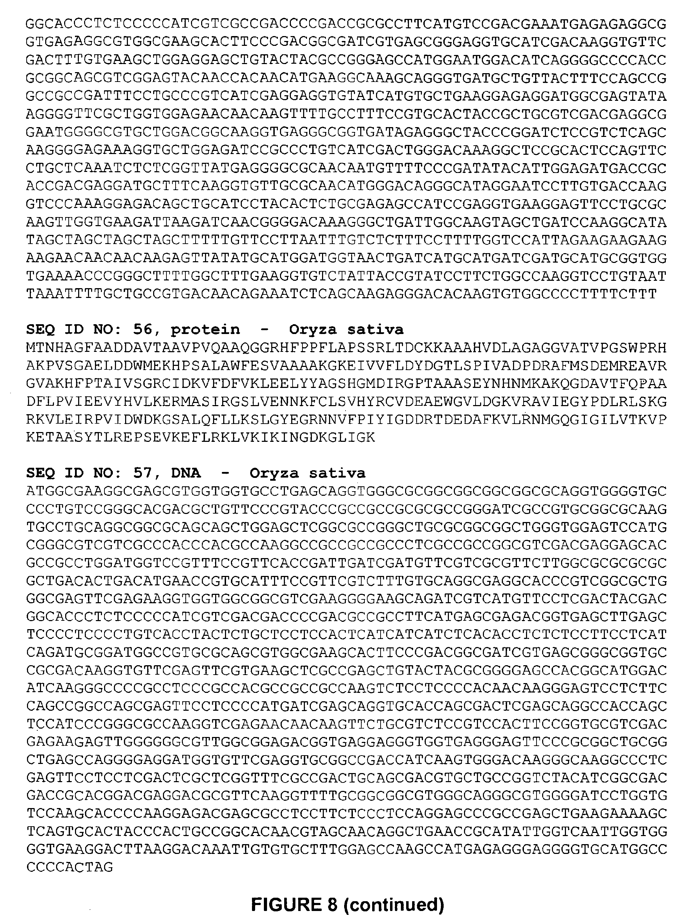

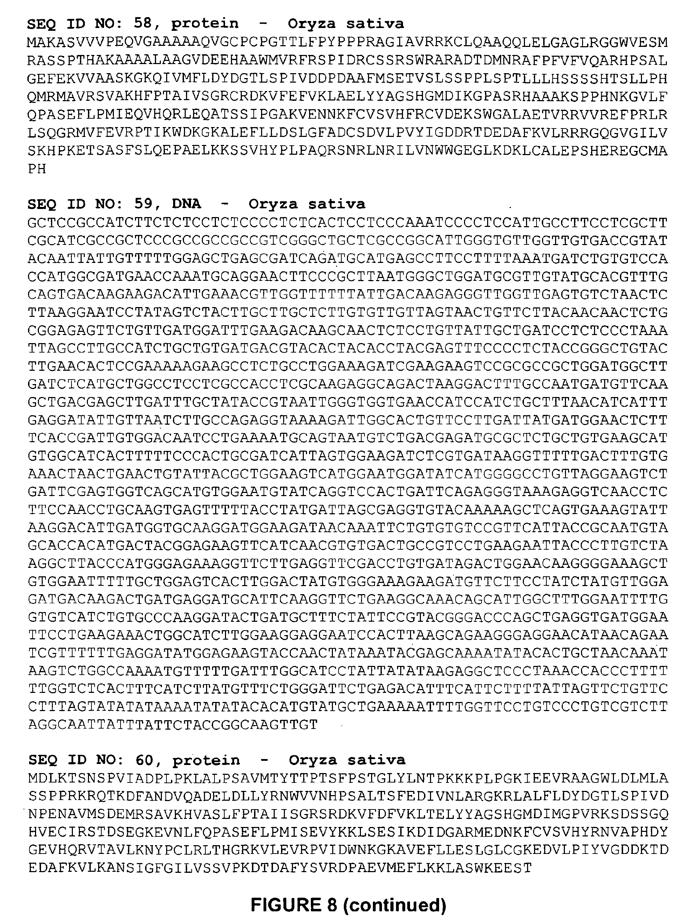

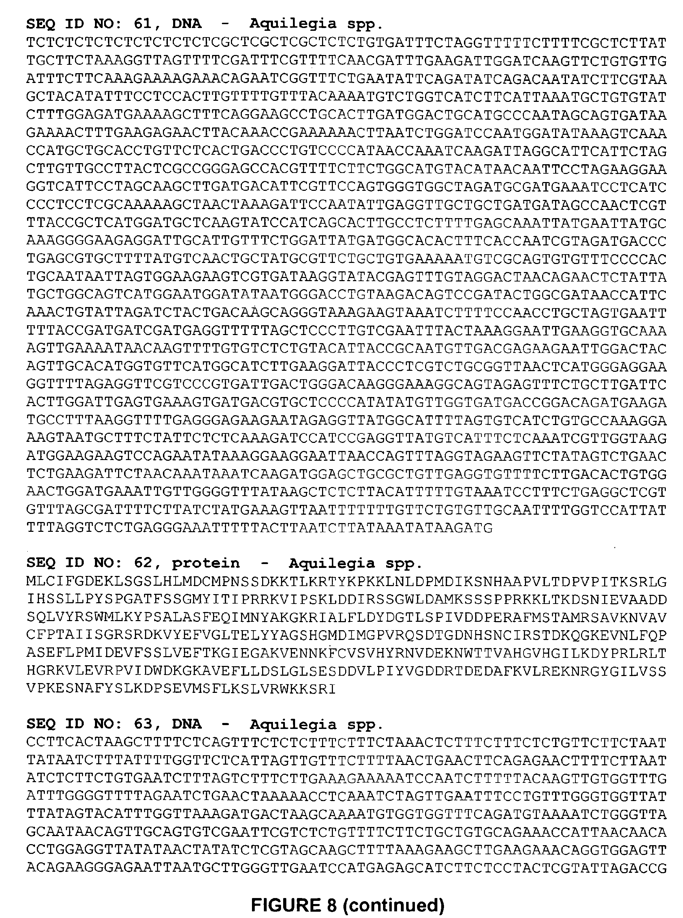

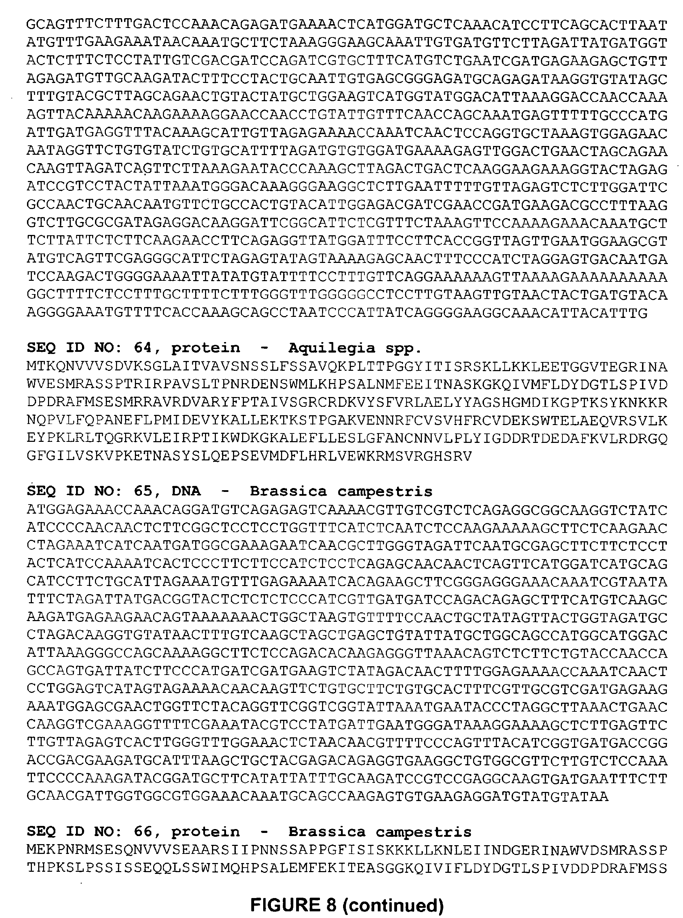

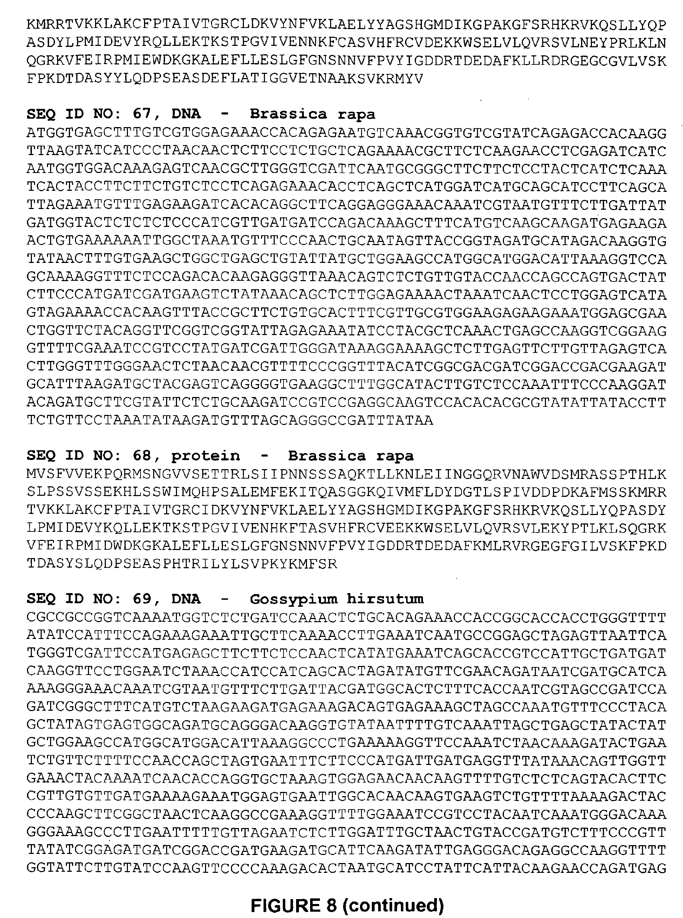

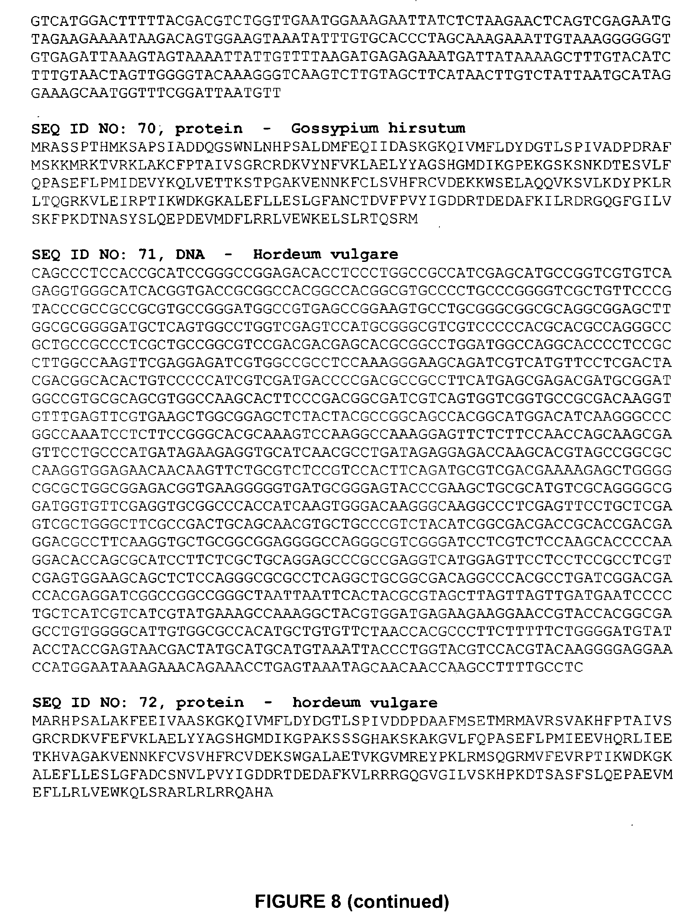

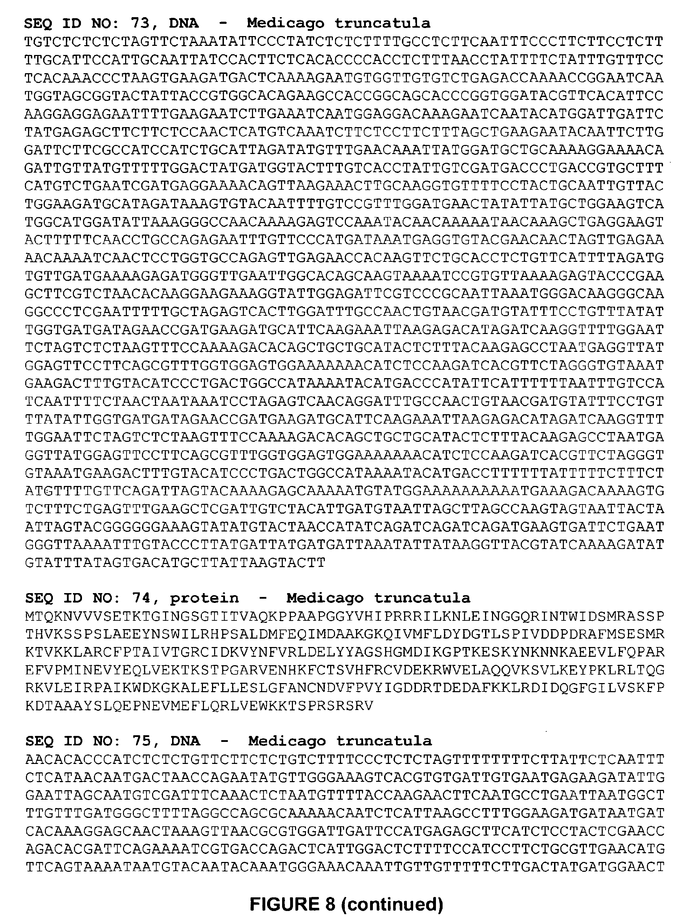

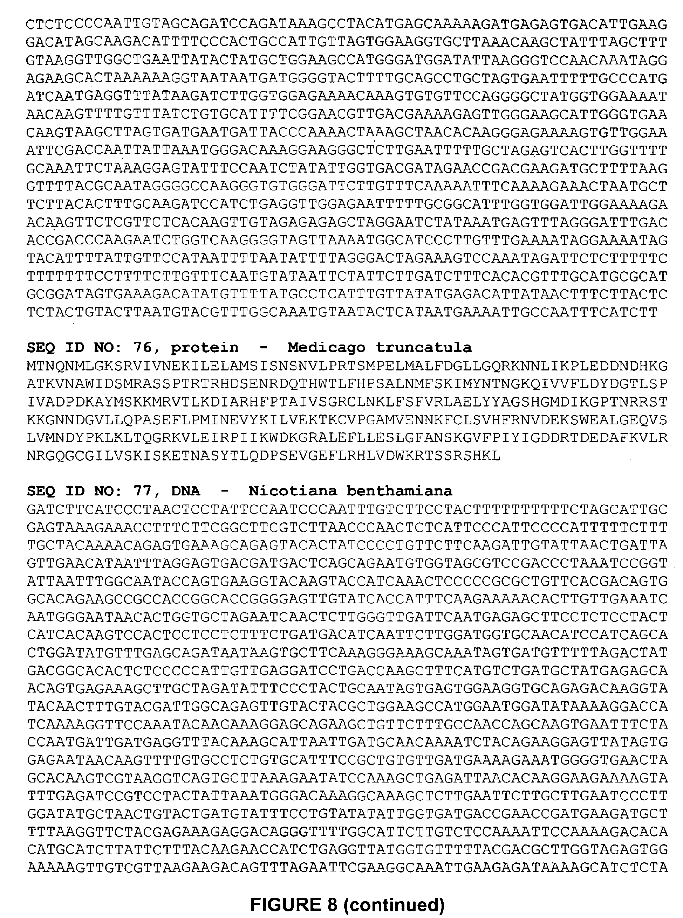

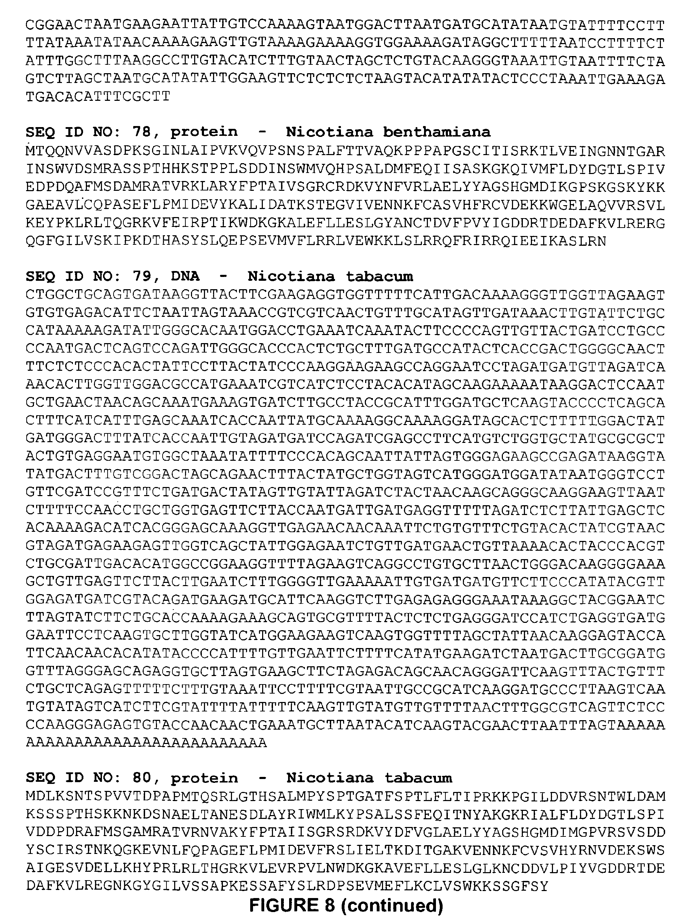

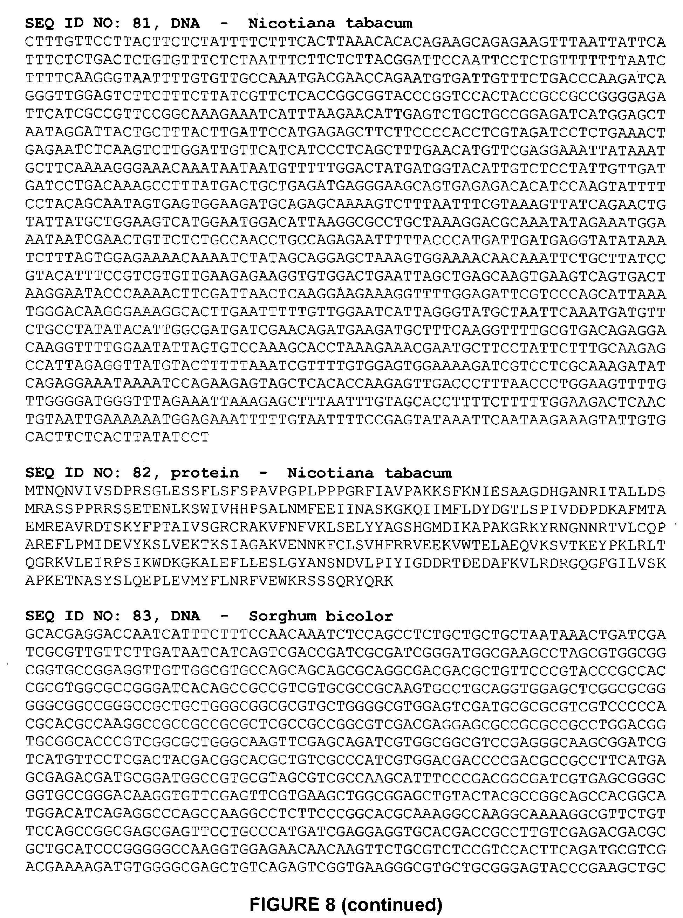

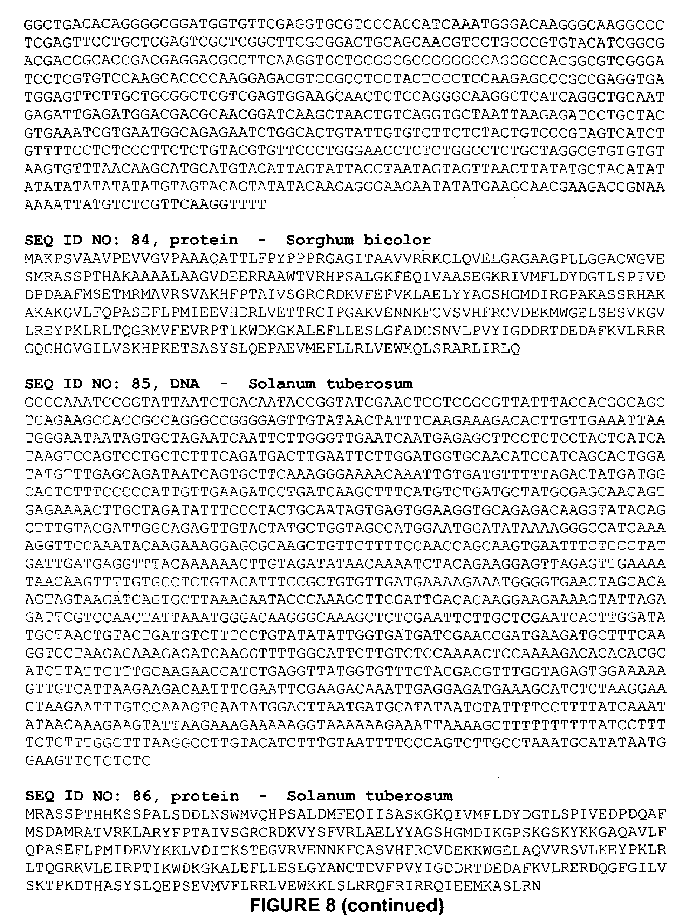

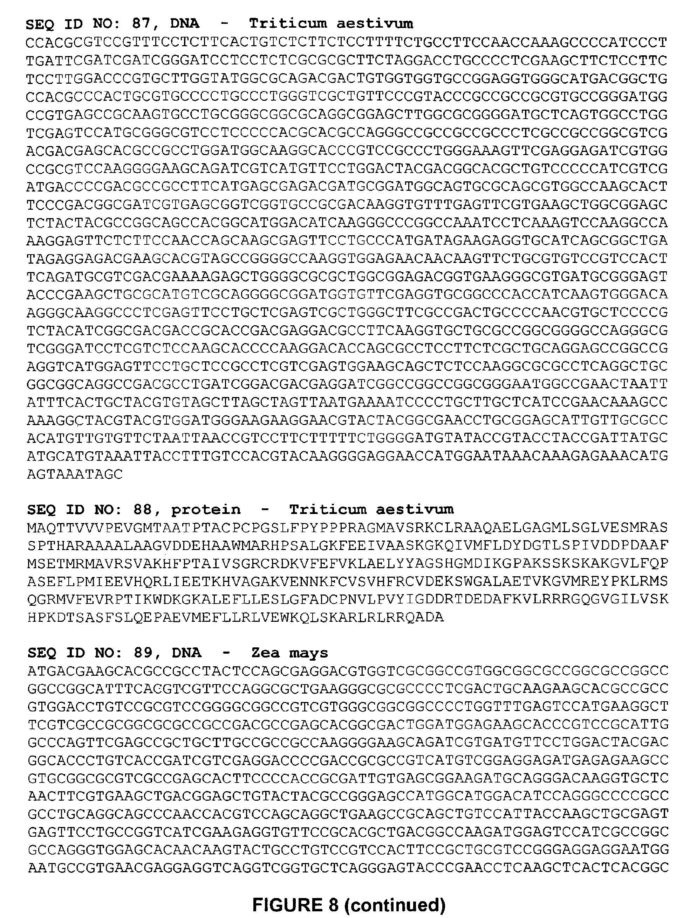

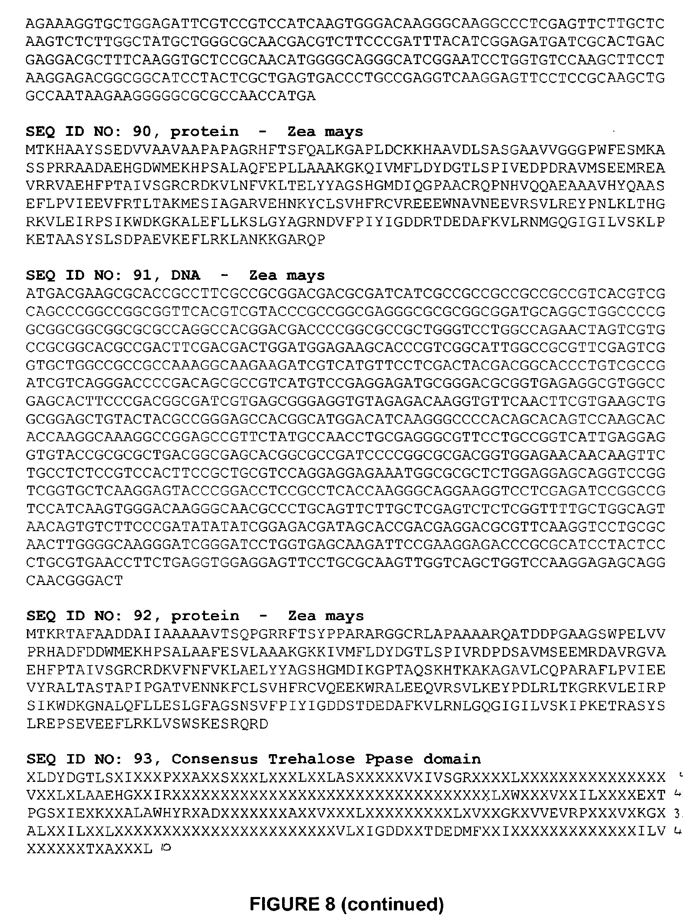

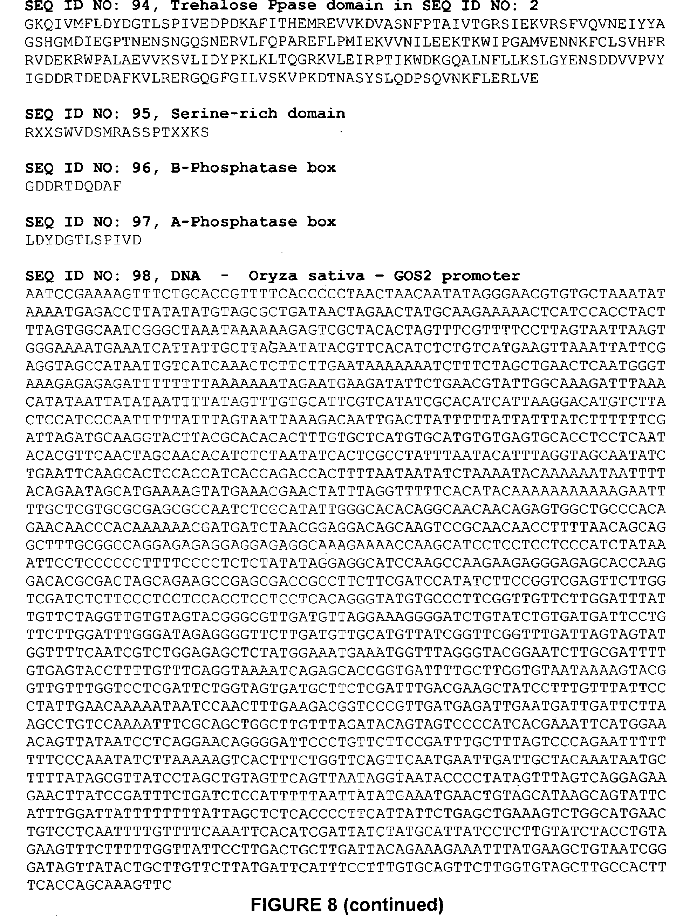

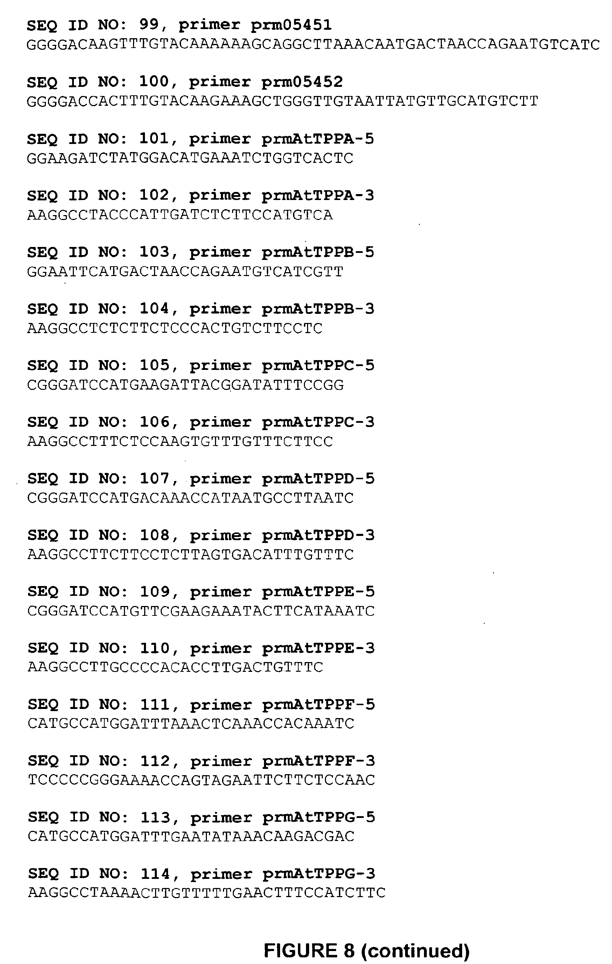

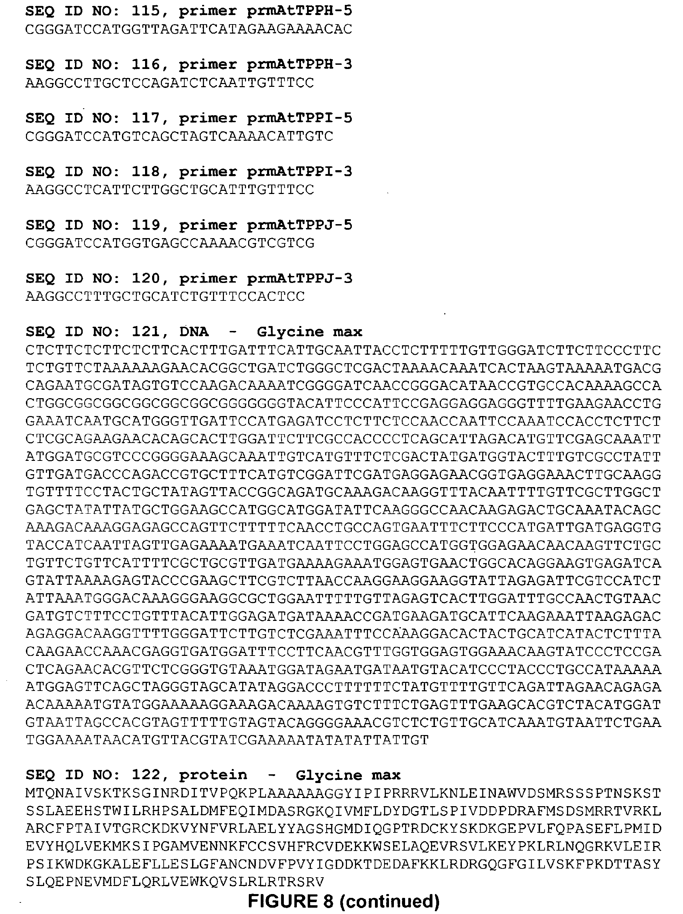

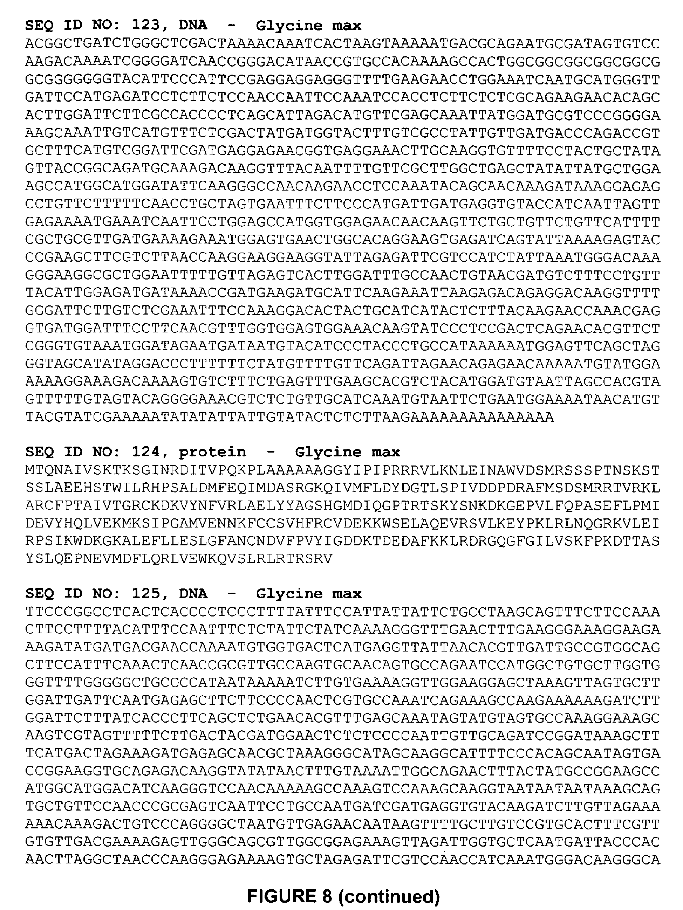

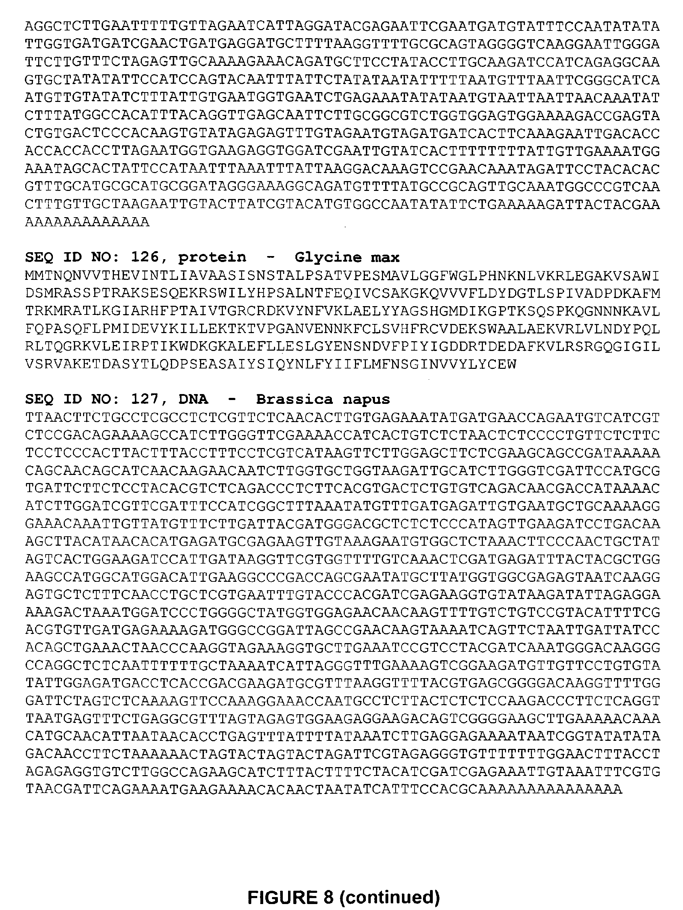

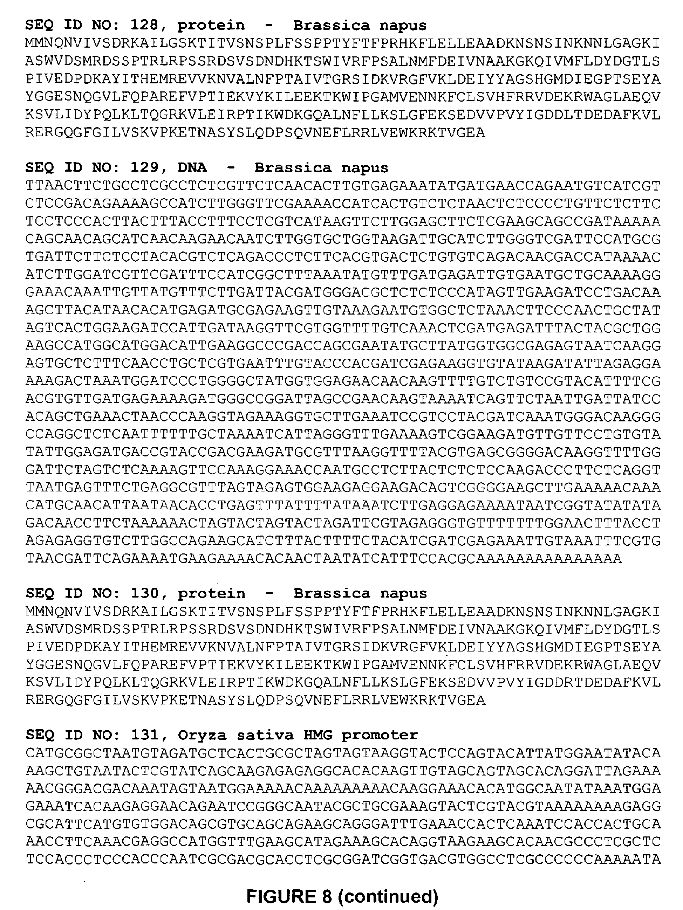

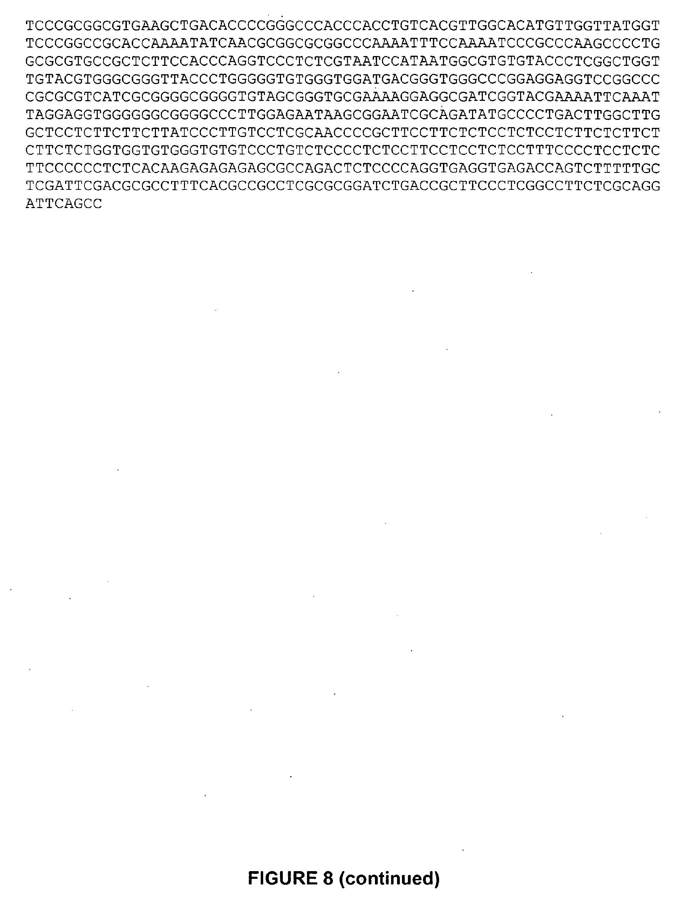

**Fig. 8** Sequence listing.

### Examples

The present invention will now be described with reference to the following examples, which are by way of illustration alone. The following examples are not intended to completely define or otherwise limit the scope of the invention.

### Example 1: Identification of sequences related to SEQ ID NO: 1 and SEQ ID NO: 2

Sequences (full length cDNA, ESTs or genomic) related to SEQ ID NO: 1 and/or protein sequences related to SEQ ID NO: 2 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program was used to find regions of local similarity between sequences by comparing nucleic acid or polypeptide sequences to sequence databases and by calculating the statistical significance of matches. The polypeptide encoded by SEQ ID NO: 1 was used for the TBLASTN algorithm, with default settings and the filter to ignore low complexity sequences set off. The output of the analysis was viewed by pairwise comparison, and ranked according to the probability score (E-value), where the score reflects the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters were adjusted to modify the stringency of the search.

Table A provides a list of nucleic acid and protein sequences related to the nucleic acid sequence as represented by SEQ ID NO: 1 and the protein sequence represented by SEQ ID NO: 2.

**Table A: Nucleic acid sequences related to the nucleic acid sequence represented by SEQ ID NO: 1 useful in the methods of the present invention, and the corresponding deduced polypeptides. For each sequence the database accession number of either the protein or the nucleic acid encoding such protein is given.**

| **Name** | **Source organism** | **NT/PROT** | **SEQ ID NO:** | **Database accession number** | **Status** |
|---|---|---|---|---|---|
| >SEQ ID NO: 2 | *Arabidopsis thaliana* | nt | 1 | NA | Full length |
| >SEQ ID NO: 2 | *Arabidopsis thaliana* | PROT | 2 | NA | Full length |
| >Ta_contig10083@1008 | *Triticum_aestivum* | nt | 3 | NA | Full length |
| >Ta_contig10083@1008 | *Triticum_aestivum* | PROT | 4 | NA | Full length |
| >Gm_contig16565 | *Glycine_max* | nt | 5 | NA | Full length |
| >Gm_contig16565 | *Glycine_max* | PROT | 6 | NA | Full length |
| >TAG_Contig-7-M6b1 | *Tagetes ssp* | nt | 7 | NA | Full length |
| >TAG_Contig-7-M6b1 | *Tagetes ssp* | PROT | 8 | NA | Full length |
| >AT1G22210 | *Arabidopsis thaliana* | nt | 9 | AT1G22210 | Full length |
| >AT1G22210 | *Arabidopsis thaliana* | PROT | 10 | AT1G22210 | Full length |
| >AT1G35910 | *Arabidopsis thaliana* | nt | 11 | AT1G35910 | Full length |
| >AT1G35910 | *Arabidopsis thaliana* | PROT | 12 | AT1G35910 | Full length |
| >AT1G78090 | *Arabidopsis thaliana* | nt | 13 | AT1G78090 | Full length |
| >AT1G78090 | *Arabidopsis thaliana* | PROT | 14 | AT1G78090 | Full length |
| >AT2G22190 | *Arabidopsis thaliana* | nt | 15 | AT2G22190 | Full length |
| >AT2G22190 | *Arabidopsis thaliana* | PROT | 16 | AT2G22190 | Full length |
| >AT4G 12430 | *Arabidopsis thaliana* | nt | 17 | AT4G12430 | Full length |
| >AT4G12430 | *Arabidopsis thaliana* | PROT | 18 | AT4G12430 | Full length |
| >AT4G22590 | *Arabidopsis thaliana* | nt | 19 | AT4G22590 | Full length |
| >AT4G22590 | *Arabidopsis thaliana* | PROT | 20 | AT4G22590 | Full length |
| >At4g39770 | *Arabidopsis thaliana* | nt | 21 | At4g39770 | Full length |
| >At4g39770 | *Arabidopsis thaliana* | PROT | 22 | At4g39770 | Full length |
| >AT5G10100 | *Arabidopsis thaliana* | nt | 23 | AT5G10100 | Full length |
| >AT5G10100 | *Arabidopsis thaliana* | PROT | 24 | AT5G10100 | Full length |
| >AT5G51460 | *Arabidopsis thaliana* | nt | 25 | AT5G51460 | Full length |
| >AT5G51460 | *Arabidopsis thaliana* | PROT | 26 | AT5G51460 | Full length |
| >AT5G65140 | *Arabidopsis thaliana* | nt | 27 | AT5G65140 | Full length |
| >AT5G65140 | *Arabidopsis thaliana* | PROT | 28 | AT5G65140 | Full length |
| >pOP-lcl\|scaff_II.875 | *Populus trichocarpa* | nt | 29 | pOP-lcl\|scaff_II.875 | Full length |
| >pOP-lcl\|scaff_II.875 | *Populus trichocarpa* | PROT | 30 | pOP-lcl\|scaff_II.875 | Full length |
| >pOP-lcl\|scaff_V.739 | *Populus trichocarpa* | nt | 31 | pOP-lcl\|scaff_V.739 | Full length |
| >pOP-lcl\|scaff_V.739. | *Populus trichocarpa* | PROT | 32 | pOP-lcl\|scaff_V.739 | Full length |
| >pOP-lcl\|scaff_VII.559 | *Populus trichocarpa* | nt | 33 | pOP-lcl\|scaff_VII.559 | Full length |
| >pOP-lcl\|scaff_VII.559 | *Populus trichocarpa* | PROT | 34 | pOP-lcl\|scaff_VII.559 | Full length |
| >pOP-llcl\|scaff_127.52 | *Populus trichocarpa* | nt | 35 | pOP-llcl\|scaff_127.52 | Full length |
| >pOP-llcl\|scaff_127.52 | *Populus trichocarpa* | PROT | 36 | POP-llcl\|scaff_127.52 | Full length |
| >pOP-llcl\|scaff_III.843 | *Populus trichocarpa* | nt | 37 | pOP-llcl\|scaff-III.843 | Full length |
| >pOP-llcl\|scaff_III.843 | *Populus trichocarpa* | PROT | 38 | pOP-llcl\|scaff_III.843 | Full length |
| >pOP-llcl\|scaff_V.167 | *Populus trichocarpa* | nt | 39 | pOP-llcl\|scaff_V.167 | Full length |
| >pOP-llcl\|scaff_V.167 | *Populus trichocarpa* | PROT | 40 | pOP-llcl\|scaff_V.167 | Full length |
| >pOP-llcl\|scaff_XII.1254 | *Populus trichocarpa* | nt | 41 | pOP-llcl\|scaff_XII.1254 | Full length |
| >pOP-llcl\|scaff_XII.1254 | *Populus trichocarpa* | PROT | 42 | pOP-llcl\|scaff_XII.1254 | Full length |
| >pOP-llcl\|scaff_XV.1052 | *Populus trichocarpa* | nt | 43 | pOP-llcl\|scaff_XV.1052 | Full length |
| >pOP-llcl\|scaff_XV.1052 | *Populus trichocarpa* | PROT | 44 | pOP-llcl\|scaff_XV.1052 | Full length |
| >0s02g0661100 | *Oryza sativa* | nt | 45 | Os02g0661100 | Full length |
| >0s02g0661100 | *Oryza sativa* | PROT | 46 | Os02g0661100 | Full length |
| >0s02g0753000 | *Oryza sativa* | nt | 47 | Os02g0753000 | Full length |
| >Os02g0753000 | *Oryza sativa* | PROT | 48 | Os02g0753000 | Full length |
| >Os03g0386500 | *Oryza sativa* | nt | 49 | Os03g0386500 | Full length |
| >Os03g0386500 | *Oryza sativa* | PROT | 50 | Os03g0386500 | Full length |
| >Os06g0222100 | *Oryza sativa* | nt | 51 | Os06g0222100 | Full length |
| >Os06g0222100 | *Oryza sativa* | PROT | 52 | Os06g0222100 | Full length |
| >Os07g0485000 | *Oryza sativa* | nt | 53 | Os07g0485000 | Full length |
| >Os07g0485000 | *Oryza sativa* | PROT | 54 | Os07g0485000 | Full length |
| >Os07g0624600 | *Oryza sativa* | nt | 55 | Os07g0624600 | Full length |
| >Os07g0624600 | *Oryza sativa* | PROT | 56. | Os07g0624600 | Full length |
| >Os09g0369400 | *Oryza sativa* | nt | 57 | Os09g0369400 | Full length |
| >Os09g0369400 | *Oryza sativa* | PROT | 58 | Os09g0369400 | Full length |
| >Os10g0553300 | *Oryza sativa* | nt | 59 | Os10g0553300 | Full length |
| >Os10g0553300 | *Oryza sativa* | PROT | 60 | Os10g0553300 | Full length |
| >Aquilegia_TC11239 | *Aquilegia ssp* | nt | 61 | Aquilegia_TC11239 | Full length |
| >Aquilegia_TC11239 | *Aquilegia ssp* | PROT | 62 | Aquilegia_TC11239 | Full length |
| >Aquilegia_TC17706 | *Aquilegia ssp* | nt | 63 | Aquilegia_TC17706 | Full length |
| >Aquilegia_TC17706 | *Aquilegia ssp* | PROT | 64 | Aquilegia_TC17706 | Full length |
| >Bc_Q4AC11 | *Brassica campestris* | nt | 65 | Bc_Q4AC11 | Full length |
| >Bc_Q4AC11 | *Brassica campestris* | PROT | 66 | Bc_Q4AC11 | Full length |
| >Br_Q4ABQ9 | *Brassica rapa* | nt | 67 | Br_Q4ABQ9 | Full length |
| >Br_Q4ABQ9 | *Brassica rapa* | PROT | 68 | Br_Q4ABQ9 | Full length |
| >Gh_TC35369 | *Gossypium_hirsutum* | nt | 69 | Gh_TC35369 | Full length |
| >Gh_TC35369 | *Gossypium_hirsutum* | PROT | 70 | Gh_TC35369 | Full length |
| >Hv_TC139314 | *Hordeum vulgare* | nt | 71 | HV_TC139314 | Full length |
| >Hv_TC139314 | *Hordeum vulgare* | PROT | 72 | HV_TC139314 | Full length |
| >Mt_TC108059 | *Medicago_truncatula* | nt | 73 | Mt_TC108059 | Full length |
| >Mt_TC108059 | *Medicago_truncatula* | PROT | 74 | Mt_TC108059 | Full length |
| >Mt_TC108097 | *Medicago_truncatula* | nt | 75 | Mt_TC108097 | Full length |
| >Mt_TC108097 | *Medicago_truncatula* | PROT | 76 | Mt_TC108097 | Full length |
| >Nb_TC7464 | *Nicotiana benthamiana* | nt | 77 | Nb_TC7464 | Full length |
| >Nb_TC7464 | *Nicotiana benthamiana* | PROT | 78 | Nb_TC7464 | Full length |
| >Nt_Q3ZTF5 | *Nicotiana tabacum* | nt | 79 | Nt_Q3ZTF5 | Full length |
| >Nt_Q3ZTF5 | *Nicotiana tabacum* | PROT | 80 | Nt_Q3ZTF5 | Full length |
| >Nt_TC7310 | *Nicotiana tabacum* | nt | 81 | Nt_TC7310 | Full length |
| >Nt_TC7310 | *Nicotiana tabacum* | PROT | 82 | Nt_TC7310 | Full length |
| >Sb_TC17204 | *Sorghum_bicolor* | nt | 83 | Sb_TC17204 | Full length |
| >Sb_TC17204 | *Sorghum_bicolor* | PROT | 84 | Sb_TC17204 | Full length |
| >St_TC151769 | *Solanum_tuberosum* | nt | 85 | St_TC151769 | Full length |
| >St_TC151769 | *Solanum_tuberosum* | PROT | 86 | St_TC151769 | Full length |
| >Ta_TC252250 | *Triticum_aestivum* | nt | 87 | Ta_TC252250 | Full length |
| >Ta_TC252250 | *Triticum_aestivum* | PROT | 88 | Ta_TC252250 | Full length |
| >Zm_ABD92779 | Zea *mays* | nt | 89 | Zm_ABD92779 | Full length |
| >Zm_ABD92779 | *Zea mays* | PROT | 90 | Zm_ABD92779 | Full length |
| >Zm_ABD92780 | Zea *mays* | nt | 91 | Zm_ABD92780 | Full length |
| >Zm_ABD92780 | *Zea mays* | PROT | 92 | Zm_ABD92780 | Full length |

| | | | | | |
|---|---|---|---|---|---|
| NA. It does not apply. Not found in public databases. nt: nucleotide sequence PROT: protein sequence | | | | | |

### Example 2: Alignment of Class III TPP polypeptide sequences

Alignment of Class III TPP polypeptide sequences was performed using the AlignX program from the Vector NTI (Invitrogen) is based on the popular Clustal algorithm of progressive alignment (Thompson et al. (1997) Nucleic Acids Res 25:4876-4882; Chenna et al. (2003). Nucleic Acids Res 31:3497-3500). A phylogenetic tree was constructed using a neighbourjoining clustering algorithm, using default values (gap open penalty of 10, gap extension penalty of 0,1 and selected weight matrix is Blosum 62 (if polypeptides are aligned)).

### Example 3: Calculation of global percentage identity between polypeptide sequences useful in performing the methods of the invention

Global percentages of similarity and identity between some of the full length polypeptide sequences given in Table A of Example 1 were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program was used to perform a series of pair-wise alignments using the Myers and Miller global alignment algorithm (with a gap opening penalty of 12, and a gap extension penalty of 2), and to calculate similarity and identity using Blosum 62 (for polypeptides) and to place the results in a distance matrix. Sequence similarity is shown in the bottom half of the dividing line and sequence identity is shown in the top half of the diagonal dividing line.

Parameters used in the comparison were:
**Scoring matrix: Blosum62**
First Gap: 12
Extending gap: 2

Results of the software analysis are shown in Table B for the global similarity and identity as displayed below and above the diagonal respectively. Global similarity of SEQ ID NO:2 to the paralogous proteins shown Table B1 is above 48% identity. Similarity of SEQ ID NO:2 to the plant homeologous sequences given in Tables B2 and B3 is above 40 % identity for proteins of dicotyledoneous plant origin and above 45% identity for monocotyledoneous.

**Table B**: MatGAT results for global similarity and identity over the full length of the polypeptide sequences.

**Table B1. Global similarity and identity amongst paralogous Class III TPP polypeptides in Arabidopsis thaliana.**

| | **1** | **2** | **3** | **4** | **5** | **6** | **7** | **8** | **9** |
|---|---|---|---|---|---|---|---|---|---|
| 1. AT2G22190 | | 48.5 | 64 | 61.8 | 57.2 | 48.6 | 48.8 | 51.9 | 55.3 |
| 2. AT5G51460 | 65.2 | | 48.6 | 48.6 | 51.5 | 59.1 | 61 | 45 | 48.2 |
| 3. AT5G65140 | 78.1 | 69.1 | | 74.1 | 60.2 | 47.1 | 47.3 | 51.1 | 60.1 |
| 4. AT5G10100 | 75.6 | 66.5 | 88.4 | | 57.3 | 45.7 | 47.2 | 50.3 | 55.1 |
| 5. AT1G35910 | 72.1 | 70.4 | 78.1 | 76.7 | | 50.9 | 52 | 54.2 | 59.7 |
| 6. AT4G22590 | 65.5 | 77.1 | 67.9 | 67.9 | 69.5 | | 81.2 | 45.8 | 47.7 |
| 7. AT4G12430 | 66.3 | 76.1 | 67.6 | 69.4 | 72.1 | 89.7 | | 46.7 | 49.4 |
| 8.AT1G22210 | 67.5 | 60.3 | 66.8 | 65.3 | 68.8 | 61.5 | 63.3 | | 58.5 |
| 9. AT1G78090 | 69.5 | 68.6 | 77 | 74.1 | 77.3 | 68.4 | 67.9 | 73.5 | |
| 10. SEQ ID NO: 2 | 69.5 | 68.6 | 77 | 74.1 | 77.3 | 68.4 | 67.9 | 73.3 | 100 |

**Table B2. Global similarity and identity amongst homologous Class III TPP polypeptides from dicotyledonous plant origin.**

| **Protein** | **1** | **2** | **3** | **4** | **5** | **6** | **7** | **8** | **9** | **10** | **11** | **12** | **13** | **14** |
|---|---|---|---|---|---|---|---|---|---|---|---|---|---|---|
| 1. Nt_Q3ZTF5 | | 49.7 | 49.4 | 50.5 | 50.8 | 50.6 | 44 | 73.7 | 74.6 | 68.2 | 50.4 | 65 | 75.6 | 60.7 |
| 2. Bc_Q4AC11 | 68.2 | | 81.7 | 57.7 | 57.2 | 62.5 | 56.2 | 47.4 | 46 | 47.3 | 60.6 | 47.8 | 46.9 | 44.4 |
| 3. Br_Q4ABQ9 | 67.7 | 88.7 | | 57.7 | 57.7 | 58.5 | 53.2 | 46 | 45.6 | 47.5 | 60.7 | 46.7 | 45.4 | 43.8 |
| 4. pOP-lcl\|scaff_II.875 | 65.6 | 75.7 | 75.1 | | 89.9 | 62 | 58.1 | 49.5 | 49.6 | 49.1 | 59.1 | 47.9 | 50.1 | 46.4 |
| 5. pOP-lcl\|scaff_V.739 | 66.9 | 75.5 | 74.3 | 94.7 | | 60.1 | 55.9 | 50.1 | 49.5 | 50 | 57 | 48.1 | 50 | 46 |
| 6. pOP-lcl\|scaff_VII.559 | 66.4 | 75.7 | 71.8 | 74.5 | 74.3 | | 81.9 | 48.3 | 47.6 | 46.5 | 61.5 | 47.3 | 49.1 | 44.1 |
| 7. pOP-llcl\|scaff_V.167 | 63.1 | 72.9 | 68.6 | 72.1 | 71.9 | 88 | | 42 | 41.7 | 41.8 | 57.4 | 42.7 | 42.6 | 40.5 |
| 8. pOP-llcl\|scaff_XV.1052 | 85 | 66.6 | 63 | 63.2 | 64.8 | 66.7 | 62.3 | | 87.1 | 69.1 | 49.1 | 64.6 | 74.4 | 57.8 |
| 9. pOP-llcl\|scaff_XII.1254 | 87.1 | 66.4 | 63.6 | 63.8 | 65.4 | 66.2 | 61.8 | 92.5 | | 68.5 | 49.2 | 64.7 | 75 | 57.1 |
| 10. pOP-llcl\|scaff_127.52 | 83.1 | 66.8 | 64.2 | 63.4 | 66.1 | 64.2 | 63.3 | 82.1 | 82.9 | 48.6 | 48.6 | 85.2 | 67.2 | 54.7 |
| 11. Gh_TC35369 | 62.8 | 70.4 | 70.7 | 70.4 | 70.6 | 67.6 | 64.3 | 60.9 | 61 | 60 | | 48.2 | 49.9 | 57.1 |
| 12. pOP-llcl\|scaff_III.843 | 80.8 | 64.5 | 63.7 | 65.3 | 64.2 | 63.7 | 62.3 | 80.3 | 81.4 | 91.5 | 59.6 | | 64.9 | 53.4 |
| 13. Gm_contig16565 | 85.9 | 67.4 | 63 | 63.5 | 64.5 | 65.4 | 63.8 | 85.9 | 86.4 | 83 | 61.4 | 81.7 | | 57.8 |
| 14. TAG_Contig-7-M6b1 | 68 | 60.6 | 60.8 | 61.4 | 62.5 | 56.6 | 53.3 | 67.6 | 65.9 | 65.8 | 76 | 64 | 66.1 | |
| 15. SEQ ID NO: 2 | 69.3 | 71.9 | 70.1 | 74.9 | 76.7 | 70.8 | 69.1 | 69.2 | 69.5 | 68.4 | 66 | 66.1 | 68.4 | 61.5 |

**Table B3. Global similarity and identity amongst homologous Class III TPP polypeptides from monocotyledonous plant origin.**

| | **1** | **2** | **3** | **4** | **5** | **6** | **7** | **8** | **9** | **10** | **11** | **12** | **13** | **14** |
|---|---|---|---|---|---|---|---|---|---|---|---|---|---|---|
| 1. Os02g0753000 | | 63 | 42.9 | 51.9 | 48 | 51.8 | 49.9 | 47.3 | 47.3 | 62.1 | 54.3 | 48.7 | 57.1 | 57.1 |
| 2. Os08g0409100 | 77 | | 38.2 | 56.2 | 59.9 | 50.8 | 52.5 | 51.6 | 49.4 | 77 | 63.7 | 52.6 | 59.4 | 56.4 |
| 3. Os06g0222100 | 50.8 | 46.3 | | 34.2 | 35 | 33.4 | 31.2 | 28.6 | 29.8 | 37.1 | 31.9 | 31.5 | 34.9 | 35.1 |
| 4. Os07g0624600 | 71.1 | 71.4 | 43.5 | | 45 | 52.9 | 50.4 | 48.3 | 47.3 | 53.9 | 49.3 | 48.4 | 64.7 | 63.4 |
| 5. Os09g0369400 | 58.8 | 69.2 | 48.7 | 57.8 | | 41.5 | 42 | 39.3 | 37.5 | 65.7 | 50.9 | 39.9 | 47.2 | 46.5 |
| 6. Os03g0386500 | 66.2 | 64.9 | 40.3 | 64.5 | 51.1 | | 45.3 | 43.6 | 44.9 | 50.5 | 56.5 | 45 | 55.9 | 55.3 |
| 7. Os02g0661100 | 66.6 | 70.6 | 41.9 | 65.5 | 55.8 | 58.2 | | 51.9 | 55.7 | 50.4 | 46.1 | 84.5 | 50.4 | 49.6 |
| 8. Os07g0485000 | 65.7 | 67 | 40.1 | 63.4 | 51.7 | 60.2 | 68.5 | | 53.9 | 48.1 | 46.7 | 50.6 | 47.2 | 48.5 |
| 9. Os10g0553300 | 65.4 | 66.5 | 40.4 | 64.4 | 53.4 | 58.1 | 72 | 66.5 | | 47.8 | 43.3 | 55.5 | 47.7 | 49.1 |
| 10. Ta_TC252250 | 75.9 | 88.5 | 47.1 | 69.7 | 71.3 | 63.5 | 69.2 | 64.6 | 66.2 | | 74.5 | 50.5 | 57.7 | 55.8 |
| 11. HV_TC139314 | 65.7 | 71.9 | 39.8 | 61.7 | 55.6 | 72.3 | 59 | 60.7 | 56.5 | 75.6 | | 45.7 | 53.6 | 50.4 |
| 12. Ta_contig10083 | 66.9 | 68 | 41.8 | 64.2 | 54.7 | 58.1 | 91.7 | 67.2 | 71.5 | 69.2 | 58.3 | | 48.3 | 47.2 |
| 13. Zm_SRA ABD92780 | 71.4 | 71.4 | 42.4 | 74.3 | 57.5 | 68.5 | 63.6 | 65.5 | 64.1 | 69.2 | 65.1 | 62.9 | | 65.5 |
| 14. Zm_RA3 | 74.1 | 74.3 | 43.8 | 75.7 | 57.3 | 66.5 | 65.5 | 66.8 | 66.2 | 71.6 | 64.5 | 62.6 | 78.1 | |
| 15. SEQ ID NO: 2 | 73.3 | 74.3 | 46.5 | 68.7 | 58.2 | 61.5 | 69.8 | 65.2 | 68.3 | 72.5 | 61.2 | 70.1 | 67.1 | 66.3 |

**Table B4. Global similarity and identity amongst homologous Class III TPP polypeptides from non-plant origin.**

| | **1** | **2** | **3** | **4** | **5** | **6** | **7** | **8** | **9** | **10** | **11** | **12** | **13** | **14** |
|---|---|---|---|---|---|---|---|---|---|---|---|---|---|---|
| 1. E.coli_OTSBP | | 36.8 | 36.4 | 35.8 | 29.2 | 30.7 | 23.5 | 26.1 | 11.3 | 36.8 | 36.4 | 29.2 | 30.7 | 23.5 |
| 2. Xo_Q5H2G4 | 54.1 | | 38 | 38.9 | 33.5 | 28.8 | 24.5 | 25.1 | 10.8 | 100 | 38 | 33.5 | 28.8 | 24.5 |
| 3. 03IZA2_RHOS4 | 55.3 | 53.1 | | 46.5 | 32 | 28.6 | 23.3 | 25.2 | 10.8 | 38 | 100 | 32 | 28.6 | 23.3 |
| 14. RHILE_087819 | 53.4 | 57.1 | 61.2 | | 31 | 26.4 | 23.3 | 24.9 | 11.3 | 38.9 | 46.5 | 31 | 26.4 | 23.3 |
| 5. THETH_Q5MCN5 | 47.4 | 48.4 | 45.8 | 44.4 | | 27.1 | 30.3 | 27 | 10.5 | 32.8 | 32 | 100 | 27.1 | 30.3 |
| 6. Arthr_Q4NG94 | 46.7 | 41.2 | 42.3 | 40.1 | 43.4 | | 26.3 | 27.7 | 9.6 | 28.7 | 28.3 | 27.1 | 100 | 26.3 |
| 7. DROME_Q9VM18 | 44.6 | 42 | 43.5 | 44.6 | 46.7 | 45.7 | | 40.6 | 10.9 | 24.5 | 23.3 | 30.3 | 26.4 | 100 |
| 8. DROME_Q9VM19 | 42.9 | 39.9 | 45.1 | 44 | 45.8 | 46.5 | 64.9 | | 10.6 | 25.1 | 25.2 | 27 | 27.7 | 40.6 |
| 9. Sc_P31688\|TPS2 | 17.5 | 16.9 | 17 | 16.4 | 15.2 | 15.1 | 17.7 | 16.6 | | 11.1 | 10.8 | 10.5 | 9.8 | 10.9 |
| 10. Xo_Q5H2G4 | 54.1 | 100 | 53.1 | 57.1 | 47.6 | 41.5 | 42 | 39.9 | 17 | | 38 | 33.5 | 28.8 | 24.5 |
| 11. Q3IZA2\|Q3IZA2_RHOS4 | 55.3 | 53.1 | 100 | 61.2 | 45.8 | 41.9 | 43.5 | 45.1 | 17 | 53.1 | | 32 | 28.6 | 23.3 |
| 12. THETH_Q5MCN5 | 47.4 | 48.4 | 45.8 | 44.4 | 100 | 43.4 | 46.7 | 45.8 | 15.6 | 48.4 | 45.8 | | 27.1 | 30.3 |
| 13. Arthr_Q4NG94 | 46.7 | 41.2 | 42.3 | 40.1 | 43.4 | 100 | 44.2 | 46.5 | 15.1 | 41.2 | 42.3 | 43.4 | | 26.3 |
| 14. DROME_Q9VM18 | 44.6 | 42 | 43.5 | 44.6 | 46.7 | 45.7 | 100 | 64.9 | 17.7 | 42 | 43.5 | 46.7 | 45.7 | |
| 15. SEQ ID NO: 2 | 37.4 | 34 | 37.2 | 34 | 36.9 | 38.5 | 41.7 | 41.2 | 23.7 | 34 | 37.2 | 36.9 | 38.5 | 41.7 |

### Example 4: Identification of domains comprised in Class III TPP polypeptides

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Interpro is hosted at the European Bioinformatics Institute in the United Kingdom.

The results of the InterPro scan of the polypeptide sequence as represented by *SEQ ID NO:* 14 are presented in Table C.

**Table C: InterPro scan results of the polypeptide sequence as represented by SEQ ID NO: 14.**

| **Database** | **Accession number** | **Accession name** | **Region of homology to the domain** |
|---|---|---|---|
| TIGRFAMs | TIGR01484 | HAD-SF-IIB: HAD-superfamily hydrolase | 119-332 |
| Pfam | PF02358 | Trehalose_PPase | 121-354 |
| TIGRFAMs | TIGR00685 | T6PP: trehalose-phosphatase | 115-365 |

### Example 5: In vitro activity assay for Trehalose Phosphate Phosphatase activity

The *Arabidopsis* Class III TPP polynucleotide corresponding to the coding region of AtTPPI isoform was cloned into the pGEX-4T-1 (Pharmacia) plasmid, designed to create GST-fusion proteins. The vector was introduced into *E. coli.* The expressed recombinant protein produced in the *E. coli* strain BL21 was purified and the TPP activity determined by HPLC.

### Expression in E. coli

Supercompetent E. *coli* strain BL21 Star™(DE3) One Shot^{®} (Invitrogen) cells were transformed with the pGEX-GST construct and incubated overnight on Luria Bertani (LB) medium + ampicilline (Amp) plates at 37°C. Colonies were collected and transferred to200 ml LB+Amp and incubated shaking at 37°C for 4h before induction of expression by adding isopropyl-β-thiogalactoside (IPTG, 0.3 mM final concentration). This was followed by an another incubation period of 4h at 30°C. Cells were collected by centrifugation at 4°C (30 min, 3000 rpm) and resuspended in 5 ml ice-cold 1x Phosphate-buffered saline (PBS) buffer (140 mM NaCl; 2.7 mM KCl; 10 mM Na₂HPO₄; 1.8 mM KH₂PO₄; pH 7.3), aliquoted in 5 chilled screw cap tubes. After centrifugation at 4°C (5 min, 6000 rpm), pellets were frozen in liquid nitrogen, and stored at-80°C.

### Protein purification

The cells were washed once with 5 ml ice-cold lysis buffer (1 x PBS; 0.4% Triton; 2 mM MgCl₂, 1 mM EDTA, 2 mM dithiothreitol (DTT); 0.2 mg/ml lysozyme and 1 tablet EDTA protease-inhibitor mix (Roche)/10 ml lysis buffer), incubated for 15 min on ice, and sonicated by three 15-s pulses of sonication (Sartorius Labsonic^{®}P). Lysates were cleared at 4°C by centrifugation at 12,000g. The supernatant fraction was mixed with 200 µl glutathion-sepharose beads (Amersham Biosciences) that had been pre-equilibrated in wash buffer (1x PBS; 0.1% Triton; 2 mM MgCl₂, 1 mM EDTA, 1 mM DTT), and incubated for 1h at 4°C in a rollerdrum. Beads were collected by centrifugation at 4°C (1 min, 1800 rpm), washed five times with 1 ml ice-cold wash buffer and stored at -20°C before proceeding with TPP activity measurements.

### TPP activity measurement

Trehalose-6-phosphate phosphatase (TPP) activity was detected by measuring the produced trehalose (T) after adding Trehalose-6-phosphate (T6P) to the protein samples. Samples were added to 150 µl assay buffer with T6P (2 mM T6P; 2 mM MgCl₂; 10 µM Validamycin A and 45 mM Tris-HCl, pH 7.5) or without T6P (2 mM MgCl₂; 10 µM Validamycin A and 45 mM Tris-HCl, pH 7.5). After incubation for 1-1.5h at 37°C, the beads were collected by centrifugation (10 min; 1,200g) and the supernatant was injected into the HPLC, together with a T standard dilution. Protein concentrations were measured by the Bradford method.

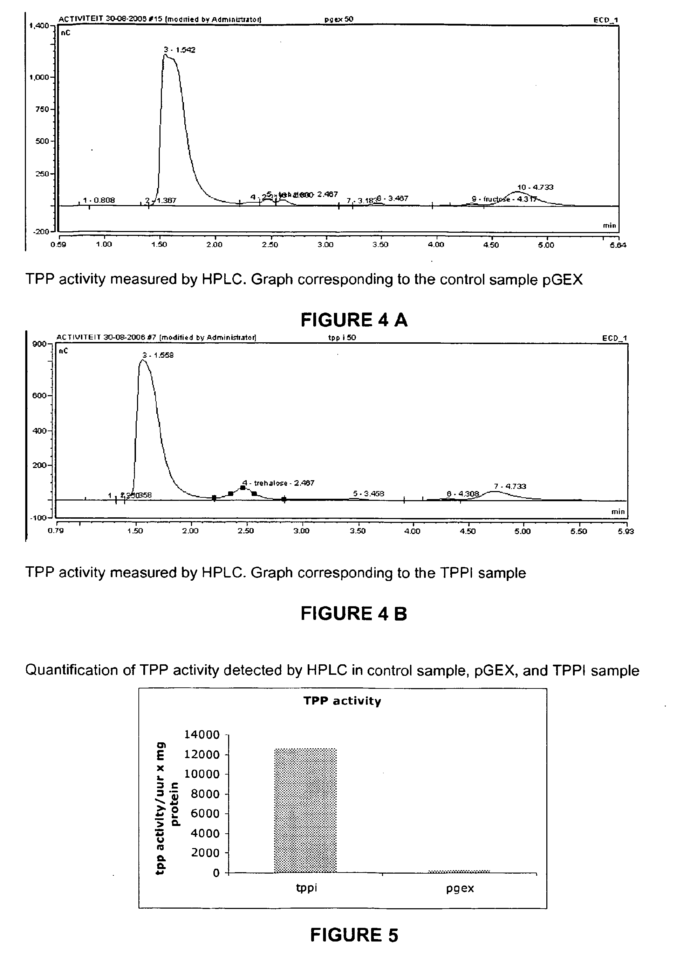

The results as indicated in Figure 4 showed that TPPI has trehalose-6-phosphate phosphatase activity.

### Example 6: In vivo activity assay for Trehalose Phosphate Phosphatase

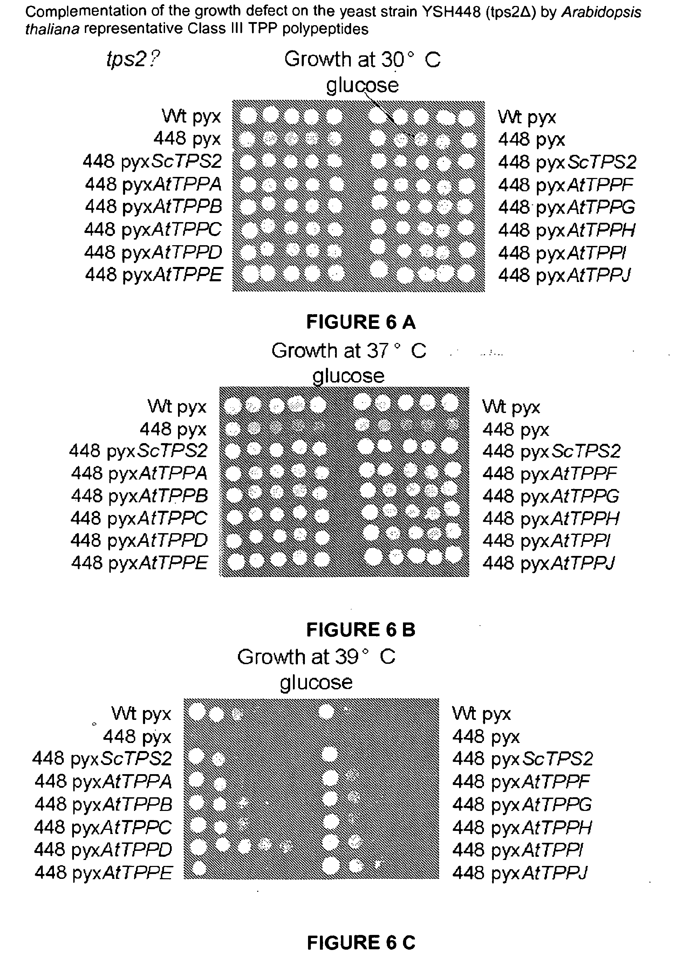

The activity of the enzyme represented by SEQ ID NO: 2 and that of its paralogous proteins having sequences represented by SEQ ID NOs: 10, 12, 16, 18, 20, 22, 24, 26 and 28 was determined by functional complementation of the yeast tps2 mutant according to the protocol described by Vogel et al. 1998 with minor modifications. Briefly, a DNA fragment comprising the coding regions of any of the sequences represented by SEQ ID No:1, 9, 11, 15, 17, 19, 21, 23, 25 and 27 amplified from *Arabidopsis thaliana* cDNA by the polymerase chain reaction using the primers listed in Table D. The DNA fragments subsequently cloned in the yeast expression vector pYX212. The constructs were introduced into the thermosensitive yeast tps2-deficient strain YSH448. The transformed yeast strains were grown at the the permissive temperature of 30 C and the restrictive conditions of 38 C and 39 C in SD-URA glucose medium. As shown in the figure 6 only constructs comprising the coding region of any of SEQ ID No: 2, 10, 12, 16, 18, 20, 22, 24, 26 and 28 were able to restore the growth of the yeast at the restrictive temperatures of 37 C and 39 C. The yeast transformants containing the empty vector pYX212 did not restored the growth of the tps2 deficient strain YSH448. The yeast transformants expressing the wild type yeast TPS2 gene from the vector pYX212 and the empty pYX212 was used as positive and negative controls respectively.

**Table D: List of primers used in the PCR to amplify Class III Polypeptides from Arabidopsis thaliana. Stop codon was provided in the cloning vector.**

| **prm name** | **origin** | **SEQ ID NO:** | **Sequence** |
|---|---|---|---|
| >PrmAtTPPA-5 | Synthetic | 101 | GGAAGATCTATGGACATGAAATCTGGTCACTC |
| >PrmAtTPPA-3 | Synthetic | 102 | AAGGCCTACCCATTGATCTCTTCCATGTCA |
| >PrmAtTPPB-5 | Synthetic | 103 | GGAATTCATGACTAACCAGAATGTCATCGTT |
| >PrmAtTPPB-3 | Synthetic | 104 | AAGGCCTCTCTTCTCCCACTGTCTTCCTC |
| >PrmAtTPPC-5 | Synthetic | 105 | CGGGATCCATGAAGATTACGGATATTTCCGG |
| >PrmAtTPPC-3 | Synthetic | 106 | AAGGCCTTTCTCCAAGTGTTTGTTTCTTCC |
| >PrmAtTPPD-5 | Synthetic | 107 | CGGGATCCATGACAAACCATAATGCCTTAATC |
| >PrmAtTPPD-3 | Synthetic | 108 | AAGGCCTTCTTCCTCTTAGTGACATTTGTTTC |
| >PrmAtTPPE-5 | Synthetic | 109 | CGGGATCCATGTTCGAAGAAATACTTCATAAATC |
| >PrmAtTPPE-3 | Synthetic | 110 | AAGGCCTTGCCCCACACCTTGACTGTTTC |
| >PrmAtTPPF-5 | Synthetic | 111 | CATGCCATGGATTTAAACTCAAACCACAAATC |
| >PrmAtTPPF-3 | Synthetic | 112 | TCCCCCGGGAAAACCAGTAGAATTCTTCTCCAAC |
| >PrmAtTPPG-5 | Synthetic | 113 | CATGCCATGGATTTGAATATAAACAAGACGAC |
| >PrmAtTPPG-3 | Synthetic | 114 | AAGGCCTAAAACTTGTTTTTGAACTTTCCATCTTC |
| >PrmAtTPPH-5 | Synthetic | 115 | CGGGATCCATGGTTAGATTCATAGAAGAAAACAC |
| >PrmAtTPPH-3 | Synthetic | 116 | AAGGCCTTGCTCCAGATCTCAATTGTTTCC |
| >PrmAtTPPI-5 | Synthetic | 117 | CGGGATCCATGTCAGCTAGTCAAAACATTGTC |
| >PrmAtTPPI-3 | Synthetic | 118 | AAGGCCTCATTCTTGGCTGCATTTGTTTCC |
| >PrmAtTPPJ-5 | Synthetic | 119 | CGGGATCCATGGTGAGCCAAAACGTCGTCG |
| >PrmAtTPPJ-3 | Synthetic | 120 | AAGGCCTTTGCTGCATCTGTTTCCACTCC |

### Example 7: Cloning of nucleic acid sequence as represented by SEQ ID NO: 1

Unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

The *Arabidopsis thaliana Class III TPP* gene was amplified by PCR using as template an *Arabidopsis thaliana* seedling cDNA library (Invitrogen, Paisley, UK). Primers prm05451 (SEQ ID NO: 99, primer; sense, start codon in bold, AttB1 site in italic: 5'-ggggacaagtttgtacaa aaaagcaggcttaaaca**atg**actaaccagaatgtcatc-3') and prm05452 (SEQ ID NO: 100, primer2; reverse, complementary, AttB2 site in italic: 5'*-ggggaccactttgtacaagaaagctgggt*tgtaattatgtt gcatgtctt-3'), which include the AttB sites for Gateway recombination, were used for PCR amplification. PCR was performed using Hifi Taq DNA polymerase in standard conditions. A PCR fragment of the expected length was amplified and purified also using standard methods. The first step of the Gateway procedure, the BP reaction, was then performed, during which the PCR fragment recombines in vivo with the pDONR201 plasmid to produce, according to the Gateway terminology, an "entry clone", pEC_*Class III TPP.* Plasmid pDONR201 was purchased from Invitrogen, as part of the Gateway^{®} technology.

### Example 8: Expression vector construction using the nucleic acid sequence as represented by SEQ ID NO: 1

The entry clone pEC_*Class III TPP* was subsequently used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 98) for constitutive expression was located upstream of this Gateway cassette.

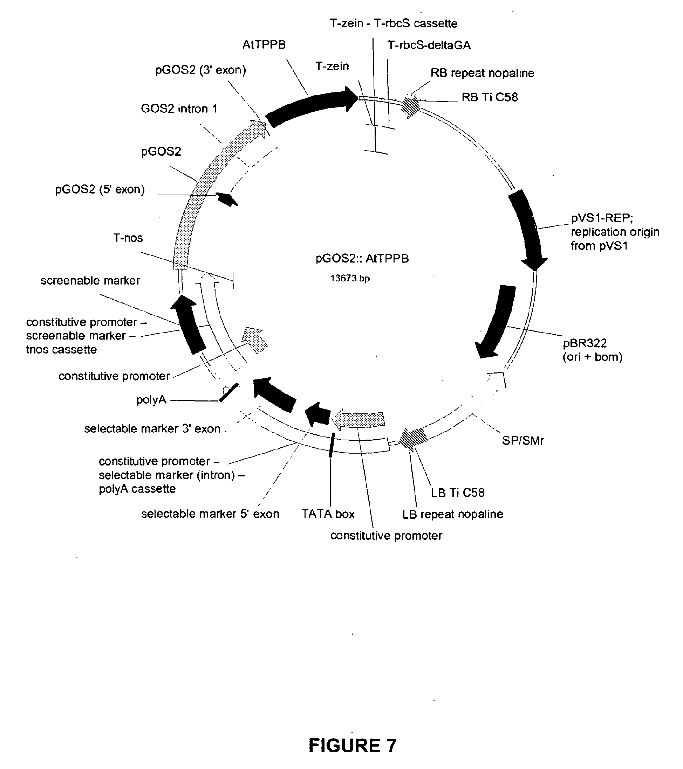

After the LR recombination step, the resulting expression vector pXC*_Class III TPP* (Figure 7) was transformed into *Agrobacterium* strain LBA4044 according to methods well known in the art.

### Example 9: Plant transformation

### Rice transformation

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2%HgCl₂, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

*Agrobacterium* strain LBA4404 containing the expression vector was used for cocultivation. *Agrobacterium* was inoculated on AB medium with the appropriate antibiotics and cultured for 3 days at 28°C. The bacteria were then collected and suspended in liquid co-cultivation medium to a density (OD600) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan *et al.* 1993, Hiei *et al.* 1994).

### Corn transformation

Transformation of maize (Zea *mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Wheat transformation

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown *in vitro* on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Soybean transformation

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

### Rapeseed/canola transformation

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for *in vitro* sowing. The cotyledon petiole explants with the cotyledon attached are excised from the *in vitro* seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

### Alfalfa transformation

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

### Cotton transformation

Cotton is transformed using *Agrobacterium tumefaciens* according to the method described in US 5,159,135. Cotton seeds are surface sterilised in 3% sodium hypochlorite solution during 20 minutes and washed in distilled water with 500 µg/ml cefotaxime. The seeds are then transferred to SH-medium with 50µg/ml benomyl for germination. Hypocotyls of 4 to 6 days old seedlings are removed, cut into 0.5 cm pieces and are placed on 0.8% agar. An *Agrobacterium* suspension (approx. 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 µg/ml MgCL2, and with 50 to 100 µg/ml cefotaxime and 400-500 µg/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants are hardened and subsequently moved to the greenhouse for further cultivation.

### Example 10: Phenotypic evaluation procedure

### 10.1 Evaluation setup

Approximately 35 independent T0 rice transformants were generated. The primary transformants were transferred from a tissue culture chamber to a greenhouse for growing and harvest of T1 seed. Five events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

Four T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

### Drought screen

Plants from T2 seeds were grown in potting soil under normal conditions until they approached the heading stage. They were then transferred to a "dry" section where irrigation was withheld. Humidity probes were inserted into randomly chosen pots to monitor the soil water content (SWC). When SWC went below certain thresholds, the plants were automatically watered until a normal level was reached. The plants were then transferred back to normal conditions. The rest of the cultivation (plant maturation, seed harvest) was the same as for plants not grown under abiotic stress conditions. Growth and yield parameters were recorded as detailed for growth under normal conditions.

### Nitrogen use efficiency screen

Rice plants from T2 seeds were grown in potting soil under normal conditions except for the nutrient solution. The pots were watered from transplantation to maturation with a specific nutrient solution containing reduced N nitrogen (N) content, usually between 7 to 8 times less than normal (control) plants. The rest of the cultivation (plant maturation, seed harvest) was the same as for plants not grown under abiotic stress. Growth and yield parameters are recorded as detailed for growth under normal conditions.

### 10.2 Statistical analysis: F-test

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify for an overall effect of the gene, also known as a global gene effect. The threshold for significance for a true global gene effect was set at a 5% probability level for the F-test. A significant F-test value points to a gene effect, meaning that it is not only the mere presence or position of the gene that is causing the differences in phenotype.

### 10.3 Parameters measured

### Biomass-related parameter measurement

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass.

Plant early vigour is estimated from the plant (seedling) aboveground area three weeks post-germination. Increase in root biomass is expressed as an increase in total root biomass (measured as maximum biomass of roots observed during the exponential growth phase of the roots); or as an increase in the root/shoot index (measured as the ratio between root mass and shoot mass in the period of active growth of root and shoot).

### Seed-related parameter measurements

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area (mm²), multiplied by a factor 10⁶. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

### Example 11: Results of the phenotypic evaluation of the transgenic plants

The results of the evaluation of transgenic rice plants expressing the nucleic acid sequence of SEQ ID NO: 1 under the control of a GOS2 promoter are presented in Table E. The percentage difference between the transgenics and the corresponding nullizygotes is also shown, with a P value from the F test below 0.05.

Total seed yield, number of filled seeds, seed fill rate and harvest index were significantly increased in the transgenic plants expressing the nucleic acid sequence of SEQ ID NO: 1, compared to the control plants (in this case, the nullizygotes).

**Table E: Results of the evaluation of transgenic rice plants expressing the nucleic acid sequence of SEQ ID NO: 1 under the control of a GOS2 promoter**

| **Trait** | **% Increase in the transgenic versus control plants** |
|---|---|
| Total seed yield | 15 |
| Number of filled seeds | 17 |
| Number of total seeds | 11 |
| Harvest index | 7 |
| Flowers per panicle | 5 |
| Aboveground Biomass | 6 |

Transgenic rice plants expressing the nucleic acid sequence of SEQ ID NO: 1 under the control of a GOS2 promoter were also subjected to a drought screen. The following parameters were increased compared to corresponding control plants and parameters marked * gave a P value from the F test of below 0.05. *Total seed yield (11% increase compared to control plants), *number of filled seeds (10% increase compared to control plants), *harvest index (11% increase compared to control plants), fill rate (8% increase compared to control plants).

**Table F: Results of the evaluation of transgenic rice plants subjected to the nitrogen use efficiency screen and expressing the nucleic acid sequence of SEQ ID NO: 1 under the control of a GOS2 promoter.**

| Plants subjected to the nitrogen use efficiency screen showed an increase in the parameters described in the table below compared to corresponding control plants. | |
|---|---|
| Parameter | % increase |
| Aboveground biomass | 9.8 |
| Root biomass | 11.4 |
| Total seed yield | 11.2 |
| No. filled seeds | 25.9 |
| No. flowers per panicle | 6.1 |
| Plant Height | 13.4 |
| Total no. seeds | 9.5 |

**Table G: Results of the evaluation of transgenic rice plants subjected to the nitrogen screen and expressing the nucleic acid sequence of SEQ ID NO: 1 under the control of an HMG promoter (High Mobility Group).**

| Plants subjected to the nitrogen use efficiency screen showed an increase in the parameters described in the table below compared to corresponding control plants. | |
|---|---|
| Parameter | % percentage increase |
| Emergence Vigour | 18.2 |
| No. filled seeds | 32.2 |

## Claims

1. A method for increasing seed yield in plants relative to control plants,
wherein said increased seed yield is any one or more of the following:
(i) Increased number of seeds per plant;
(ii) Increased number of filled seeds per plant;
(iii) Increased seed weight per plant;
comprising increasing expression in a plant of a nucleic acid encoding a Class III Trehalose Phosphate Phosphatase (TPP) polypeptide, wherein said increased expression is effected by introducing and expressing in a plant a nucleic acid encoding a Class III TPP polypeptide,
wherein said a nucleic acid encodes a Class III TPP polypeptide having at least 85% sequence identity to the amino acid sequence given in SEQ ID NO: 4, or
wherein said Class III TPP polypeptide comprises:
(i) a Trehalose-PPase domain having at least 50% amino acid sequence identity to SEQ ID NO: 93 or SEQ ID NO: 94, wherein said Trehalose-PPase domain comprises:
a. a Phosphatase Box A having at least 50% sequence identity to SEQ ID NO: 96; and/or
b. a Phosphatase Box B having at least 50% sequence identity to SEQ ID NO: 97; and/or
(ii) a Serine-rich domain having at least 50% sequence identity to SEQ ID NO: 95,
wherein said control plants are corresponding wild-type plants or corresponding plants without said nucleic acid encoding said class III trehalose phosphate polypeptide.

2. Method according to claim 1, wherein said nucleic acid encoding a Class III TPP protein :
(i) is the nucleic acid having SEQ ID NO: 3 or a portion thereof being at least 625 consecutive nucleotides in length, or a sequence capable of hybridising under stringent conditions with nucleic acid having SEQ ID NO: 4 , and/or
(ii) encodes a Class III TPP polypeptide having SEQ ID NO: 4.

3. Method according to any one of claims 1 to 2, wherein said nucleic acid is operably linked to a constitutive promoter.

4. Plant or part thereof, including seeds, obtainable by a method according to any one of claims 1 to 4 wherein said plant or part thereof comprises a recombinant nucleic acid encoding a Class III TPP protein of SEQ ID NO: 4, operably linked to a constitutive promoter.

5. An isolated nucleic acid molecule comprising:
(i) a nucleic acid of SEQ ID NO: 3;
(ii) the complement of the SEQ ID NO given in (i);
(iii) a nucleic acid encoding a Class III TPP protein having at least 90% sequence identity to the amino acid sequence given in SEQ ID NO: 4,

6. An isolated polypeptide comprising:
(i) an amino acid sequence of SEQ ID NO: 4;
(ii) an amino acid sequence having at least 90% sequence identity to the amino acid sequence given in SEQ ID NO: 4;

7. Construct comprising:
(a) a nucleic add encoding a Class III TPP polypeptide according to claim 6; and
(b) one or more control sequences capable of driving expression of the nucleic acid sequence of (a), wherein said one control sequence is a GOS2 promoter; and
(c) optionally a transcription termination sequence.

8. Use of a construct according to claim 7 in a method for making plants having increased seed yield relative to control plants, wherein said seed yield is one or more of the following:
(i) Increased number of seeds per plant;
(ii) Increased number of filled seeds per plant;
(iii) Increased (total) seed weight per plant,
wherein said control plants are corresponding wild-type plants or corresponding plants without said nucleic acid encoding said class III trehalose phosphate polypeptide.

9. Plant, plant part or plant cell transformed with a construct according to claim 7, wherein said construct is integrated into the genome of the plant, plant part or plant cell.

10. Method for the production of a transgenic plant having increased seed yield,
wherein said seed yield is one or more of the following:
(i) Increased number of seeds per plant;
(ii) Increased number of filled seeds per plant;
(iii) Increased (total) seed weight per plant.
comprising:
(i) Introducing and expressing in a plant a nucleic acid encoding a Class III TPP protein according to claim 1 or 6 and
(ii) cultivating the plant cell under conditions promoting plant growth and development.

11. Transgenic plant having increased seed yield relative to control plants, said increased seed yield resulting from increased expression of an introduced nucleic acid encoding a Class III TPP protein as defined in claim 6, or a transgenic plant cell obtainable from said transgenic plant, wherein said transgenic plant is a crop plant or a monocot or a cereal, such as rice, maize, wheat, barley, millet, rye, sorghum and oats, or a transgenic plant cell derived from said transgenic plant, wherein said control plants are corresponding wild-type plants or corresponding plants without said nucleic acid encoding said class III trehalose phosphate polypeptide.

12. Harvestable parts of a plant as defined in claim 11, wherein said harvestable parts comprise a construct as defined in claim 7.

13. Harvestable parts according to claim 12, wherein said harvestable parts are seeds.

14. Use of a nucleic acid encoding a Class III TPP protein according to claim 1 or 6 in increasing seed yield in plants relative to control plants when introduced and expressed in said plants, wherein said seed yield is one or more of the following:
(i) Increased number of seeds per plant;
(ii) Increased number of filled seeds per plant;
(iii) Increased (total) seed weight per plant,
wherein said control plants are corresponding wild-type plants or corresponding plants without said nucleic acid encoding said class III trehalose phosphate polypeptide.

## Patentansprüche

1. Verfahren zur Steigerung des Samenertrags bei Pflanzen unter Bezugnahme auf Kontrollpflanzen, wobei der gesteigerte Samenertrag einem oder mehreren der folgenden Fälle entspricht:
(i) gesteigerte Anzahl von Samen pro Pflanze;
(ii) gesteigerte Anzahl von ausgefüllten Samen pro Pflanze;
(iii) gesteigertes Samengewicht pro Pflanze;
wobei es das Verstärken, bei einer Pflanze, der Expression einer Nukleinsäure umfasst, die für ein Trehalose-Phosphat-Phosphatase(TPP)-Polypeptid der Klasse III codiert, wobei die verstärkte Expression bewirkt wird, indem eine Nukleinsäure, die für ein TPP-Polypeptid der Klasse III codiert, in eine Pflanze eingebracht und dort exprimiert wird,
wobei die Nukleinsäure für ein TPP-Polypeptid der Klasse III codiert, das mindestens 85 % Sequenzidentität mit der Aminosäuresequenz aufweist, die in der SEQ-ID Nr. 4 angegeben ist, oder
wobei das TPP-Polypeptid der Klasse III Folgendes umfasst:
(i) eine Trehalose-PPase-Domäne, die mindestens 50 % Aminosäuresequenzidentität mit der SEQ-ID Nr.: 93 oder der SEQ-ID Nr.: 94 aufweist, wobei die Trehalose-PPase-Domäne folgendes aufweist:
a. eine Phosphatase-Box A, die mindestens 50 % Sequenzidentität mit der SEQ-ID Nr.: 96 aufweist; und/oder
b. eine Phosphatase-Box B, die mindestens 50 % Sequenzidentität mit der SEQ-ID Nr.: 97 aufweist; und/oder
(ii) eine serinreiche Domäne, die mindestens 50 % Sequenzidentität mit der SEQ-ID Nr.: 95 aufweist,
wobei es sich bei den Kontrollpflanzen um die entsprechenden Wildtyp-Pflanzen handelt oder um entsprechende Pflanzen ohne die Nukleinsäure, welche für das Trehalose-Phosphat-Phosphatase-Polypeptid der Klasse III codiert.

2. Verfahren nach Anspruch 1, wobei die Nukleinsäure, welche für das TPP-Protein der Klasse III codiert:
(i) der Nukleinsäure mit der SEQ-ID Nr.: 3 oder einem Abschnitt derselben mit einer Länge von mindestens 625 aufeinander folgenden Nukleotiden entspricht, oder einer Sequenz, die dazu befähigt ist, unter stringenten Bedingungen mit der Nukleinsäure zu hybridisieren, welche die SEQ-ID Nr.: 4 aufweist, und/oder
(ii) für ein TPP-Polypeptid der Klasse III mit der SEQ-ID Nr.: 4 codiert.

3. Verfahren nach einem beliebigen der Ansprüche 1 bis 2, wobei die Nukleinsäure operativ mit einem konstitutiven Promotor verknüpft ist.

4. Pflanze oder Teil derselben, einschließlich der Samen, die/der mittels eines Verfahren nach einem beliebigen der Ansprüche 1 bis 4 erhalten werden kann, wobei die Pflanze oder der Teil derselben eine rekombinante Nukleinsäure enthält, welche für ein TPP-Protein der Klasse III mit der SEQ-ID Nr.: 4 codiert, wobei sie mit einem konstitutiven Promotor operativ verknüpft ist.

5. Isoliertes Nukleinsäuremolekül, das Folgendes umfasst:
(i) eine Nukleinsäure der SEQ-ID Nr.: 3;
(ii) das komplementäre Element zu der SEQ-ID Nr., die in (i) angegeben ist;
(iii) eine Nukleinsäure, die für ein TPP-Polypeptid der Klasse III codiert, welches mindestens 90 % Sequenzidentität mit der Aminosäuresequenz aufweist, die in der SEQ-ID Nr. 4 angegeben ist.

6. Isoliertes Polypeptid, das Folgendes umfasst:
(i) eine Aminosäuresequenz gemäß SEQ-ID Nr. 4;
(ii) eine Aminosäuresequenz, die mindestens 90 % Sequenzidentität mit der Aminosäuresequenz aufweist, die in der SEQ-ID Nr. 4 angegeben ist.

7. Konstrukt, das Folgendes umfasst:
(a) eine Nukleinsäure, die für ein TPP-Polypeptid der Klasse III nach Anspruch 6 codiert; und
(b) eine oder mehrere Steuerungssequenzen, die dazu befähigt sind, die Expression der Nukleinsäuresequenz von (a) zu steuern, wobei es sich bei einer der Steuersequenzen um einen GOS2-Promotor handelt, und
(c) gegebenenfalls eine transkriptionsbeendende Sequenz.

8. Verwendung eines Konstrukts nach Anspruch 7 in einem Verfahren zur Herstellung von Pflanzen mit einem unter Bezugnahme auf Kontrollpflanzen gesteigerten Samenertrag, wobei der Samenertrag einem oder mehreren der folgenden Fälle entspricht:
(i) gesteigerte Anzahl von Samen pro Pflanze;
(ii) gesteigerte Anzahl von ausgefüllten Samen pro Pflanze;
(iii) gesteigertes (Gesamt-)Samengewicht pro Pflanze,
wobei es sich bei den Kontrollpflanzen um die entsprechenden Wildtyp-Pflanzen handelt oder um entsprechende Pflanzen ohne die Nukleinsäure, welche für das Trehalose-Phosphat-Phosphatase-Polypeptid der Klasse III codiert.

9. Pflanze, Pflanzenteil oder Pflanzenzelle, die bzw. der mit einem Konstrukt nach Anspruch 7 transformiert wurde, wobei das Konstrukt in das Genom der Pflanze, des Pflanzenteils oder der Pflanzenzelle integriert ist.

10. Verfahren zur Herstellung einer transgenen Pflanze mit gesteigertem Samenertrag, wobei der Samenertrag einem oder mehreren der folgenden Fälle entspricht:
(i) gesteigerte Anzahl von Samen pro Pflanze;
(ii) gesteigerte Anzahl von ausgefüllten Samen pro Pflanze;
(iii) gesteigertes (Gesamt-)Samengewicht pro Pflanze;
wobei es Folgendes umfasst:
(i) Einbringen einer Nukleinsäure, die für ein TPP-Protein der Klasse III nach Anspruch 1 oder 6 codiert, in eine Pflanze, um sie dort zu exprimieren, und
(ii) Kultivieren der Pflanzenzelle unter Bedingungen, die das Wachstum und die Entwicklung der Pflanze fördern.

11. Transgene Pflanze mit einem unter Bezugnahme auf Kontrollpflanzen gesteigertem Samenertrag, wobei sich der gesteigerte Samenertrag aus der verstärkten Expression einer eingebrachten Nukleinsäure ergibt, welche für ein TPP-Protein der Klasse III nach der Begriffsbestimmung in Anspruch 6 codiert, oder transgene Pflanzenzelle, die aus der transgenen Pflanze erhalten werden kann, wobei die transgene Pflanze eine Feldfruchtpflanze oder eine einkeimblättrige Pflanze oder eine Getreidepflanze wie etwa Reis, Mais, Weizen, Gerste, Hirse, Roggen, Sorghumhirse oder Hafer ist, oder transgene Pflanzenzelle, die von der transgenen Pflanze abstammt, wobei es sich bei den Kontrollpflanzen um die entsprechenden Wildtyp-Pflanzen handelt oder um entsprechende Pflanzen ohne die Nukleinsäure, welche für das Trehalose-Phosphat-Phosphatase-Polypeptid der Klasse III codiert.

12. Bei der Ernte gewinnbare Teile einer Pflanze nach der Begriffsbestimmung in Anspruch 11, wobei die bei der Ernte gewinnbaren Teile ein Konstrukt gemäß der Begriffsbestimmung in Anspruch 7 umfassen.

13. Bei der Ernte gewinnbare Teile nach Anspruch 12, wobei es sich bei den bei der Ernte gewinnbaren Teilen um Samen handelt.

14. Verwendung einer Nukleinsäure, die für ein TPP-Protein der Klasse III nach Anspruch 1 oder 6 codiert, zur Steigerung des Samenertrags bei Pflanzen unter Bezugnahme auf Kontrollpflanzen, wenn sie in die Pflanzen eingebracht und dort exprimiert wird, wobei der gesteigerte Samenertrag einem oder mehreren der folgenden Fälle entspricht:
(i) gesteigerte Anzahl von Samen pro Pflanze;
(ii) gesteigerte Anzahl von ausgefüllten Samen pro Pflanze;
(iii) gesteigertes (Gesamt-)Samengewicht pro Pflanze,
wobei es sich bei den Kontrollpflanzen um die entsprechenden Wildtyp-Pflanzen handelt oder um entsprechende Pflanzen ohne die Nukleinsäure, welche für das Trehalose-Phosphat-Phosphatase-Polypeptid der Klasse III codiert.

## Revendications

1. Procédé pour augmenter le rendement en graines dans des plantes par rapport à des plantes témoins, dans lequel ledit rendement en graines augmenté est l'un ou plusieurs quelconques des suivants :
(i) nombre de graines par plante augmenté ;
(ii) nombre de graines pleines par plante augmenté ;
(iii) poids de graines par plante augmenté ;
comprenant l'augmentation de l'expression dans une plante d'un acide nucléique codant pour un polypeptide de tréhalose phosphate phosphatase (TPP) de classe III, dans lequel ladite expression augmentée est effectuée par introduction et expression dans une plante d'un acide nucléique codant pour un polypeptide TPP de classe III,
dans lequel ledit acide nucléique code pour un polypeptide TPP de classe III ayant au moins 85 % d'identité de séquence à la séquence d'acides aminés décrite dans SEQ ID NO: 4, ou
dans lequel ledit polypeptide TPP de classe III comprend :
(i) un domaine de tréhalose-PPase ayant au moins 50 % d'identité de séquence d'acides aminés à SEQ ID NO: 93 ou SEQ ID NO: 94, ledit domaine de tréhalose-PPase comprenant :
a. une boîte phosphatase A ayant au moins 50 % d'identité de séquence à SEQ ID NO: 96 ; et/ou
b. une boîte phosphatase B ayant au moins 50 % d'identité de séquence à SEQ ID NO: 97 ; et/ou (ii) un domaine riche en sérine ayant au moins 50 % d'identité de séquence à SEQ ID NO: 95,
dans lequel lesdites plantes témoins sont des plantes de type sauvage correspondantes ou des plantes correspondantes sans ledit acide nucléique codant pour ledit polypeptide de tréhalose phosphate phosphatase de classe III.

2. Procédé selon la revendication 1, dans lequel ledit acide nucléique codant pour une protéine TPP de classe III :
(i) est l'acide nucléique ayant SEQ ID NO: 3 ou une partie de celui-ci étant d'au moins 625 nucléotides consécutifs de longueur, ou une séquence capable de s'hybrider dans des conditions stringentes avec un acide nucléique ayant SEQ ID NO: 4, et/ou
(ii) code pour un polypeptide TPP de classe III ayant SEQ ID NO: 4.

3. Procédé selon l'une quelconque des revendications 1 à 2, dans lequel ledit acide nucléique est fonctionnellement lié à un promoteur constitutif.

4. Plante ou partie de celle-ci, comprenant des graines, pouvant être obtenue par un procédé selon l'une quelconque des revendications 1 à 4, ladite plante ou partie de celle-ci comprenant un acide nucléique recombinant codant pour une protéine TPP de classe III de SEQ ID NO: 4, fonctionnellement liée à un promoteur constitutif.

5. Molécule d'acide nucléique isolé comprenant :
(i) un acide nucléique de SEQ ID NO: 3 ;
(ii) le complément du SEQ ID NO décrit dans (i) ;
(iii) un acide nucléique codant pour une protéine TPP de classe III ayant au moins 90 % d'identité de séquence à la séquence d'acides aminés décrite dans SEQ ID NO: 4,

6. Polypeptide isolé comprenant :
(i) une séquence d'acides aminés de SEQ ID NO: 4 ;
(ii) une séquence d'acides aminés ayant au moins 90 % d'identité de séquence à la séquence d'acides aminés décrite dans SEQ ID NO: 4 ;

7. Construction comprenant :
(a) un acide nucléique codant pour un polypeptide TPP de classe III selon la revendication 6 ; et
(b) une ou plusieurs séquences de contrôle capables d'induire l'expression de la séquence d'acide nucléique de (a), ladite séquence de contrôle étant un promoteur GOS2 ; et
(c) facultativement une séquence de terminaison de transcription.

8. Utilisation d'une construction selon la revendication 7 dans un procédé de fabrication de plantes ayant un rendement en graines augmenté par rapport à des plantes témoins, dans laquelle ledit rendement en graines est un ou plusieurs des suivants :
(i) nombre de graines par plante augmenté ;
(ii) nombre de graines pleines par plante augmenté ;
(iii) poids (total) de graines par plante augmenté, dans laquelle lesdites plantes témoins sont des plantes de type sauvage correspondantes ou des plantes correspondantes sans ledit acide nucléique codant pour ledit polypeptide de tréhalose phosphate phosphatase de classe III.

9. Plante, partie de plante ou cellule de plante transformée avec une construction selon la revendication 7, ladite construction étant intégrée dans le génome de la plante, partie de plante ou cellule de plante.

10. Procédé pour the production de a plante transgénique ayant rendement en graines augmenté, dans lequel ledit rendement en graines est un ou plusieurs des suivants :
(i) nombre de graines par plante augmenté ;
(ii) nombre de graines pleines par plante augmenté ;
(iii) poids (total) de graines par plante augmenté ;
comprenant :
(i) l'introduction et l'expression dans une plante d'un acide nucléique codant pour une protéine TPP de classe III selon la revendication 1 ou 6 et
(ii) la culture de la cellule de plante dans des conditions favorisant la croissance et le développement de la plante.

11. Plante transgénique ayant un rendement en graines augmenté par rapport à des plantes témoins, ledit rendement en graines augmenté résultant d'une expression augmentée d'un acide nucléique introduit codant pour une protéine TPP de classe III telle que définie dans la revendication 6, ou une cellule de plante transgénique pouvant être obtenue à partir de ladite plante transgénique, ladite plante transgénique étant une plante cultivée ou une monocotylédone ou une céréale, telle que le riz, le maïs, le blé, l'orge, le millet, le seigle, le sorgho et l'avoine, ou une cellule de plante transgénique dérivée de ladite plante transgénique, lesdites plantes témoins étant des plantes de type sauvage correspondantes ou des plantes correspondantes sans ledit acide nucléique codant pour ledit polypeptide de tréhalose phosphate phosphatase de classe III.

12. Parties récoltables d'une plante telle que définie dans la revendication 11, lesdites parties récoltables comprenant une construction telle que définie dans la revendication 7.

13. Parties récoltables selon la revendication 12, lesdites parties récoltables étant des graines.

14. Utilisation d'un acide nucléique codant pour une protéine TPP de classe III selon la revendication 1 ou 6 dans l'augmentation du rendement en graines dans des plantes par rapport à des plantes témoins lorsqu'il est introduit et exprimé dans lesdites plantes, dans laquelle ledit rendement en graines est un ou plusieurs des suivants :
(i) nombre de graines par plante augmenté ;
(ii) nombre de graines pleines par plante augmenté ;
(iii) poids (total) de graines par plante augmenté,
dans laquelle lesdites plantes témoins sont des plantes de type sauvage correspondantes ou des plantes correspondantes sans ledit acide nucléique codant pour ledit polypeptide de tréhalose phosphate phosphatase de classe III.
